# EUROPEAN PATENT SPECIFICATION

(11) **EP 2 763 971 B1**
(45) Date of publication and mention of the grant of the patent: **08.06.2016**
(21) Application number: 12754018.5
(22) Date of filing: 07.09.2012
(51) Int. Cl.: C07D 261/08, C07D 413/12, C07D 413/14, C07D 417/12, C07K 7/56, A61K 38/12, A61P 31/00

(54) **PREPARATION OF MICAFUNGIN INTERMEDIATES**
HERSTELLUNG VON MICAFUNGIN ZWISCHENPRODUKTEN
PRÉPARATION D'INTERMÉDIARES DE MICAFONGINE

(30) Priority: 09.09.2011 EP 11180686; 09.09.2011 EP 11180689; 09.09.2011 EP 11180690
(43) Date of publication of application: 13.08.2014
(73) Proprietor: Sandoz AG, 4056 Basel (CH)
(72) Inventor: BARTH, Roland, A-6250 Kundl (AT); KNEPPER, Kerstin, A-6250 Kundl (AT); STURM, Hubert, A-6250 Kundl (AT)
(74) Representative: Wibbelmann, Jobst
(86) International application number: PCT/EP2012/067475
(87) International publication number: WO 2013/034670

(56) References cited:
- WO-A1-00/75178
- WO-A1-96/11210
- WO-A1-2004/014879
- TOMISHIMA M ET AL: "Novel echinocandin antifungals. Part 2: Optimization of the side chain of the natural product FR901379. Discovery of micafungin", BIOORGANIC & MEDICINAL CHEMISTRY LETTERS, PERGAMON, ELSEVIER SCIENCE, GB, vol. 18, no. 9, 1 May 2008 (2008-05-01), pages 2886-2890, XP022634974, ISSN: 0960-894X, DOI: 10.1016/J.BMCL.2008.03.093 [retrieved on 2008-04-08] cited in the application
- M. TOMISHIMA ET AL: "FK463, a novel water-soluble Echinocandin lipopeptide: synthesis and antifungal activity", THE JOURNAL OF ANTIBIOTICS, vol. 52, no. 7, 1999, pages 674-676, XP008157956, cited in the application

## Description

The present invention relates to the preparation of compounds, in particular to the preparation of compounds which may be used as intermediates for the preparation of antifungal agents, preferably micafungin (MICA), and salts thereof.

Background prior art Micafungin (CAS Registry Number 235114-32-6; IUPAC Name: {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4.7,13,16,22-hexaazatricyclo[22.3.0.0^{9,13}]heptacosan-6-yl]-1,2-dihydroxyethyl]-2-hydroxyphenyl}oxidanesulfonic acid) is an echinocandin antifungal agent represented by the structure:

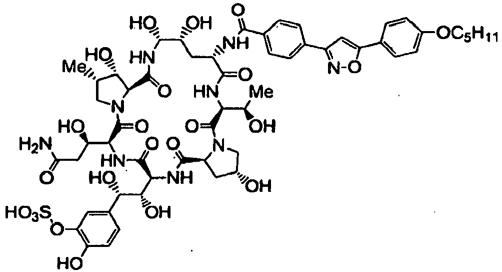

Micafungin is used to treat fungal infections caused by candidemia, acute disseminated candidiasis, *Candida* peritonitis, abscesses and esophageal candidiasis. One of the important intermediates for the preparation of micafungin (MICA) is the compound of formula (VI), (II'), or (II"), 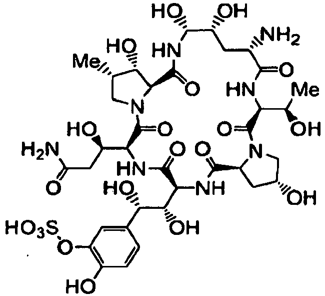 called CMICA or FR179642, which is typically acylated with an isolable benzotriazole active ester of 4-{5-[4-(pentyloxy)phenyl]-3-isooxazyl}benzoic acid (PPIB-OBT) of the following structure 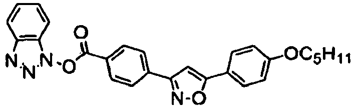 to obtain MICA. The benzotriazole active ester of PPIB is typically obtained after the conversion of PPIB with 1-Hydroxy-1H-benzotriazole (HOBT) in the presence of 1-ethyl-3-(3-dimethylaminopropyl) carbodiimide (EDCI). WO 1996011210A1 discloses a process wherein MICA is obtained after conversion of CMICA with the benzotriazole active ester of PPIB in the presence of 4-dimethylaminopyridine (DMAP). Similar procedures described by Kanasaki et al., J. Antibiotics 1999, 52, 674; and Tomishima et al., Bioorg. Med. Chem. Lett. 2008, 18, 2886; disclose yields of the conversion of CMICA with the benzotriazole active ester of PPIP in the range of 53.3 % and 75 %, respectively. WO 2004014879 discloses the conversion of CMICA with the benzotriazole active ester of PPIB in the presence of dimethylformamide (DMF) providing a calculated yield of 75 %.

WO00/75178 (A1) discloses the 2,3,4,5,6-pentafluorophenyl ester of 4-[5-[4-(pentyloxy)phenyl]-3-isoxazolyl]-benzoic acid having CAS RN 310459-15-5 and its use in a process for the preparation of an antifungal agent.

**Part A**: However, said prior art provides only processes having the major drawback of including a three step synthetic protocol when MICA is produced at a larger scale. In particular, PPIB is activated to give an active ester of PPIB in a first step, followed by purification and isolation of the obtained active ester of PPIB in a second step and subsequent conversion with CMICA to give MICA in a third step. There is a constant search for new key intermediates, in particular for new active esters of PPIB, more particular for new active esters of PPIB which allow a one-pot synthesis from PPIB to give an antifungal agent, preferably MICA, or a salt thereof, preferably without the need to isolate and/or purify the active ester of PPIB before the conversion with another key intermediate, preferably CMICA. There is also a need for improved synthetic protocols for such key intermediates, in particular such active esters of PPIB. Therefore, it was an object of the present invention to provide new key intermediates, in particular new active esters of PPIB, more particular new active esters of PPIB which allow a one-pot synthesis from PPIB to give an antifungal agent, preferably MICA, or a salt thereof, without the need to isolate and/or purify the active ester of PPIB before the conversion with another key intermediate, preferably CMICA, or a salt thereof. It was another object of the present invention to provide an improved process for the preparation of such key intermediates, in particular such active esters of PPIB. It was a further object of the present invention to provide an improved process for the preparation of an antifungal agent, preferably MICA, or a salt thereof, starting from PPIB. It was another object of the present invention to provide an improved process for the preparation of an antifungal agent, preferably MICA, or a salt thereof, starting from PPIB, wherein PPIB is first activated and then converted with another key intermediate, preferably CMICA, to give an antifungal agent, preferably MICA, or a salt thereof, in a one-pot synthesis.

**Part B.** The benzotriazole active ester of PPIB also has several drawbacks. First of all, it is typically obtained by a process which uses HOBT, an expensive, hygroscopic, highly inflammable and unstable compound, and EDCI, also an expensive, moisture-sensitive, and allergenic compound, as reactants, therewith rendering the process costly and complex in terms of the reaction conditions. Secondly, the conversion with CMICA to obtain MICA sometimes involves the use of acylating agents such as pyridine, therewith rendering the overall process to obtain MICA even less economic. There is a constant search for new key intermediates, in particular for new active esters of PPIB, more particular for new active esters of PPIB which can be obtained via an economic and undemanding synthetic route. There is also a need for improved synthetic protocols for such key intermediates, in particular such active esters of PPIB. There is also a need for new key intermediates, in particular for new active esters of PPIB, more particular for new active esters of PPIB which allow a conversion with another key intermediate, preferably CMICA, to obtain an antifungal agent, preferably MICA, without the need to use catalyst or promoters, such as acylating agents. Therefore, it was an object of the present invention to provide new key intermediates, in particular new active esters of PPIB, more particular new active esters of PPIB which can be obtained via an economic and/or undemanding synthetic route. It was another object of the present invention to provide a process for the preparation of such key intermediates, in particular such active esters of PPIB. It was another object of the present invention to provide new key intermediates, in particular new active esters of PPIB, more particular new active esters of PPIB which allow a conversion with another key intermediate, preferably CMICA or a salt thereof, to obtain an antifungal agent, preferably MICA or a salt thereof, without the need to use catalysts or promoters, such as acylating agents. It was a further object of the present invention to provide an improved process for the preparation of an antifungal agent, preferably MICA, or a salt thereof, starting from PPIB.

**Part C.** Due to the above mentioned drawbacks of the the benzotriazole active ester of PPIB there is a constant search for new key intermediates, in particular for new active esters of PPIB, more particular for new active esters of PPIB which can be obtained via an economic and undemanding synthetic route. There is also a constant search for new key intermediates, in particular for new active esters of PPIB, more particular for new active esters of PPIB which are easy to purify. There is also a need for improved synthetic protocols for such key intermediates, in particular such active esters of PPIB. There is also a need for new key intermediates, in particular for new active esters of PPIB, more particular for new active esters of PPIB which allow a conversion with another key intermediate, preferably CMICA, to obtain an antifungal agent, preferably MICA, in satisfactory yields. Therefore, it was an object of the present invention to provide new key intermediates, in particular new active esters of PPIB, more particular new active esters of PPIB which can be obtained via an economic and/or undemanding synthetic route. It was another object of the present invention to provide new key intermediates, in particular new active esters of PPIB, more particular new active esters of PPIB which are easy to purify. It was another object of the present invention to provide a process for the preparation of such key intermediates, in particular such active esters of PPIB. It was another object of the present invention to provide new key intermediates, in particular new active esters of PPIB, more particular new active esters of PPIB which allow a conversion with another key intermediate, preferably CMICA or a salt thereof, to obtain an antifungal agent, preferably MICA or a salt thereof, in satisfactory yields. It was a further object of the present invention to provide an improved process for the preparation of an antifungal agent, preferably MICA, or a salt thereof, starting from PPIB.

### Summary of the Invention:

**Part A:** According to one aspect the present invention relates to a compound of formula (I) 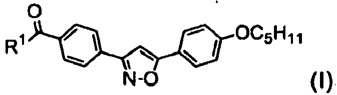 wherein R¹ is selected from:
a) Z-O- residues, wherein Z-O- is selected from
   (i) residues of formula (II) 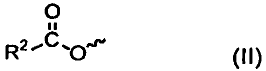
   (ii) residues of formula (III) 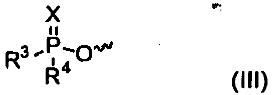 wherein R³ and R⁴ are identical or different; and wherein X is selected from O or S;
   (iii) residues of formula (IV) 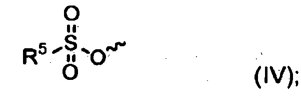 and
   (iv) residues of formula (V): 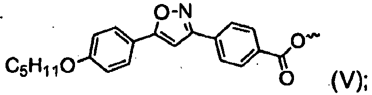
wherein R², R³, R⁴, and R⁵ are independently selected from alkyl residues, particularly having from 1 to 12 carbon atoms, wherein the alkyl residues are optionally aryl and/or aryloxy substituted; aryl residues, particularly having from 6 to 24 carbon atoms, wherein the aryl residues are optionally alkyl and/or alkyloxy substituted; alkyloxy residues, having from 1 to 12 carbon atoms, wherein the alkyloxy residues are optionally alkyl and/or aryl substituted; aryloxy residues, having from 6 to 24 carbon atoms, wherein the aryloxy residues are optionally alkyl and/or aryl substituted; heterocyclic residues; particularly having 2 to 14 carbon atoms; and having 1 to 4 heteroatoms as ring component atoms; wherein the heterocyclic residues are optionally alkyl, aryl, alkyloxy, aryloxy and/or oxo substituted; wherein the heteroatoms are same or different, and independently selected from N, O and S atoms; or (b) a group consisting of halides and pseudohalides.

According to a further aspect of the present invention there is provided the use of a compound of formula (I) as defined herein in a process for the preparation of another compound of formula (I) as defined herein. According to another aspect of the present invention there is provided the use of a compound of formula (I) as defined herein in a process for the preparation of compounds suitable as intermediates for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]₋2-hydroxyphenyl}oxidanesulfonic acid or a salt thereof. In still another aspect, there is provided the use of a compound of any of formula (I) as defined herein in a process for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof. According to a further aspect of the present invention there is provided a process for the preparation of compound of formula (I) 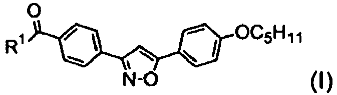 wherein R¹ is a Z-O- residue as defined herein; wherein the process comprises the step of reacting a compound of formula (VII) or a salt thereof 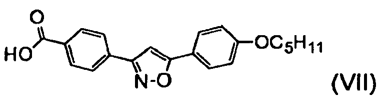 with a compound of formula (VIII)

Z-L (VIII)

wherein L is a leaving group. In still another aspect, there is provided a process for the preparation of a compound of formula (I) 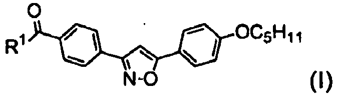 wherein R¹ is selected from a group consisting of halides and pseudohalides; wherein the process comprises the step of reacting a compound of formula (VII) or a salt thereof 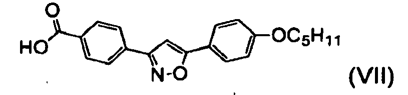 with a reagent capable of halogenating or pseudohalogenating a carboxylic acid. In yet another aspect of the present invention there is provided a process for the preparation of an antifungal agent, preferably {5-[(1*S*,2*S*)-2-[(3*S*,6*S*,9*S*,11*R,*15*S*,18*S*,20*R,*21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid, comprising a process to obtain a compound of formula (I) as defined herein, preferably by a process as defined herein.

The present invention represents an improvement over the known active ester of PPIB as the compounds for formula (I) may allow a one-pot synthesis from PPIB to give an antifungal agent, preferably MICA, or a salt thereof, preferably without the need to isolate and/or purify the active ester of PPIB before the conversion with another key intermediate, preferably CMICA. In particular, such a one-pot synthesis may result in the advantage of having a higher throughput at lower costs. A further advantage may be that less material is needed resulting in a more economical process.

**Part B:** According to one aspect the present invention relates to a compound of formula (I') 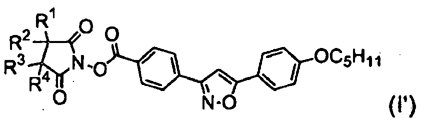 wherein R¹, R², R³ and R⁴ are same or different, and are independently selected from: hydrogen; alkyl residues, particularly having from 1 to 12 carbon atoms, wherein the alkyl residues are optionally aryl and/or aryloxy substituted; aryl residues, particularly having from 6 to 24 carbon atoms, wherein the aryl residues are optionally alkyl and/or alkyloxy substituted; alkyloxy residues, having from 1 to 12 carbon atoms, wherein the alkyloxy residues are optionally alkyl and/or aryl substituted; aryloxy residues, having from 6 to 24 carbon atoms, wherein the aryloxy residues are optionally alkyl and/or aryl substituted; heterocyclic residues; particularly having 2 to 14 carbon atoms; and having 1 to 4 heteroatoms as ring component atoms; wherein the heterocyclic residues are optionally alkyl, aryl, alkyloxy, aryloxy and/or oxo substituted; wherein the heteroatoms are same or different, and independently selected from N, O and S atoms; and wherein one of R¹ and R², and one of R³ and R⁴ are optionally fused to form a non-aromatic ring, wherein the other two are as defined above or form a bond; or an aromatic ring; or a bond, wherein the other two are as defined above. According to a further aspect of the present invention there is provided the use of a compound of formula (I') as defined herein in a process for the preparation of another compound of formula (I') as defined herein. According to another aspect of the present invention there is provided the use of a compound of formula (I') as defined herein in a process for the preparation of compounds suitable as intermediates for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxacol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof. In still another aspect, there is provided the use of a compound of any of formula (I') as defined herein in a process for the preparation of an antifungal agent, preferably {5-[(1*S*,2*S*)-2-[(3*S*.6*S*.9*S*,11*R*,15*S*,18*S*,20*R,*21*R,*24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof. According to a further aspect of the present invention there is provided a process for the preparation of compound of formula (I') 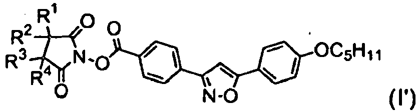 wherein R¹, R², R³ and R⁴ are as defined herein; wherein the process comprises the step of reacting a compound of formula (III') or a salt thereof 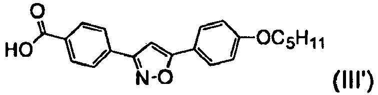 with a compound of formula (IV') 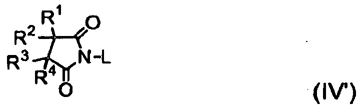 wherein L is a group to be formally substituted in the reaction of the compound of formula (III') or a salt thereof with the compound of formula (IV'). In yet another aspect of the present invention there is provided a process for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof, comprising a process to obtain a compound of formula (I') as defined herein, preferably by a process as defined herein. The present invention represents an improvement over the known active ester of PPIB as the compounds of formula (I') may be obtained in a process starting from PPIB without the use of HOBT and EDCI. In particular, such a process may result in the advantage of being more economic than the processes of the prior art. Furthermore, the compounds of formula (I') may be converted with CMICA to MICA without the use of catalyst or promoters.

**Part C:** According to one aspect the present invention relates to a compound of formula (I") 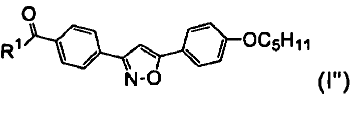 wherein R¹ is selected from Z-S- residues, wherein Z is selected from alkyl residues, particularly having from 1 to 12 carbon atoms, wherein the alkyl residues are optionally aryl and/or aryloxy substituted; aryl residues, particularly having from 6 to 24 carbon atoms, wherein the aryl residues are optionally alkyl and/or alkyloxy substituted; heterocyclic residues; particularly having 2 to 14 carbon atoms; particularly having 1 to 4 heteroatoms as ring component atoms, more particularly 2 to 3 heteroatoms; wherein the heteroatoms are same or different, and are independently selected from N, O and S atoms; wherein the heterocyclic residues are optionally alkyl, aryl, alkyloxy, aryloxy and/or oxo substituted; wherein the heterocyclic residues are optionally condensed with a hydrocarbon ring. According to a further aspect of the present invention there is provided the use of a compound of formula (I") as defined herein in a process for the preparation of another compound of formula (I") as defined herein. According to another aspect of the present invention there is provided the use of a compound of formula (I") as defined herein in a process for the preparation of compounds suitable as intermediates for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof. In still another aspect, there is provided the use of a compound of any of formula (I") as defined herein in a process for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof. According to a further aspect of the present invention there is provided a process for the preparation of compound of formula (I") 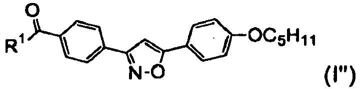 wherein R¹ is a Z-S- residue as defined herein; wherein the process comprises the step of reacting a compound of formula (III") or a salt thereof 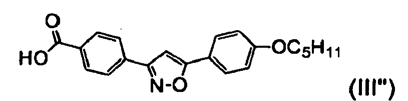 with a compound of formula (IV")

Z-S-L (IV")

wherein L is a group to be formally substituted in the reaction of the compound of formula (III") or a salt thereof with the compound of formula (IV"). Also disclosed is a process for the preparation of compound of formula (I") 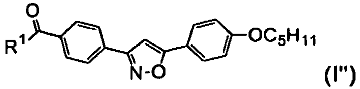 wherein **R¹** is an Y-O- residues as defined herein; wherein the process comprises the step of reacting a compound of formula (III") or a salt thereof 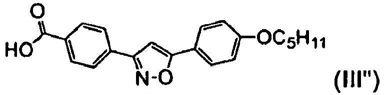 with a compound of formula (V")

Y-O-L (V")

wherein L is a group to be formally substituted in the reaction of the compound of formula (III") or a salt thereof with the compound of formula (V"). In yet another aspect of the present invention there is provided a process for the preparation of an antifungal agent, preferably (5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof, comprising a process to obtain a compound of formula (I") as defined herein, preferably by a process as defined herein. The present invention represents an improvement over the known active ester of PPIB as the compounds of formula (I") may be obtained in a process starting from PPIB without the use of HOBT. In particular, such a process may result in the advantage of being more economic than the processes of the prior art. In addition, the compounds of formula (I") may represent the improvement that they are easy to purify. Furthermore, the compounds of formula (I") may be converted with CMICA to MICA in satisfactory yields.

### Definitions

The terms "alkyl" and "alkyl residue" as used herein shall be understood as including at least any saturated or unsaturated; substituted or non-substituted; linear, branched or cyclic; hydrocarbon. Preferably, the alkyl or alkyl residue is a C1 to C12 alkyl. The alkyl or alkyl residue is optionally aryl and/or aryloxy substituted. The term "alkyl" as used herein also includes "alkenyl" groups comprising at least one carbon-to-carbon double bond, and "alkynyl" groups comprising at least one carbon-to-carbon triple bond. The terms "aryl" and "aryl residue" as used herein shall be understood as including at least any substituted or non-substituted; single-ring aromatic group or multicyclic aromatic group, for example tricyclic or bicyclic aryl groups. Preferably, the aryl or aryl residue is a C₆ to C₂₄ aryl residue. The aryl or aryl residue is optionally alkyl and/or alkyloxy substituted. The terms "alkyloxy" and "alkyloxy residue" as used herein shall be understood as including at least any saturated or unsaturated; substituted or non-substituted; linear, branched or cyclic; via oxygen bonded hydrocarbon. Preferably, the alkyloxy or alkyloxy residue is a C₁ to C₁₂ alkyloxy. The alkyloxy or alkyloxy residue is optionally alkyl and/or aryl substituted. The term alkyloxy as used herein also includes "alkenyloxy" groups comprising at least one carbon-to-carbon double bond, and "alkynyloxy" groups comprising at least one carbon-to-carbon triple bond. The terms "aryloxy" and "aryloxy residue" as used herein shall be understood as including at least any substituted or non-substituted; via oxygen bonded single-ring aromatic group or via oxygen bonded multicyclic aromatic group, for example tricyclic or bicyclic aromatic groups. Preferably, the aryloxy or aryloxy residue is a C₆ to C₂₄ aryloxy residue. The aryloxy or aryloxy residue is optionally alkyl and/or aryl substituted. The term "heterocyclic residue" or "heterocyclic ring" as used herein shall be understood as including at least any substituted or non-substituted; saturated, unsaturated or aromatic; single-ring heterocyclic group or multicyclic heterocyclic group, for example tricyclic or bicyclic heterocyclic groups. Preferably the heterocyclic residue is a 4 to 7-membered, more preferably 5 to 6-membered, mono-heterocyclic ring and comprising one or more, preferably from 1 to 4 such as 1, 2, 3 or 4, heteroatoms, wherein in case the heterocyclic residue comprises more than 1 heteroatom, the heteroatoms may be the same or different. The heterocyclic residues are optionally alkyl, aryl, alkyloxy, aryloxy and/or oxo substituted. The term "heteroatom" as used herein shall be understood as including at least an oxygen atom ("O"), a sulphur atom ("S"), a phosphorus atom ("P") or a nitrogen atom ("N"). It will be recognized that when the heteroatom is nitrogen, it may form an NR"R"' in which R" and R"' are, independent from another, hydrogen, C₁₋₄ alkyl, C₂₋₄ aminoalkyl, C₁-₄ haloalkyl or halo benzyl. Aromatic heterocyclic residues including from 1 to 4 heteroatoms are, for example, benzodioxolyl, pyrrolyl, furanyl, thiophenyl, thiazolyl, isothiazolyl, imidazolyl, triazolyl, tetrazolyl, pyrazolyl, oxazolyl, isoxazolyl, pyridinyl, pyrazinyl, pyridazinyl, benzoxazolyl, benzodioxazolyl, benzothiazolyl, benzoimidazolyl, benzothiophenyl, methylenedioxyphenylyl, naphthridinyl, quinolinyl, isoquinolinyl, indolyl, benzofuranyl, purinyl, benzofuranyl, deazapurinyl, or indolizinyl. The term "halide" or "halogen" as used herein shall be understood as including at least chlorine, iodine, bromine or fluorine. The term "pseudohalide" as used herein shall be understood as including at least cyanide, azide, cyanate, isocyanate, thiocyanate, isothiocyanate, selenocyanate, and tellurocyanate. The term "leaving group" as used herein shall be understood as known in the art referring to a group which is suitable to depart with a pair of electrons in heterolytic bond cleavage. Suitable leaving groups are listed for example in standard text books such as March's Advanced Organic Chemistry: Reactions, Mechanisms, and Structure, 6th Edition; Wiley & Sons. Preferred leaving groups are alkoxy residues; halides, in particular chloride, bromide and iodide; and sulphonate esters, in particular alkyl sulfonate, particularly mesylate, fluoroalkyl sulfonate; aryl sulfonate, particularly tosylate, nosylate, brosylate; fluorosulfonate, particularly triflate and nonaflate; nitrate; alkyl phosphate; alkyl borate; trialkylammonium; dialkylsulfonium; 2,4,5-trichlorophenoxy; 2,4-dinitrophenoxy; succinimido-N-oxy; and imidazolyl. The term "without purification" as used herein shall be understood as describing a process or step in which a compound is obtained in a first step and converted in a second step, wherein the compound is not isolated and/or purified between the first and the second step. The term "reagent capable of halogenating or pseudohalogenating a carboxylic acid" as used herein shall be understood as known in the art. In particular, it shall be understood as including reagents, which are capable of converting a hydroxyl group of a carboxylic acid into a halide or a pseudohalide, respectively. The terms "ambient temperature" and "room temperature" used herein will be understood by the person skilled in the art as referring to a temperature between about 20 °C and about 25 °C, particularly between 20 °C and 25 °C. The term "group to be formally substituted" used herein shall be understood as known in the art. In particular, it shall be understood as a group, which is formally substituted or replaced during a reaction, for example in the reaction of the compound of formula (III') or a salt thereof with the compound of formula (IV'). It will be understood that the "group to be formally substituted" may be a "leaving group" as defined above. However, it will also be understood that "group to be formally substituted" may be a group, which is formally substituted or replaced during a reaction, for example in the reaction of the compound of formula (III') or a salt thereof with the compound of formula (IV'), but which does not depart with a pair of electrons in heterolytic bond cleavage. A further example is the reaction of the compound of formula (III") or a salt thereof with the compound of formula (IV") or the compound of formula (V"). The corresponding reaction mechanisms, in particular the reaction mechanisms of the reaction of the compound of formula (III') or a salt thereof with the compound of formula (IV'), or the reaction of the compound of formula (III") or a salt thereof with the compound of formula (IV") or the compound of formula (V") are known to the person skilled in the art. Preferred "groups to be formally substituted" for part B of the invention are hydroxyl or alkoxy residues. Preferred "group to be formally substituted" are hydrogen for the compounds of formula (IV") and (V"), and Z-S- residues for the compound of formula (IV"). The term "activating agent" as used herein shall be understood as describing an agent which is suitable to activate a compound and to thereby obtain an activated compound. In particular, an activating agent is an agent that increases the activity of a compound towards an upcoming reaction. More particular, an activating agent activates a functional group in a compound to increase the activity toward an upcoming reaction of said functional group. The term "coupling agent" as used herein shall be understood as known in the art. In particular, it shall be understood as an agent having at least one functional group, which is capable of reacting with functional groups. The term "forming a bond" or "residues forming a bond" as used herein shall be understood as known in the art. In particular, it shall be understood as referring to the formation of a covalent bond, wherein each residue which "forms" the bond represents an electron in the resulting electron pair. It shall be understood that in part B of the invention when one of R¹ and R², and one of R³ and R⁴ are optionally fused to form a non-aromatic ring, the other two may form a carbon-carbon bond. It shall be understood that in part B of the invention when one of R¹ and R², and one of R³ and R⁴ are fused to form an aromatic ring, the other two consequently form a bond to allow the formation of an aromatic ring. Therewith, when one of R¹ and R², and one of R³ and R⁴ are fused in part B of the invention to form an aromatic ring, the other two form a bond. It shall also be understood that in part B of the invention when one of R¹ and R², and one of R³ and R⁴ are optionally fused to form bond, each residue which "forms" the bond represents an electron in the resulting electron pair.

### Detailed Description of the Invention; Part A

Compounds of formula (I) According to one aspect the present invention relates to a compound of formula (I) 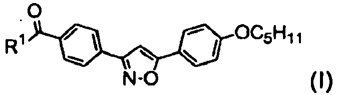 wherein R¹ is selected from:
a) Z-O- residues, wherein Z-O- is selected from
   (i) residues of formula (II) 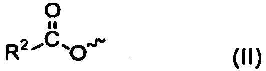
   (ii) residues of formula (III) 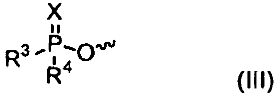 wherein R³ and R⁴ are identical or different; and wherein X is selected from O or S;
   (iii) residues of formula (IV) 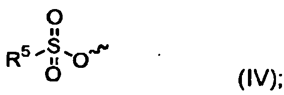 and
   (iv) residues of formula (V): 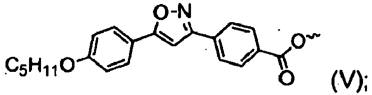
wherein R², R³, R⁴, and R⁵ are independently selected from alkyl residues, particularly having from 1 to 12 carbon atoms, wherein the alkyl residues are optionally aryl and/or aryloxy substituted; aryl residues, particularly having from 6 to 24 carbon atoms, wherein the aryl residues are optionally alkyl and/or alkyloxy substituted; alkyloxy residues, having from 1 to 12 carbon atoms, wherein the alkyloxy residues are optionally alkyl and/or aryl substituted; aryloxy residues, having from 6 to 24 carbon atoms, wherein the aryloxy residues are optionally alkyl and/or aryl substituted; heterocyclic residues; particularly having 2 to 14 carbon atoms; and having 1 to 4 heteroatoms as ring component atoms; wherein the heterocyclic residues are optionally alkyl, aryl, alkyloxy, aryloxy and/or oxo substituted; wherein the heteroatoms are same or different, and independently selected from N, O and S atoms; or (b) a group consisting of halides and pseudohalides.

The alkyl residues may be selected from methyl, ethyl, propyl, cyclopropyl, isobutyl, pentyl, hexyl, *iso*propyl, *iso*pentyl, and *tert*-butyl. The aryl substituted alkyl residues may be selected from benzyl, p-methylbenzyl, phenylpropyl and naphthylmethyl. The aryl residues may be typically selected from phenyl, 1-naphthyl, 2-naphthyl, biphenylyl, 1-anthracenyl, 2-anthracenyl, and 9-anthracenyl. The alkyloxy residues may be selected from methoxy, ethoxy, propoxy, cyclopropoxy, isobutoxy, pentoxy, hexoxy, *iso*propoxy, *iso*pentoxy, and *tert-*butoxy. The aryl substituted alkyloxy residues may be selected from benzyloxy, p-methylbenzyloxy, phenylpropyloxy and naphthylmethyloxy. The aryloxy residues may be selected from phenoxy, 1-naphthoxy, 2-naphthoxy, biphenyloxy, 1-anthracenyloxy, 2-anthracenyloxy, and 9-anthracenyloxy. The heterocyclic residues are typically selected from saturated heterocyclic residues, unsaturated heterocyclic residues, and aromatic heterocyclic residues, and can comprise at least one N atom as ring component atom and/or at least one O atom as ring component atom and/or at least one S atom as ring component atom. The heterocyclic residues can be 5 membered, 6 membered or 7 membered heterocyclic residues. Typically, when the heterocyclic residues are saturated heterocyclic residues, they are selected from 2-oxo-3-oxazolidinyl, tetrahydrofuranyl, tetrahydrothiophenyl, pyrrolidinyl, piperidinyl, tetrahydropyranyl, tetrahydrothiopyranyl, azapanyl, oxepanyl, and thiepanyl. When the heterocyclic residues are unsaturated heterocyclic residues, they are typically selected from imidazolidinyl and thiazinyl. Typically, when the heterocyclic residues are aromatic heterocyclic residues, they are selected from benzothiazo-2-yl, 5-methyl-1,3,4-thiadiazo-2-yl, 1,3,4-thiadiazo-2-yl, pyrrolyl, furyl, thiophenyl, pyridinyl, azepinyl, oxepinyl, thiepinyl, pyrazolyl, triazolyl, imidazolyl, benzimidazolyl, indolyl, isoindolyl, quinolinyl, isoquinolinyl, pyrimidinyl, oxazolyl, and isoxazolyl. In one embodiment, the Z-O- residue is a residue of formula (II). In another embodiment, the Z-O- residue is a residue of formula (III). According to a preferred embodiment, X is S. According to another preferred embodiment, X is O. In each case, R³ and R⁴ can be identical or different. In another embodiment, the Z-O- residue is a residue of formula (IV). In yet another embodiment, the Z-O- residue is a residue of formula (V). In a further embodiment, R¹ is a halide, particularly selected from fluoride, chloride, bromide and iodide, and is particularly chloride. In another embodiment, R¹ is a pseudohalide, particularly selected from cyanide, azide, cyanate, isocyanate, thiocyanate, isothiocyanate, selenocyanate, and tellurocyanate. In particularly preferred embodiments, the compound of formula (I) is

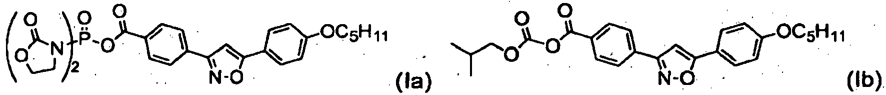

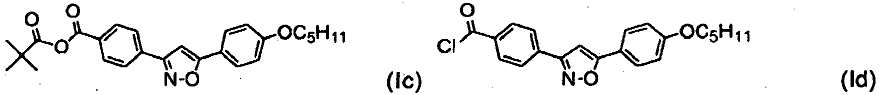

The compound of formula (I) as defined herein can also be present in form of a salt. The compounds of formula (I) as defined herein may have the advantage that they can be obtained by economical improved processes (see below), in particular processes with less demanding synthetic protocols with fewer steps, a low number of cheap reagents, a low amount of waste products and satisfactory yields. Furthermore, they may have the advantage that they can be used in a one-pot synthesis to obtain an antifungal agent, preferably MICA, or a salt thereof, wherein the compounds of formula (I) are first prepared and in situ reacted with other key intermediates, preferably CMICA, to give an antifungal agent, preferably MICA, or a salt thereof. Some of the compounds of formula (I), e.g. the compounds of formula (Ic) and (Id), may have the advantage that they can be isolated. According to a further aspect, any compound of formula (I) as defined herein can be used in a process for the preparation of any other compound of formula (I) as defined herein. According to another aspect, any compound of formula (I) as defined herein can be used in a process for the preparation of compounds suitable as intermediates for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid (MICA), or a salt thereof. According to yet another aspect, any compound of formula (I) as defined herein can be used in a process for the preparation of an antifungal agent, preferably {5-[(1*S*,2*S*)₋2 [(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid (MICA), or a salt thereof. According to all aspects and embodiments, MICA may be prepared in form of a salt, particularly in form of a sodium salt, a potassium salt, an ammonium salt, an ethanolamine salt, an alkylamine salt, and particularly in form of a sodium salt, a triethylamine salt, or a diisopropylethylamine salt. According to one embodiment of the process for the preparation of an antifungal agent, preferably MICA, or a salt thereof, the process comprises the step of reacting any compound of formula (I) as defined herein with a compound of formula (VI) or a salt thereof

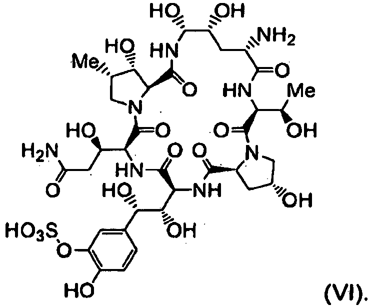

The step is typically carried out at a temperature of -30 °C to 50 °C, particularly from -20 °C to 30 °C, and more particularly -10 °C to room temperature. The step is typically carried out in a solvent, particularly an aprotic solvent, particularly selected from tetrahydrofuran (THF), dimethylformamide (DMF), toluene, 2-methyltetrahydrofuran, N-Methyl-2-pyrrolidone (NMP), and any combination thereof, and is particularly DMF. The step can be carried out in the presence of a base. The base is typically present in an amount of 1-50 equivalent, particularly of 20-40 equivalent, and more particularly 30-35 equivalent, with respect to the compound of formula (VI) or a salt thereof, when in the compound of formula (I) R¹ is a halide or a pseudohalide as defined herein. The base is then typically selected from pyridines, lutidines, picolines, dimethylaminopyridine, and collidines, and any combination thereof. The base is typically present in an amount of 1.0-4.0 equivalent, particularly of 1.2-3.0 equivalent, and more particularly 1.5-2.1 equivalent, with respect to the compound of formula (VI) or a salt thereof, when in the compound of formula (I) R¹ is selected from Z-O-residues as defined herein. The base is then typically selected from tertiary amines, particularly triethylamine and diisopropylethylamine; cyclic tertiary amines, particularly N-methylpiperidine and N-methylmorpholine; aromatic bases, particularly pyridines, lutidines, picolines, dimethylaminopyridine, and collidines, and any combination thereof; guanidine bases, particularly guanidine and tetramethylguanidine, amidine bases, particularly 1,8-Diazabicyclo[5.4.0]undec-7-en; and any combination thereof. When in the compound of formula(I) R¹ is selected from Z-O- residues as defined herein, the step may be carried out in the absence of a base. This may have the further advantage that fewer reagents are needed. According to all aspects and embodiments, the compound of formula (VI) may be used in form of a salt, particularly in form of a sodium salt, a potassium salt, an ammonium salt, an ethanolamine salt, an alkylamine salt, and particularly in form of a sodium salt, a triethylamine salt or a diisopropylethylamine salt.

### Processes for the preparation of a compound of formula (I)

According to one aspect, there is provided a process for the preparation of a compound of formula (I) 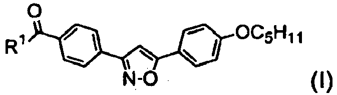 wherein R¹ is a Z-O- residue as defined herein, and wherein the process comprises the step of reacting a compound of formula (VII) or a salt thereof 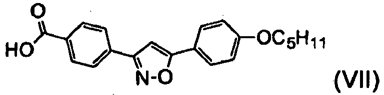 with a compound of formula (VIII)

Z-L (VIII)

wherein L is a leaving group. According to all aspects and embodiments, the compound of formula (VII) may be used in form of a salt, particularly in form of a sodium salt, a potassium salt, an ammonium salt, an ethanolamine salt, an alkylamine salt, and particularly in form of a sodium salt, a triethylamine salt or a diisopropylethylamine salt. It will be understood that L is selected to be suitable to depart with a pair of electrons in heterolytic bond cleavage during the process as described above. L is typically selected from halides, particularly chloride; alkyl sulfonate; fluoroalkyl sulfonate; aryl sulfonate; fluorosulfonate; nitrate; alkyl phosphate; alkyl borate; trialkylammonium; dialkylsulfonium; 2,4,5-trichlorophenoxy; 2,4-dinitrophenoxy; succinimido-N-oxy; and imidazolyl. Examples for the compounds of formula (VIII) are methyl chloroformate, ethyl chloroformate, propyl chloroformate, cyclopropyl chloroformate, *iso*butyl chloroformate, pentyl chloroformate, hexyl chloroformate, *iso*propyl chloroformate, *iso*pentyl chloroformate, *tert*-butyl chloroformate, pivalic acid chloride, and bis(2-oxo-3-oxazolidinyl)phosphonic chloride. Such a process may have the advantage that only one reagent, for example bis(2-oxo-3-oxazolidinyl)phosphonic chloride (BOP-Cl) or pivalic acid chloride, is needed to obtain a compound of formula (I) compared to the processes to obtain the benzotriazole active ester of 4-{5-[4-(pentyloxy)phenyl]-3-isooxazyl}benzoic acid (PPIB-OBT) as described in the art, while providing yields in the same range. In addition, the use of expensive EDCI may be avoided. The compound of formula (VIII) is typically present in an amount of 1.05-3 equivalent, particularly of 1.1-2 equivalent, and more particularly 1.1-1.4 equivalent, with respect to the compound of formula (VII) or a salt thereof. The step is typically carried out in the presence of a base, particularly selected from tertiary amines, particularly triethylamine and diisopropylethylamine; cyclic tertiary amines, particularly N-methylpiperidine and N-methylmorpholine; aromatic bases, particularly pyridines, lutidines, picolines, dimethylaminopyridine, and collidines, and any combination thereof; guanidine bases, particularly guanidine and tetramethylguanidine; amidine bases, particularly 1,8-Diazabicyclo[5.4.0]undec-7-en; and any combination thereof. The base is then typically present in an amount of 1.05-3 equivalent, particularly of 1.1-2 equivalent, and more particularly 1.1-1.4 equivalent, with respect to the compound of formula (VII) or a salt thereof. The step is typically carried out at a temperature of -30 °C to 50 °C, particularly from -20 °C to 10 °C, and more particularly -10 °C to 0 °C. When the step is carried out in a solvent, the solvent is particularly an aprotic solvent, particularly selected from tetrahydrofuran (THF), dimethylformamide (DMF), toluene, 2-methyltetrahydrofuran, and any combination thereof, and is particularly DMF. According to a preferred embodiment, there is provided a process for the preparation of a compound of formula (la) 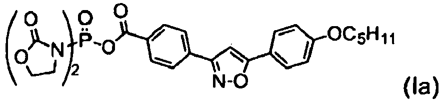 wherein the process comprises the step of reacting a compound of formula (VII) or a salt thereof with bis(2-oxo-3-oxazolidinyl)phosphonic chloride present in an amount of 1.1 equivalents, in the presence of 1.1 equivalents triethylamine, each with respect to the compound of formula (VII) or a salt thereof; at a temperature of 0 °C in DMF. According to another preferred embodiment, there is provided a process for the preparation of a compound of formula (Ib) 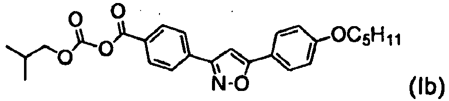 wherein the process comprises the step of reacting a compound of formula (VII) or a salt thereof with *iso*butyl chloroformate present in an amount of 1.1 equivalents, in the presence of 1.4 equivalents triethylamine, each with respect to the compound of formula (VII) or a salt thereof; at a temperature of - 10 °C in DMF. According to yet another preferred embodiment, there is provided a process for the preparation of a compound of formula (Ic) 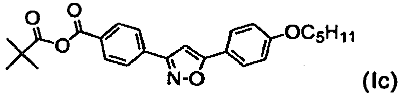 wherein the process comprises the step of reacting a compound of formula (VII) or a salt thereof with pivalic acid chloride present in an amount of 1.1 equivalents, in the presence of 1.4 equivalents triethylamine, each with respect to the compound of formula (VII) or a salt thereof; at a temperature of 0 °C in DMF. According to another aspect, there is provided herein a process for the preparation of a compound of formula (I), 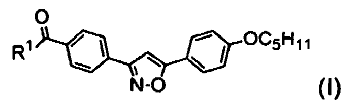 wherein R¹ is selected from a group consisting of halides or pseudohalides; wherein the process comprises the step of reacting a compound of formula (VII) or a salt thereof 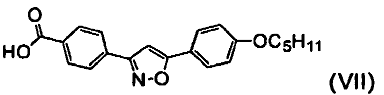 with a reagent capable of halogenating or pseudohalogenating a carboxylic acid. The halide is typically selected from fluoride, chloride, bromide and iodide, and is particularly chloride.

When the halide is chloride, the reagent capable of halogenating a carboxylic acid can be selected from thionyl chloride, oxalyl chloride, carbonyl dichloride, cyanuric chloride, phosphorous(III) chloride, and phosphorous(V) chloride. When the halide is iodide, the reagent capable of halogenating a carboxylic acid can be selected from iodide and acetyl iodide. When the halide is bromide and the reagent capable of halogenating a carboxylic acid can be phosphorus(V) bromide. When the halide is fluoride, the reagent capable of halogenating a carboxylic acid can be cyanuric fluoride. The pseudohalide is typically selected from cyanide, azide, cyanate, isocyanate, thiocyanate, isothiocyanate, selenocyanate, and tellurocyanate. The reagent capable of pseudobalogenating a carboxylic acid can be selected from organic cyanides, inorganic cyanides, organic azides, inorganic azides, particularly sodium azide, organic cyanates, inorganic cyanates, organic isocyanate, inorganic isocyanates, organic thiocyanates, inorganic thiocyanates, organic isothiocyanates, inorganic isothiocyanates, organic selenocyanaten, inorganic selenocyanates, organic tellurocyanates and inorganic tellurocyanates. Such a process may have the advantage that only one reagent, for example thionyl chloride or oxalyl chloride, is needed to obtain a compound of formula (I) compared to the processes to obtain the benzotriazole active ester of 4-{5-[4-(pentyloxy)phenyl]-3-isooxazyl}benzoic acid (PPIB-OBT) as described in the art, while providing yields in the same range. In addition, the use of expensive EDCI may be avoided. The reagent capable of halogenating or pseudohalogenating a carboxylic acid is typically present in an amount of 1.5-10 equivalent, particularly of 1.7-8 equivalent, and more particularly 2-4 equivalent, with respect to the compound of formula (VII) or a salt thereof. The step is typically carried out at a temperature of -30 °C to 50 °C, particularly from -20 °C to 10 °C, and more particularly -10 °C to 0 °C. When the step is carried out in a solvent, the solvent is particularly an aprotic solvent, particularly selected from tetrahydrofuran (THF), dimethylformamide (DMF), toluene, 2-methyltetrahydrofuran, and any combination thereof, and is particularly DMF. According to a preferred embodiment, there is provided a process for the preparation of a compound of formula (Id) 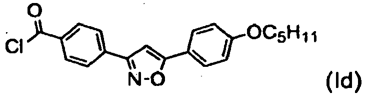 wherein the process comprises the step of reacting a compound of formula (VII) or a salt thereof with oxalyl chloride in an amount of 2 equivalents with respect to the compound of formula (VII) or a salt thereof, at a temperature of 0 °C in DMF.

### Processes for the preparation of an antifungal agent

According to a further aspect, there is provided a process for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amjdo]-1,4,7,13,16,22-hexaazatricyclo[22.3.0.0^{9,13}]heptacosan-6-yl]-1,2-.dihydroxyethyl]-2-hydroxyphenyl}oxidanesulfonic acid, comprising a process to obtain a compound of formula (I) as defined herein. Preferably, the process to obtain a compound of formula (I) is a process as defined herein. The process for the preparation of an antifungal agent typically comprises the step of reacting the obtained compound of formula (I) with a compound of formula (VI) or a salt thereof

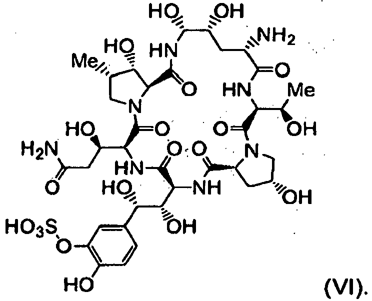

According a preferred embodiment, the process comprises a step of reacting the obtained compound of formula (I) with a compound of formula (VI) or a salt thereof, wherein the obtained compound is not isolated and/or purified after it has been obtained and before it is reacted with the compound of formula (VI) or a salt thereof. The term "without purification" as used herein shall be understood as relating to such a step or process. Therewith, the process for the preparation of an antifungal agent comprising such a step is a so called one-pot process or synthesis, which may have the advantage of being more economical than the processes known in the art while they may provide yields in the same range. In one particularly preferred embodiment, the process for the preparation of an antifungal agent comprises the step of reacting a compound of formula (VII) or a salt thereof 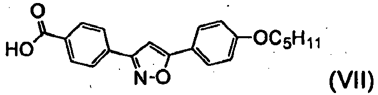 with bis(2-oxo-3-oxazolidinyl)phosphonic chloride present in an amount of 1.1 equivalents, in the presence of 1.1 equivalents triethylamine, each with respect to the compound of formula (VII) or a salt thereof; at a temperature of 0 °C in DMF; to obtain a compound of formula (Ia) 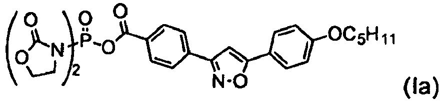 reacting the compound of formula (Ia) without purification, with a compound of formula (VI) or a salt thereof in the presence of 2.1 equivalents triethylamine, with respect to the compound of formula (VI) or a salt thereof; at a temperature of 0 °C in DMF. In another particularly preferred embodiment, the process for the preparation of an antifungal agent comprises the step of reacting a compound of formula (VII) or a salt thereof with *iso*butyl chloroformate present in an amount of 1.1 equivalents, in the presence of 1.4 equivalents triethylamine, each with respect to the compound of formula (VII) or a salt thereof; at a temperature of - 10 °C in DMF to obtain a compound of formula (Ib) 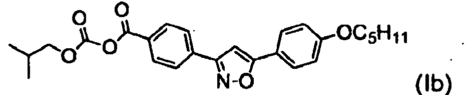 reacting the compound of formula (Ib) without purification, with a compound of formula (VI) or a salt thereof in the presence of 1.5 equivalents triethylamine, with respect to the compound of formula (VI) or a salt thereof; at a temperature of -10 °C to -5 °C in DMF. In yet another particularly preferred embodiment, the process for the preparation of an antifungal agent comprises the step of reacting a compound of formula (VII) or a salt thereof with pivalic acid chloride present in an amount of 1.1 equivalents, in the presence of 1.4 equivalents triethylamine, each with respect to the compound of formula (VII) or a salt thereof; at a temperature of 0 °C in DMF; to obtain a compound of formula (Ic) 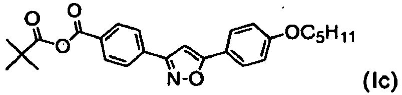 reacting the compound of formula (Ic) without purification, with a compound of formula (VI) or a salt thereof in the presence of 1.5 equivalents triethylamine, with respect to the compound of formula (VI) or a salt thereof; at room temperature in DMF. In yet another particularly preferred embodiment, the process for the preparation of an antifungal agent comprises the step of reacting a compound of formula (VII) or a salt thereof with oxalyl chloride in an amount of 2 equivalents with respect to the compound of formula (VII) or a salt thereof, at a temperature of 0 °C in DMF; to obtain a compound of formula (Id) 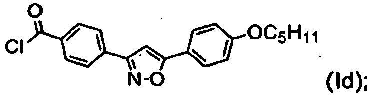 reacting the compound of formula (Id) without purification, with a compound of formula (VI) or a salt thereof in the presence of 29 equivalents pyridine, with respect to the compound of formula (VI) or a salt thereof; at a temperature of 0 °C in DMF. According to all aspects and embodiments, the compound of formula (VI) may be used in form of a salt, particularly in form of a sodium salt, a potassium salt, an ammonium salt, an ethanolamine salt, an alkylamine salt, and particularly in form of a sodium salt, a triethylamine salt or a diisopropylethylamine salt.

Part B: Compounds of formula (I') According to one aspect the present invention relates to a compound of formula (I') 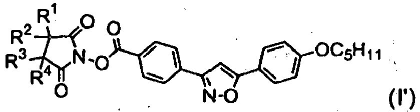 wherein R¹, R², R³ and R⁴ are same or different, and are independently selected from: hydrogen; alkyl residues, particularly having from 1 to 12 carbon atoms, wherein the alkyl residues are optionally aryl and/or aryloxy substituted; aryl residues, particularly having from 6 to 24 carbon atoms, wherein the aryl residues are optionally alkyl and/or alkyloxy substituted; alkyloxy residues, having from 1 to 12 carbon atoms, wherein the alkyloxy residues are optionally alkyl and/or aryl substituted; aryloxy residues, having from 6 to 24 carbon atoms, wherein the aryloxy residues are optionally alkyl and/or aryl substituted; heterocyclic residues; particularly having 2 to 14 carbon atoms; and having 1 to 4 heteroatoms as ring component atoms; wherein the heterocyclic residues are optionally alkyl, aryl, alkyloxy, aryloxy and/or oxo substituted; wherein the heteroatoms are same or different, and independently selected from N, O and S atoms; and wherein one of R¹ and R², and one of R³ and R⁴ are optionally fused to form a non-aromatic ring, wherein the other two are as defined above or form a bond; or an aromatic ring; or a bond, wherein the other two are as defined above.The alkyl residues may be selected from methyl, ethyl, propyl, cyclopropyl, *iso*butyl, pentyl, hexyl, *iso*propyl, *iso*pentyl, and *tert*-butyl. The aryl substituted alkyl residues may be selected from benzyl, p-methylbenzyl, phenylpropyl and naphthylmethyl. The aryl residues may be typically selected from phenyl, 1-naphthyl, 2-naphthyl, biphenylyl, 1-anthracenyl, 2-anthracenyl, and 9-anthracenyl. The alkyloxy residues may be selected from methoxy, ethoxy, propoxy, cyclopropoxy, *iso*butoxy, pentoxy, hexoxy, *iso*propoxy, *iso*pentoxy, and *tert*-butoxy. The aryl substituted alkyloxy residues may be selected from benzyloxy, p-methylbenzyloxy, phenylpropyloxy and naphthylmethyloxy. The aryloxy residues may be selected from phenoxy, 1-naphthoxy, 2-naphthoxy, biphenyloxy, 1-anthracenyloxy, 2-anthracenyloxy, and 9-anthracenyloxy. In one embodiment, the residues R¹, R², R³ and R⁴ are same. In a preferred embodiment, R¹, R², R³ and R⁴ are same and are hydrogen. In another preferred embodiment, R¹, R², R³ and R⁴ are same and are methyl. In another embodiment, three of R¹, R², R³ and R⁴ are same. In a preferred embodiment R¹, R² and R³ are same. In another preferred embodiment R¹, R³ and R⁴ are same. In yet another preferred embodiment R¹, R² and R⁴ are same. In a further preferred embodiment R², R³ and R⁴ are same. In any of these embodiments, the three residues of R¹, R², R³ and R⁴ which are same may be hydrogen. In yet another embodiment, two of R¹, R², R³ and R⁴ are same, wherein the others are different and are independently selected from each other. In a preferred embodiment R¹ and R² are same. In another preferred embodiment R¹ and R³ are same. In yet another preferred embodiment R¹ and R⁴ are same. In a further preferred embodiment R² and R³ are same. In another preferred embodiment R² and R⁴ are same. In yet another preferred embodiment R³ and R⁴ are same. In any of these embodiments, the two residues of R¹, R², R³ and R⁴ which are same may be hydrogen. In a further embodiment, R¹, R², R³ and R⁴ are different and are independently selected from each other. R¹ may be fused with R³ to form a ring. R¹ may be fused with R⁴ to form a ring. R² may be fused with R³ to form a ring. R² may be fused with R⁴ to form a ring. In a preferred embodiment one of R¹ and R² is fused with one of R³ and R⁴ to form a ring. In these embodiments, the two residues which are not fused may be same or different and are independently selected from each other. When the two residues which are not fused are same they are preferably hydrogen or methyl. It is also possible that when two of R¹, R², R³ and R⁴ are fused to form a ring, also the other two of R¹, R², R³ and R⁴ are fused to form another ring. When the ring is a non-aromatic ring, the other two residues are as defined herein. In a preferred embodiment, when the ring is a non-aromatic ring, the other two residues are hydrogen. In a more preferred embodiment, the ring is a cyclohexyl ring and the other two residues are hydrogen. In another embodiment, when the ring is a non-aromatic ring, the other two form a bond. When the ring is an aromatic ring, the other two residues consequently form the respective bond belonging to the aromatic system. The ring may be a saturated ring, an unsaturated ring, and an aromatic ring. In one embodiment, the ring is a 5 membered ring. In another embodiment, the ring is a 6 membered ring. In yet another embodiment, the ring is a 7 membered ring. The ring as described herein may be substituted, particularly with at least one residue selected from the group of alkyl residues, particularly having from 1 to 12 carbon atoms, wherein the alkyl residues are optionally aryl and/or aryloxy substituted; aryl residues, particularly having from 6 to 24 carbon atoms, wherein the aryl residues are optionally alkyl and/or alkyloxy substituted; alkyloxy residues, having from 1 to 12 carbon atoms, wherein the alkyloxy residues are optionally alkyl and/or aryl substituted; aryloxy residues, having from 6 to 24 carbon atoms, wherein the aryloxy residues are optionally alkyl and/or aryl substituted; and halides, particularly fluoride, chloride, bromide, and iodide. Examples for aromatic rings as addressed herein are benzene, naphthylene, biphenylene, and anthracenylene. Examples for saturated rings as addressed herein are cyclopentyl, cyclohexyl and cycloheptyl rings. Examples for unsaturated rings as addressed herein are cyclopentenyl, cyclohexenyl and cycloheptenyl rings. In one embodiment, the ring is a heterocyclic ring. The heterocyclic ring is typically selected from saturated heterocyclic rings, non-saturated heterocyclic rings, and aromatic heterocyclic rings, and can comprise at least one N atom as ring component atom and/or at least one O atom as ring component atom and/or at least one S atom as ring component atom. The heterocyclic ring can be a 5 membered, 6 membered or 7 membered heterocyclic ring. Typically, when the heterocyclic ring is a saturated heterocyclic ring, it is selected from 2-oxo-3-oxazolin, tetrahydrofuran, tetrahydrothiophene, pyrrolidine, piperidine, tetrahydropyran, tetrahydrothiopyran, azapane, oxepane, and thiepane. When the heterocyclic ring is an unsaturated heterocyclic ring, it is typically selected from imidazoline and thiazine. Typically, when the heterocyclic ring is an aromatic heterocyclic ring, it is selected from pyrrole, furan, thiophene, pyridine, azepine, oxepin, thiepin, pyrazole, triazole, imidazole, berizimidazole, indole, isoindole, quinoline, isoquinoline, pyrimidine, oxazole, and isoxazole. It will be understood that the term "heterocyclic residue" as used herein is defined as the term "heterocyclic ring", respectively. R¹ may be fused with R³ to form a bond. R¹ may be fused with R⁴ to form a bond. R² may be fused with R³ to form a bond. R² may be fused with R⁴ to form a bond. In a preferred embodiment, R¹ is fused with R³ to form a bond and the other two are hydrogen. In another preferred embodiment, R¹ is fused with R⁴ to form a bond and the other two are hydrogen. In another preferred embodiment, R² is fused with R³ to form a bond and the other two are hydrogen. In yet another preferred embodiment, R² is fused with R⁴ to form a bond and the other two are hydrogen. In particularly.preferred embodiments, the compound of formula (I') is

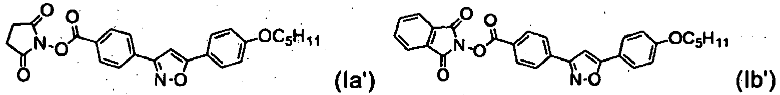

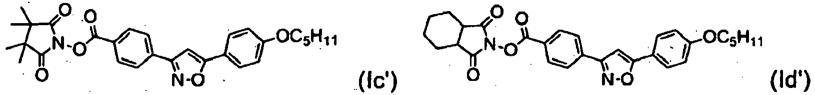

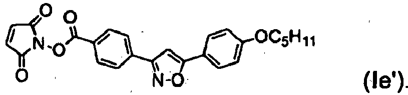

The compounds of formula (I') as defined herein can also be present in form of a salt. The compounds of formula (I') as defined herein may have the advantage that they can be obtained by economical improved processes (see below), in particular processes with less demanding synthetic protocols with a low number of cheap reagents and satisfactory yields comparable to the yields of the prior art. Furthermore, they may have the advantage that they can be used in a synthesis to obtain an antifungal agent, preferably MICA, or a salt thereof, wherein the compounds of formula (I') are reacted with other key intermediate, preferably CMICA, to give an antifungal agent, preferably MICA, or a salt thereof; without the use of catalysts or promoters. Some of the compounds of formula (I'), e.g. the compounds of formula (Ia') and (Ib'), may have the advantage that they can be isolated. According to a further aspect, any compound of formula (I') as defined herein can be used in a process for the preparation of any other compound of formula (I') as defined herein. According to another aspect, any compound of formula (I') as defined herein can be used in a process for the preparation of compounds suitable as intermediates for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9.13}]heptacosan-6-yl]-1,2-dihydroxyethyl]-2-hydroxyphenyl}oxidanesulfonic acid (MICA), or a salt thereof. According to yet another aspect, any compound of formula (I') as defined herein can be used
in a process for the preparation of an antifungal agent, preferably {5-[(1*S,*2*S*)-2 [(3*S*,6*S*,9*S*,11*R*,15*S*, 18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9.13}]heptacosan-6-yl]-1,2-dihydroxyethyl]-2-hydroxyphenyl}oxidanesulfonic acid (MICA), or a salt thereof. According to all aspects and embodiments, MICA may be prepared in form of a salt, particularly in form of a sodium salt, a potassium salt, an ammonium salt, an ethanolamine salt, an alkylamine salt, and particularly in form of a sodium salt, a triethylamine salt or a diisopropylamine salt. According to one embodiment of the process for the preparation of an antifungal agent, preferably MICA, or a salt thereof, the process comprises the step of reacting any compound of formula (I') as defined herein with a compound of formula (II') or a salt thereof

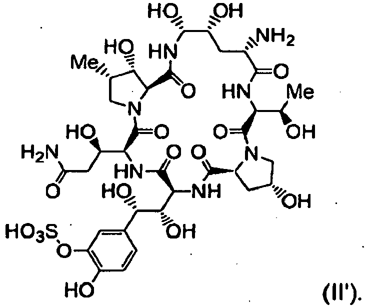

The step is typically carried out at a temperature of -30°C to 50°C, particularly from -20°C to 30°C, more particularly 0 °C to 30 °C, and most particularly at room temperature. The step is typically carried out in a solvent, particularly an aprotic solvent, particularly selected from tetrahydrofuran (THF), dimethylsulfoxid (DMSO), dimethylformamide (DMF), toluene, 2-methyltetrahydrofuran, *N*-Methyl-2-pyrrolidone (NMP), and any combination thereof, and is particularly DMF. The step can be carried out in the presence of a base. The base is typically present in an amount of 1-10 equivalent, and more particularly 1-2 equivalent, with respect to the compound of formula (II') or a salt thereof. The base is then typically selected from pyridines, lutidines, picolines, dimethylaminopyridine, triethylamine, isopropylethylamine, N-methylmorpholine, tetramethylguanidine, and collidines, and any combination thereof. The step can also be carried out in the absence of a base. According to all aspects and embodiments, the compound of formula (II') may be used in form of a salt, particularly in form of a sodium salt, a potassium salt, an ammonium salt, an ethanolamine salt, an alkylamine salt, and particularly in form of a sodium salt, a triethylamine salt or a diisopropylamine salt.

Processes for the preparation of a compound of formula (I') According to one aspect, there is provided a process for the preparation of a compound of formula (I') 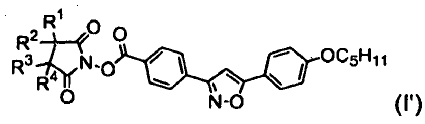 wherein R¹, R², R³ and R⁴ are as defined herein; wherein the process comprises the step of reacting a compound of formula (III') or a salt thereof 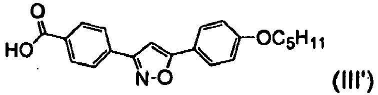 with a compound of formula (IV') 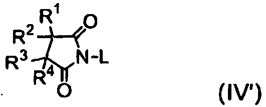 wherein L is a group to be formally substituted in the reaction of the compound of formula (III') or a salt thereof with the compound of formula (IV'). It will be understood that L is selected to be a group which is suitable to be formally substituted during the process as described above. L is typically hydroxyl. Examples for the compounds of formula (IV') are N-hydroxysuccinimide (HOSU), and N-hydroxyphthalimide (HOPHT). Such a process may have the advantage that expensive and problematic reagents can be avoided compared to the processes to obtain the benzotriazole active ester of 4-{5-[4-(pentyloxy)phenyl]-3-isooxazyl}benzoic acid (PPIB-OBT) as described in the art, while providing yields in the same range. The compound of formula (IV') is typically present in an amount of 1.05-3 equivalent, particularly of 1.1-2.5 equivalent, and more particularly 1.1-1.9 equivalent, with respect to the compound of formula (III') or a salt thereof. The step is typically carried out in the presence of a coupling agent, particularly a carbodiimide, particularly selected from 1-ethyl-3-(3-dimethylaminopropyl) carbodiimide (EDCI), N,N'-dicyclohexylcarbodiimide (DCC), N,N'-diisopropylcarbodiimide (DIC), and is particularly EDCI. The coupling agent is then typically present in an amount of 1.05-3 equivalent, particularly of 1.1-2 equivalent, and more particularly 1.1-1.4 equivalent, with respect to the compound of formula (III') or a salt thereof. The step is typically carried out at a temperature of -30°C to 50°C, particularly from -20°C to 10°C, and more particularly -10°C to 0 °C. If the step is carried out in a solvent, the solvent is particularly an aprotic solvent, particularly selected from tetrahydrofuran (THF), dimethylsulfoxide (DMSO), dimethylformamide (DMF), toluene, 2-methyltetrahydrofuran, and any combination thereof, and is particularly a combination of DMF and THF. According to a preferred embodiment, there is provided a process for the preparation of a compound of formula (Ia') 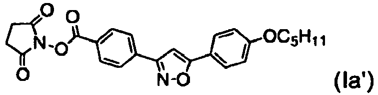 wherein the process comprises the step of reacting a compound of formula (III') or a salt thereof 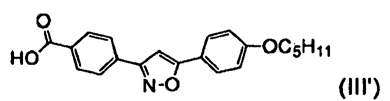 with N-hydroxysuccinimide, present in an amount of 1.1 equivalents, in the presence of 1.2 equivalents 1-ethyl-3-(3-dimethylaminopropyl) carbodiimide, each with respect to the compound of formula (III') or a salt thereof; at room temperature in a mixture of DMF and THF. According to another preferred embodiment, there is provided a process for the preparation of a compound of formula (Ib') 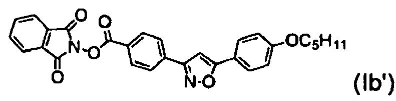 wherein the process comprises the step of reacting a compound of formula (III') or a salt thereof 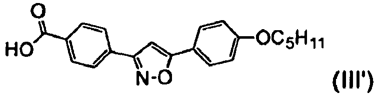 with N-hydroxyphthalimide, present in an amount of 1.2 equivalents, in the presence of 1.4 equivalents 1-ethyl-3-(3-dimethylaminopropyl) carbodiimide, each with respect to the compound of formula (III') or a salt thereof; at room temperature in a mixture of DMF and THF. According to a further embodiment, the process of reacting a compound of formula (III') or a salt thereof 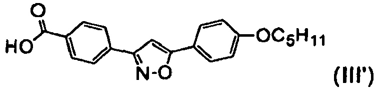 with a compound of formula (IV') 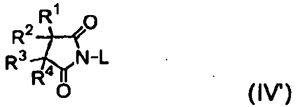 wherein L is a group to be formally substituted in the reaction of the compound of formula (III') or a salt thereof with the compound of formula (IV'), comprises or consists of the steps of (a) reacting the compound of formula (III') or a salt thereof with an activating agent to obtain an activated compound of formula (III') or a salt thereof, and then (b) reacting the activated compound of formula (III') or a salt thereof with the compound of formula (IV'). Such a process may have the advantage that expensive and problematic reagents can be avoided compared to the processes to obtain the benzotriazole active ester of 4-{5-[4-(pentyloxy)phenyl]-3-isooxazyl}benzoic acid (PPIB-OBT) as described in the art, while providing yields in the same range. In addition, the use of expensive EDCI may be avoided. The activating agent is typically selected from oxalyl chloride, thionyl chloride, carbonyl dichloride, phosphorous(III') chloride, phosphorous(V') chloride, phosphorous(V) bromide, and cyanuric chloride. The coupling agent is typically present in an amount of 1.05-3 equivalent, particularly of 1.5-2.5 equivalent, and more particularly 1.8-1.9 equivalent, with respect to the compound of formula (III') or a salt thereof. The step is then typically carried out at a temperature of -30°C to 30°C, particularly from -10°C to 10°C, and more particularly -5 °C to 0 °C. If the step is carried out in a solvent, the solvent is particularly an aprotic solvent, particularly selected from tetrahydrofuran (THF), dimethylsulfoxide (DMSO), dimethylformamide (DMF), toluene, 2-methyltetrahydrofuran, and any combination thereof, and is particularly a combination of DMF and THF. The step can then be carried out in the presence of a base. The base is typically present in an amount of 4-7 equivalent, particularly of 5-6.5 equivalent, and more particularly 5.5-6.2 equivalent, with respect to the compound of formula (III') or a salt thereof. The base is then typically selected from tertiary amines, particularly triethylamine and di-isopropylethylamine; cyclic tertiary amines, particularly N-methylpiperidine; pyridines, particularly pyridine, and any combination thereof. The compound of formula (III') may be used in form of salt. According to a preferred embodiment, there is provided a process for the preparation of a compound of formula (Ia') 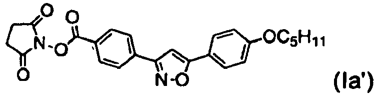 wherein the process comprises the steps of (a) reacting a compound of formula (III') or a salt thereof 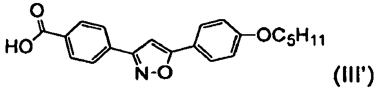 with oxalyl chloride, present in an amount of 1.9 equivalents with respect to the compound of formula (III') or a salt thereof; at a temperature of 0 °C in DMF, to obtain an activated compound of formula (III') or a salt thereof, and (b) reacting the activated compound of formula (III') or a salt thereof with *N*-Hydroxysuccinimide, present in an amount of 1.9 equivalents, in the presence of 5.9 equivalents triethylamine, each with respect to the compound of formula (III') or a salt thereof; at room temperature in DMF. According to another preferred embodiment, there is provided a process for the preparation of a compound of formula (Ib') 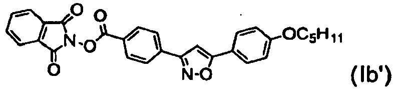 wherein the process comprises the steps of (a) reacting a compound of formula (III') or a salt thereof 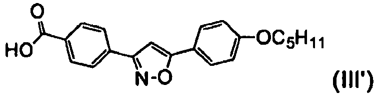 with oxalyl chloride, present in an amount of 1.9 equivalents with respect to the compound of formula (III') or a salt thereof; at a temperature of 0 °C in DMF, to obtain an activated compound of formula (III') or a salt thereof and (b) reacting the activated compound of formula (III') or a salt thereof with N-Hydroxyphthalimide, present in an amount of 1.9 equivalents, in the presence of 6.2 equivalents triethylamine, each with respect to the compound of formula (III') or a salt thereof; at room temperature in DMF.

Processes for the preparation of an antifungal agent According to a further aspect, there is provided a process for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9.13}]heptacosan-6-yl]-1,2-dihydroxyethyl]-2-hydroxyphenyl}oxidanesulfonic acid or a salt thereof, comprising a process to obtain a compound of formula (I') as defined herein. Preferably, the process to obtain a compound of formula (I') is a process as defined above. The process for the preparation of an antifungal agent typically comprises the step of reacting the obtained compound of formula (I') with a compound of formula (II') or a salt thereof

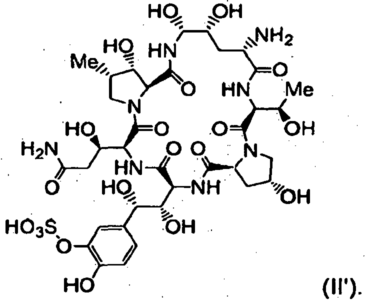

According to all aspects and embodiments, the compound of formula (II') may be used in form of a salt, particularly in form of a sodium salt, a potassium salt, an ammonium salt, an ethanolamine salt, an alkylamine salt, and particularly in form of a sodium salt, a triethylamine salt or a diisopropylamine salt.

**Part C:** Compounds of formula (I") According to one aspect the present invention relates to a compound of formula (I") 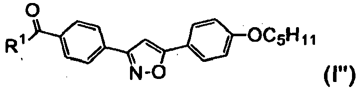 wherein R¹ is selected from Z-S- residues, wherein Z is selected from alkyl residues, particularly having from 1 to 12 carbon atoms, wherein the alkyl residues are optionally aryl and/or aryloxy substituted; aryl residues, particularly having from 6 to 24 carbon atoms, wherein the aryl residues are optionally alkyl and/or alkyloxy substituted; heterocyclic residues; particularly having 2 to 14 carbon atoms; particularly having 1 to 4 heteroatoms as ring component atoms, more particularly 2 to 3 heteroatoms; wherein the heteroatoms are same or different, and are independently selected from N, O and S atoms; wherein the heterocyclic residues are optionally alkyl, aryl, alkyloxy, aryloxy and/or oxo substituted; wherein the heterocyclic residues are optionally condensed with a hydrocarbon ring. The alkyl residues may be selected from methyl, ethyl, propyl, cyclopropyl, *iso*butyl, pentyl, hexyl, *i*sopropyl, *iso*pentyl, and *tert*-butyl. The aryl substituted alkyl residues may be selected from benzyl, p-methylbenzyl, phenylpropyl and naphthylmethyl. The aryl residues may be typically selected from phenyl, 1-naphthyl, 2-naphthyl, biphenylyl, 1-anthracenyl, 2-anthracenyl, and 9-anthracenyl. The heterocyclic residues are typically selected from saturated heterocyclic residues, unsaturated heterocyclic residues, and aromatic heterocyclic residues, and can comprise at least one N atom as ring component atom and/or at least one O atom as ring component atom and/or at least one S atom as ring component atom. The heterocyclic residues can be 5 membered, 6 membered or 7 membered heterocyclic residues. Typically, when the heterocyclic residues are saturated heterocyclic residues, they are selected from 2-oxo-3-oxazolidinyl, tetrahydrofuranyl, tetrahydrothiophenyl, pyrrolidinyl, piperidinyl, tetrahydropyranyl, tetrahydrothiopyranyl, azapanyl, oxepanyl, and thiepanyl. When the heterocyclic residues are unsaturated heterocyclic residues, they are typically selected from imidazolidinyl and thiazinyl. Typically, when the heterocyclic residues are aromatic heterocyclic residues, they are selected from benzothiazo-2-yl, 5-methyl-1,3,4-thiadiazo-2-yl, 1,3,4-thiadiazo-2-yl, pyrrolyl, furyl, thiophenyl, pyridinyl, azepinyl, oxepinyl, thiepinyl, pyrazolyl, triazolyl, imidazolyl, benzimidazolyl, indolyl, isoindolyl, quinolinyl, isoquinolinyl, pyrimidinyl, oxazolyl, and isoxazolyl. The heterocyclic residues as described herein may be substituted, particularly with at least one residue selected from the group of alkyl residues, particularly having from 1 to 12 carbon atoms, wherein the alkyl residues are optionally aryl and/or aryloxy substituted; aryl residues, particularly having from 6 to 24 carbon atoms, wherein the aryl residues are optionally alkyl and/or alkyloxy substituted; alkyloxy residues, having from 1 to 12 carbon atoms, wherein the alkyloxy residues are optionally alkyl and/or aryl substituted; aryloxy residues, having from 6 to 24 carbon atoms, wherein the aryloxy residues are optionally alkyl and/or aryl substituted; and halides, particularly fluoride, chloride, bromide, and iodide. The heterocyclic residues as described herein may be condensed with a hydrocarbon ring, wherein the hydrocarbon ring is selected from non-aromatic or aromatic hydrocarbon rings. Typically, when the ring is an aromatic hydrocarbon ring it is selected from benzyl, naphthyl, biphenylyl, and anthracenylyl rings. When the ring is a non-aromatic hydrocarbon ring it is typically selected from cyclopentyl, cyclohexyl, cycloheptyl, cyclopentenyl, cyclohexenyl and cycloheptenyl rings. The ring may be a 5 membered, a 6 membered or a 7 membered ring. The phenyl residues or the naphthyl residues may be substituted with at least two, three, four, or five electron withdrawing residues. The electron withdrawing residues may be same. The electron withdrawing residues may also be different and are then independently selected from each other. The electron withdrawing residues are typically selected from halides, particularly selected from fluoride, chloride, bromide and iodide, more particularly fluoride; cyano, nitro, trifluoromethyl, carbamoyl, carboxy, alkanoyl, alkoxycarbonyl, and alkylsulphonyl residues. In particularly preferred embodiments, the compound of formula (I") is

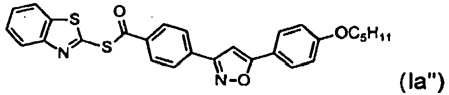

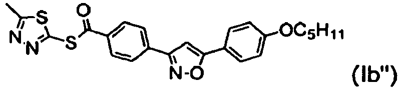

The compound of formula (I") as defined herein can also be present in form of a salt. The compounds of formula (I") as defined herein may have the advantage that they can be obtained by economical improved processes (see below), in particular processes with less demanding synthetic protocols and satisfactory yields comparable to the yields of the prior art. The compounds of formula (I") as defined herein may also have the advantage that they are easy to purify. The compounds of formulae (Ia"), and (Ib") for example are easy to purify due to their low solubility in common solvents. According to a further aspect, any compound of formula (I") as defined herein can be used in a process for the preparation of any other compound of formula (I") as defined herein. According to another aspect, any compound of formula (I") as defined herein can be used in a process for the preparation of compounds suitable as intermediates for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9.13}]heptacosan-6-yl]-1,2-dihydroxyethyl]-2-hydroxyphenyl}oxidanesulfonic acid (MICA), or a salt thereof. According to yet another aspect, any compound of formula (I") as defined herein can be used in a process for the preparation of an antifungal agent, preferably {5-[(1*S*,2*S*)-2 [(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9.13}]heptacosan-6-yl]-1,2-dihydroxyethyl]-2-hydroxyphenyl}oxidanesulfonic acid (MICA), or a salt thereof. According to all aspects and embodiments, MICA may be prepared in form of a salt, particularly in form of a sodium salt, a potassium salt, an ammonium salt, an ethanolamine salt, an alkylamine salt, and particularly in form of a sodium salt, a triethylamine salt or a diisopropylethylamine salt. According to one embodiment of the process for the preparation of an antifungal agent, preferably MICA, or a salt thereof, the process comprises the step of reacting any compound of formula (I") as defined herein with a compound of formula (II") or a salt thereof

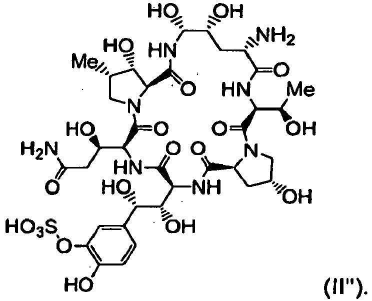

The step is typically carried out at a temperature of -30°C to 50°C, particularly from -20 °C to 30 °C, more particularly 0 °C to 30°C, and most particularly at room temperature. The step is typically carried out in a solvent, particularly an aprotic solvent, particularly selected from tetrahydrofuran (THF), dimethylsulfoxid (DMSO), dimethylformamide (DMF), toluene, 2-methyltetrahydrofuran, *N*-Methyl-2-pyrrolidone (NMP), and any combination thereof, and is particularly DMF. The step can be carried out in the presence of a base. The base is typically present in an amount of 1-50 equivalent, and more particularly 1-10 equivalent, and more particularly 1-3 equivalent, with respect to the compound of formula (II") or a salt thereof. The base is then typically selected from pyridines, lutidines, picolines, dimethylaminopyridine, and collidines, tertiary amines, particularly triethylamine and diisopropylethylamine; cyclic tertiary amines, particularly N-methylpiperidine; aromatic bases, particularly pyridines, lutidines, picolines, dimethylaminopyridine and N-methylmorpholine, and collidines, and any combination thereof; guanidine bases, particularly guanidine and tetramethylguanidine; amidine bases, particularly 1,8-Diazabicyclo[5.4.0]undec-7-en; and any combination thereof. The step may also be carried out in the absence of a base depending on the residue R₁. According to all aspects and embodiments, the compound of formula (II") may be used in form of a salt, particularly in form of a sodium salt, a potassium salt, an ammonium salt, an ethanolamine salt, an alkylamine salt, and particularly in form of a sodium salt, a triethylamine salt or a diisopropylethylamine salt.

Processes for the preparation of a compound of formula (I") According to one aspect, there is provided a process for the preparation of a compound of formula (I") 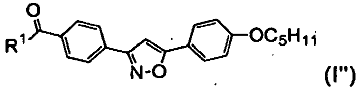 wherein R¹ is a Z-S- residue as defined herein; wherein the process comprises the step of reacting a compound of formula (III") or a salt thereof 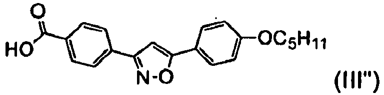 with a compound of formula (IV")

Z-S-L (IV")

wherein L is a group to be formally substituted in the reaction of the compound of formula (III") or a salt thereof with the compound of formula (IV"). It will be understood that L is selected to be suitable to be replaced during the process as described above. L is typically hydrogen or a Z-S- residue. Examples for the compounds of formula (IV") are 2,2'-dithiobis(benzothiazole), 2-mercapto-5-methyl-1,3,4-thiadiazole, 2-mercapto-1,3,4-thiadiazole. Such a process may have the advantage that expensive and problematic reagents can be avoided compared to the processes to obtain the benzotriazole active ester of 4-{5-[4-(pentyloxy)phenyl]-3-isooxazyl}benzoic acid (PPIB-OBT) as described in the art, while providing yields in the same range. The compound of formula (IV") is typically present in an amount of 1.05-3 equivalent, particularly of 1.1-2.0 equivalent, and more particularly 1.4-1.5 equivalent, with respect to the compound of formula (III") or a salt thereof. The step is typically carried out at a temperature of -30 °C to 50 °C, particularly from 10 °C to 30 °C, and more particularly at room temperature. When the step is carried out in a solvent, the solvent is particularly an aprotic solvent, particularly selected from tetrahydrofuran (THF), dimethylsulfoxide (DMSO), dimethylformamide (DMF), toluene, 2-methyltetrahydrofuran, and any combination thereof, and is a combination of DMF and THF. The step may be carried out in the presence of a coupling agent, particularly a carbodiimide, particularly selected from 1-ethyl-3-(3-dimethylaminopropyl) carbodiimide (EDCI), N,N'-dicyclohexylcarbodiimide (DCC), N,N'-diisopropylcarbodiimide (DIC), and is particularly EDCI. The coupling agent is then typically present in an amount of 1.05-3 equivalent, particularly of 1.2-2 equivalent, and more particularly 1.4-1.5 equivalent, with respect to the compound of formula (III") or a salt thereof. The step may be carried out in the presence of an activating agent, particularly a reducing agent, particularly a tertiary phosphine, and is more particularly triphenylphosphine. The activating agent is then typically present in an amount of 1.05-3 equivalent, particularly of 1.2-2 equivalent, and more particularly 1.4-1.5 equivalent, with respect to the compound of formula (III") or a salt thereof. According to a preferred embodiment, there is provided a process for the preparation of a compound of formula (Ia") 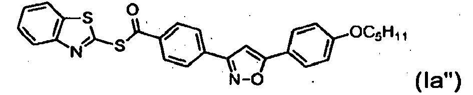 wherein the process comprises the step of reacting a compound of formula (III") or a salt thereof 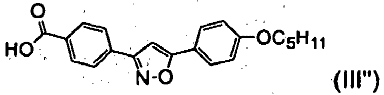 with 2,2'-dithiobis(benzothiazole), present in an amount of 1.5 equivalents, in the presence of 1.5 equivalents triphenylphosphine; each with respect to the compound of formula (III") or a salt thereof; at room temperature in DMF. According to another preferred embodiment, there is provided a process for the preparation of a compound of formula (Ib") 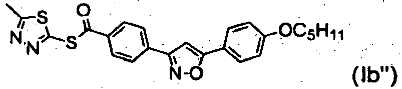 wherein the process comprises the step of reacting a compound of formula (III") or a salt thereof 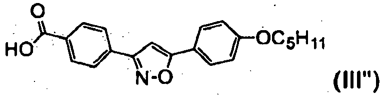 with 2-mercapto-5-methyl-1,3,4-thiadiazole present in an amount of 1.4 equivalents, in the presence of 1.4 equivalents EDCI, each with respect to the compound of formula (III") or a salt thereof; at room temperature in DMF. Also disclosed is a process for the preparation of a compound of formula (I") 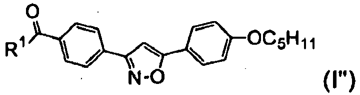 wherein R¹ is an Y-O- residues as defined herein; wherein the process comprises the step of reacting a compound of formula (III") or a salt thereof 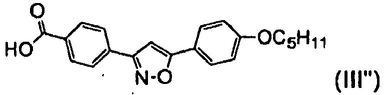 with a compound of formula (V")

Y-O-L (V")

wherein L is a group to be formally substituted in the reaction of the compound of formula (III") or a salt thereof with the compound of formula (V"). It will be understood that L is selected to be suitable to be replaced during the process as described above. L is typically hydrogen. Examples for the compounds of formula (V") are pentafluorophenol, pentachlorophenol, trifluorophenol, and trichlorophenol. Such a process may have the advantage that expensive and problematic reagents can be avoided compared to the processes to obtain the benzotriazole active ester of 4-{5-[4-(pentyloxy)phenyl]-3-isooxazyl}benzoic acid (PPIB-OBT) as described in the art, while providing yields in the same range.The compound of formula (V") is typically present in an amount of 1.05-3 equivalent, particularly of 1.1-2 equivalent, and more particularly 1.1-1.5 equivalent, with respect to the compound of formula (III") or a salt thereof. The step is typically carried out at a temperature of -30°C to 50°C, particularly from 10°C to 30 °C, and more particularly at room temperature. If the step is carried out in a solvent, the solvent is particularly an aprotic solvent, particularly selected from tetrahydrofuran (THF), dimethylsulfoxide (DMSO), dimethylformamide (DMF), toluene, 2-methyltetrahydrofuran, and any combination thereof, and is particularly DMF. The step may be carried out in the presence of a coupling agent, particularly a carbodiimide, particularly selected from 1-ethyl-3-(3-dimethylaminopropyl) carbodiimide (EDCI), N,N'-dicyclohexylcarbodiimide (DCC), N,N'-diisopropylcarbodiimide (DIC), and is particularly EDCI. The coupling agent is then typically present in an amount of 1.05-3 equivalent, particularly of 1.1-2 equivalent, and more particularly 1.1-1.4 equivalent, with respect to the compound of formula (III") or a salt thereof. Further disclosed is a process for the preparation of a compound of formula (Ic") 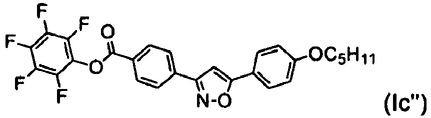 wherein the process comprises the step of reacting a compound of formula (III") or a salt thereof 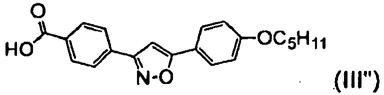 with pentafluorophenol present in an amount of 1.1 equivalents, in the presence of 1.1 equivalents EDCI, each with respect to the compound of formula (III") or a salt thereof; at room temperature in DMF.

Processes for the preparation of an antifungal agent According to a further aspect, there is provided a process for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl)-1,2-oxazol-3-yl)benzene)amido]-1,4,7,13,16,22-hexaazatricyclo[22.3.0.0^{9.13}]heptacosan-6-yl]-1,2-dihydroxyethyl]-2-hydroxyphenyl}oxidanesulfonic acid or a salt thereof, comprising a process to obtain a compound of formula (I") as defined herein. Preferably, the process to obtain a compound of formula (I") is a process as defined above.The process for the preparation of an antifungal agent typically comprises the step of reacting the obtained compound of formula (I") with a compound of formula (II") or a salt thereof

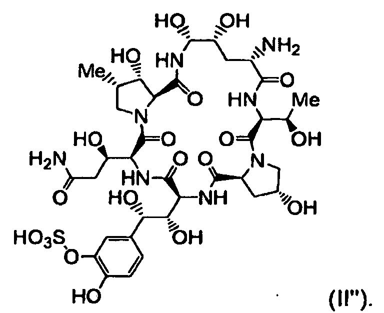

According to all aspects and embodiments, the compound of formula (II") may be used in form of a salt, particularly in form of a sodium salt, a potassium salt, an ammonium salt, an ethanolamine salt, an alkylamine salt, and particularly in form of a sodium salt, a triethylamine salt or a diisopropylethylamine salt.

The invention will be more fully understood by references to the following examples. They should not, however, be construed as limiting the scope of the invention.

### Examples

**Part A:** Example 1: Process for the preparation of MICA in a one-pot synthesis including a process to obtain a compound of formula (Ia). Triethylamine (55 µL, 0.40 mmol) was added to a stirred suspension of PPIB (126 mg, 0.36 mmol) in DMF (6 mL) at 0 °C under N₂-atmosphere. Stirring was continued for 10 minutes and the suspension turned into a cloudy solution. BOP-CI (103 mg, 0.40 mmol) was added in one portion and the reaction mixture was stirred for 35 minutes at 0 °C resulting in a white suspension. CMICA (177 mg, 0.19 mmol) was added in one portion to the suspension followed by triethylamine (55µL, 0.40 mmol). The reaction mixture was stirred for 2.5 hours at 0 °C before being quenched by the addition of 90 µL H₂O. Ethyl acetate was added dropwise under stirring at room temperature. A precipitate formed which was filtered off, washed with ethyl acetate and dried under vacuum to give 316 mg MICA crude.

Example 2: Process for the preparation of MICA in a one-pot synthesis including a process to obtain a compound of formula (Ib). Triethylamine (55 µL, 0.395 mmol) was added to a suspension of PPIB (100 mg, 0.285 mmol) in DMF (2 mL) at -10°C and stirred for 15 minutes. *Iso*butyl chloroformate (41 µL, 0.315 mmol) was added and the reaction mixture was stirred for 30 minutes. A solution of CMICA (245 mg, 0.261 mmol) and triethylamine (55µL, 0.395 mmol) in DMF (1 mL) was added dropwise to the suspension of the mixed anhydride. The reaction mixture was stirred for 40 minutes at -10°C to -5 °C. The suspension turned into an almost clear solution during this period. The reaction mixture was added dropwise to ethyl acetate (10 mL) and the solvent was evaporated in a rotary evaporator. Ethyl acetate (10 mL) was added dropwise to the residue and the resulting suspension was stirred overnight. The precipitate was filtered off (suction filter, Type 3) and washed with ethyl acetate. The crude product was dried under vacuum overnight at room temperature. Yield: 272 mg Micafungin-NEt₃ (crude), 76%. Amberlite ion-exchange resin CG 120 I (5 g) / grain size 100-200 / counter ion= sodium, which was slurried 2x with 10 mL MeOH:H₂O=75:25 (v:v) and filtered, was used for purification. The conditioned resin was filled into a column having a diameter of 1.5 cm. 260 mg MICA-NEt₃ (crude) was dissolved in a mixture of MeOH:H₂O = 75:25 (v:v) (7 mL), the slightly cloudy solution was applied on the ion-exchange column, eluted with MeOH:H₂O = 75:25 (v:v) collection fractions of 10 mL. Fractions 1 to 3 were combined and methanol was evaporated in a rotary evaporator. The aqueous MICA-Na solution was lyophilized overnight. Yield: 212 mg Micafungin-Na, = 66%. White, amorphous solid.

¹H NMR (MICA-Na, 300 MHz, DMSO-d6): δ = 8.90 (m, 2H), 8.28 (br d*,J* = 8.0 Hz, 1H), 7.99 - 8.15 (m, 5H), 7.89 (d, *J*= 8.7 Hz, 2H), 7.59 (s, 1 H), 7.29 - 7.50 (series of m, 3H), 7.16 (d, *J* = 8.9 Hz, 2H), 7.09 (m, 1 H), 6.76 - 6.90 (series of m, 3H), 5.58 (d, *J* = 5.7 Hz, 1H), 5.29.(d, *J* = 4.2 Hz, 1H), 4.82 - 5.21 (series of m, 9H), 3.72 - 4.54 (series of m, 16H), 3.23 (m, 1H), 1.88-2.61 (series of m, 7H), 1.79 (m, 2H), 1.42 (m, 4H), 1.13 (d, *J* = 5.8 Hz, 3H), 1.01 (d,*J* = 6.7 Hz, 3H), 0.95 (t, *J* = 7.1 Hz, 3H).

Example 3: Process for the preparation of MICA in a one-pot synthesis including a process to obtain a compound of formula (Ic). Pivalic acid chloride (40 µL, 0.325 mmol) was added to a stirred suspension of PPIB (100 mg, 0.285 mmol) and NEt₃ (55 µL, 0.394 mmol) in DMF (2 mL) at 0 °C. The reaction was stirred for 3 hours at 0 °C. A solution of CMICA (245 mg, 0.261 mmol) and triethylamine (55µL, 0.395 mmol) was added to the suspension of the mixed anhydride and the reaction mixture was stirred for 25 hours at room temperature. The reaction was monitored with HPLC. Ethyl acetate (3 mL) was added dropwise to the reaction mixture and the suspension was stirred for 1 hour at room temperature. The precipitate was filtered off (suction filter, Type 3) and the crude product was dried under vacuum. Yield: 194 mg Micafungin-NEt₃ (crude), 54%.

Example 4: Process for the preparation of MICA in a one-pot synthesis including a process to obtain a compound of formula (Id). Oxalyl chloride (44 µL, 0.52 mmol) was added dropwise to a stirred suspension of PPIB (91 mg, 0.26 mmol) in DMF (3 mL) under N₂-atmosphere at 0 °C. CO and CO₂ evolve immediately and the suspension turns yellow. Stirring was continued for 10 minutes. This suspension was added dropwise (with a syringe) to a stirred solution of CMICA (160 mg, 0.17 mmol) in DMF (2 mL) and pyridine (0.4 mL) at 0 °C under N₂ atmosphere. The reaction mixture was stirred for 30 minutes at 0 °C. Ethyl acetate (30 mL) was added dropwise to the crude product within 60 minutes under stirring at room temperature. After complete addition of ethyl acetate stirring was continued for 90 minutes. A slightly yellow precipitate forms during that period. The precipitate was filtered, washed with ethyl acetate and dried under vacuum to give 229 mg MICA crude.

**Part B:** Example 1a': Process for the preparation of a compound of formula (Ia') (PPIB-OSU); initial activation with EDCI. To a stirred suspension of PPIB (2.00 g, 5.69 mmol) and N-Hydroxysuccinimide (0.72 g, 6.26 mmol) in THF (20 mL) under N₂ atmosphere DMF (8 mL) was added at room temperature and stirring was continued for 15 minutes. EDCI (1.31 g, 6.83 mmol) was added in one portion and the reaction mixture was stirred at room temperature. The progress of the reaction was monitored by HPLC. After 4.5 hours the reaction was quenched by the slow addition of ethyl acetate (60 mL). H₂O (8 mL) was added slowly and the crude product was stirred at 0 °C for one hour. The precipitate was filtered off, washed with H₂O and ethyl acetate, and dried under reduced pressure to give 1.92 g (75% yield) PPIB-OSU. The product can be further purified by re-crystallization from acetone.

Example 1b': Process for the preparation of a compound of formula (Ia') (PPIB-OSU); activation by acid chloride formation. Oxalyl chloride (0.34 mL, 3.99 mmol) was added to a stirred suspension of PPIB (727 mg, 2.07 mmol) in DMF (20 mL) under N₂ atmosphere at 0 °C. CO₂ and CO evolve immediately and the initially white suspension turns into a yellow-orange viscous slurry. The acid chloride suspension was added to a stirred solution of HOSU (443 mg, 3.85 mmol) in DMF (8 mL) with a syringe (2x 5 mL DMF rinse) at 0 °C. NEt₃(1.7 mL, 12.2 mmol) was added slowly and the reaction mixture was stirred for 1.5 hours at room temperature. The reaction was quenched with H₂O (8 mL). Ethyl acetate (150 mL) was added and the organic phase was separated. The aqueous/DMF phase was extracted with ethyl acetate. The organic phases were combined and concentrated under reduced pressure. The resulting slurry (approximately 10 mL) was kept at 0 °C over night and a white precipitate formed during that time. The precipitate was filtered, washed with H₂O and dried under vacuum to give 1.397 g of a slightly yellow solid. The crude product was purified by re-crystallization from acetone (15 mL) to give PPIB-OSU (658 mg, 71% yield) as colorless/white needles (under the microscope).

¹H NMR (300 MHz, CDCl₃): δ = 8.24 (d, *J* = 8.4 Hz, 2H), 8.01 (d, *J* = 8.4 Hz, 2H), 7.77 (d, *J* = 8.8 Hz, 2H), 6.99 (d, *J* = 8.8 Hz), 6.77 (s, 1H), 4.02 (t, *J* = 6.5 Hz, 2H), 2.93 (br. s, 4H), 1.82 (m, 2H), 1.51 - 1.33 (m, 4H), 0.94 (t, *J* = 7.0 Hz, 3H).¹³C NMR (300 MHz, CDCl₃):δ=171.2, 169.1, 161.6, 161.4, 161.0, 135.4, 131.1, 127.5, 127.1, 126.1, 119.6, 114.9, 96.0, 68.2, 28.8, 28.1, 25.7, 22.4, 14.0.

Example 2a': Process for the preparation of a compound of formula (Ib') (PPIB-OPHT); initial activation with EDCI DMF (8 mL) was added to a stirred suspension of PPIB (1.00 g, 2.85 mmol) and HOPHT (0.56 g, 3.43 mmol) in THF (10 mL) under N₂ atmosphere at room temperature. Stirring was continued for 10 minutes. EDCI (0.77 g, 4.02 mmol) was added in one portion and the reaction progress was monitored by HPLC. After complete conversion of the starting material (after 3.5 hours) the reaction was quenched by the slow addition of ethyl acetate (30 mL). H₂O (4 mL) was added dropwise and the crude product was stirred at 0 °C for one hours. The precipitate was filtered off, washed with H₂O and ethyl acetate, and dried under reduced pressure to give 1.2 g (85% yield) PPIB-OPHT.

Example 2b': Process for the preparation of a compound of formula (Ib') (PPIB-OPHT); activation by acid chloride formation. Oxalyl chloride (0.32 mL, 3.76 mmol) was added dropwise to a stirred suspension of PPIB (735 mg, 2.09 mmol) in DMF (20 mL) under N₂ atmosphere at 0 °C. CO₂ and CO evolve immediately and the reaction mixture turns yellow-orange. The suspension is stirred for 10 minutes. A solution of HOPHT (650 mg, 3.98 mmol) in DMF (8 mL) was added dropwise followed by slow addition of NEt₃ (1.8 mL, 12.9-mmol). The orange suspension was stirred for 10 minutes at 0 °C and then for 2.5 hours at room temperature. The reaction was quenched by the slow addition of H₂O (15 mL) and stirred for 10 minutes at room temperature. The precipitate was filtered and washed with a small portion of H₂O. The crude product (1.389 g) was purified by re-crystallization from 15 mL acetone to give PPIB-OPHT (894 mg, 86% yield) as slightly yellow needles (under the microscope).

¹H NMR (300 MHz, CDCI₃): δ=8.29 (d, *J* = 8.4 Hz, 2H), 8.03 (d, *J* = 8.4 Hz, 2H), 7.94 (m, 2H), 7.82 (m, 2H), 7.77 (d, *J* = 8.8 Hz, 2H), 7.00 (d, *J* = 8.8 Hz, 2H), 6.78 (s, 1H), 4.02 (t, *J* = 6.5 Hz, 2H), 1.82 (m, 2H), 1.51 - 1.34 (m, 4H), 0.94 (t, *J* = 7.0 Hz, 3H). ¹³C NMR (300 MHz, CDCl₃): δ = 171.2, 162.3, 162.0, 161.7, 161.0, 135.4, 134.8, 131.2, 129.0, 127.5, 127.1, 126.3, 124.1, 119.6, 115.0, 96.1, 68.2, 28.8, 28.1, 22.4, 14.0.

**Part C:** Example 1": Process for the preparation of a compound of formula (Ia") (PPIB-MBT). To a stirred suspension of 2,2'-dithiobis-(benzothiazole) (710 mg, 2.14 mmol) and triphenylphosphine (560 mg, 2.14 mmol) in DMF (15 mL) PPIB (500 mg, 1.42 mmol) was added at room temperature. The progress of the reaction was monitored by HPLC. After 2 hours conversion was complete. The suspension was filtered (G3 nutsche filter) and washed with CH₂CI₂. The solid was dried overnight under reduced pressure at room temperature. Yield: 670 mg PPIB-MTB, = 95 %. Melting point: 199 °C - 205 °C. Colourless, to a great extent insoluble powder.

¹H NMR (300 MHz, CDCl₃, sample temperature = 50 °C): δ = 7.94 - 8.14 (series of m, 5H), 7.78 (m, 2H), 7.49 (m, 2H), 7.25 (m, 1H), 7.01 (m, 2 H), 6.75 (m, 1H), 4.02 (t, *J =* 6.4 Hz, 2H), 1.82 (m, 2H), 1.45 (m, 4H), 0.96 (t, *J =* 6.8 Hz, 3H).

Example 2": Process for the preparation of a compound of formula (Ib") (PPIB-MMTDA). To a stirred suspension of PPIB (1.00 g, 2.85 mmol) in a mixture of THF (10 mL) und DMF (8 mL) 2-Mercapto-5-methyl-1,3,4-thiadiazole (528 mg, 3.99 mmol) and EDCI (770 mg, 4.02 mmol) was added at room temperature. The reaction was stirred at room temperature and was monitored by HPLC (reaction time approx. 3 hours). Acetic ester (50 mL) was added over a period of 30 min. The solid was filtered off, washed with acetic ester and dried overnight under reduced pressure at room temperature. Yield: 1.22 g PPIB-MMTDA, = 92%. Melting point: 198 °C - 233 °C. Colourless, in common solvents insoluble powder. (no HPLC or NMR measurement possible).

Example 3": Process for the preparation of a compound of formula (Ic") (PPIB-PFP) (not according to invention). A suspension of PPIB (1.00 g, 2.85 mmol), EDCI (600 mg, 3.13 mmol) and pentafluorophenol (580 mg, 3.15 mmol) in DMF (20 mL) was stirred at room temperature for 1.5 hours. The precipitate was filtered and dried. Yield: 1.05 g PPIB-PFP, = 71%. Melting point: 130 °C - 132 °C. White solid.

¹H NMR (300 MHz, CDCl₃): δ = 8.30 (d, *J* = 8.5 Hz, 2H), 8.04 (d, *J* = 8.5 Hz, 2H), 7.78 (d, *J* = 8.8 Hz, 2H), 7.00 (d, *J =* 8.9 Hz, 2H), 6.78 (s, 1H), 4.02 (t, *J* = 6.5 Hz, 2H), 1.82 (m, 2H), 1.34 - 1.51 (m, 4H), 0.94 (t, *J =* 7.0 Hz, 3H).

### List of references

WO 1996011210A1
WO 2004014879
Kanasaki et al., J. Antibiotics 1999, 52, 674.
Tomishima et al., Bioorg. Med. Chem. Lett. 2008, 18, 2886.

The following pages of the description refer to the embodiments of the invention listed as separate items.

### Items of part A:

1. A compound of formula (I) 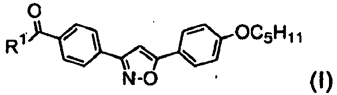 wherein R¹ is selected from:
a) Z-O- residues, wherein Z-O- is selected from
   (v) residues of formula (II) 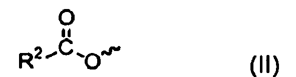
   (vi) residues of formula (III) 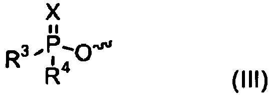 wherein R³ and R⁴ are identical or different; and wherein X is selected from O or S;
   (vii) residues of formula (IV) 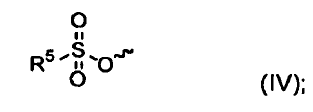 and
   (viii) residues of formula (V): 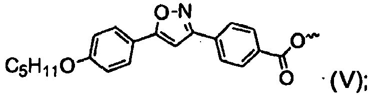
wherein R², R³, R⁴, and R⁵ are independently selected from alkyl residues, particularly having from 1 to 12 carbon atoms, wherein the alkyl residues are optionally aryl and/or aryloxy substituted; aryl residues, particularly having from 6 to 24 carbon atoms, wherein the aryl residues are optionally alkyl and/or alkyloxy substituted; alkyloxy residues, having from 1 to 12 carbon atoms, wherein the alkyloxy residues are optionally alkyl and/or aryl substituted; aryloxy residues, having from 6 to 24 carbon atoms, wherein the aryloxy residues are optionally alkyl and/or aryl substituted; heterocyclic residues; particularly having 2 to 14 carbon atoms; and having 1 to 4 heteroatoms as ring component atoms; wherein the heterocyclic residues are optionally alkyl, aryl, alkyloxy, aryloxy and/or oxo substituted; wherein the heteroatoms are same or different, and independently selected from N, O and S atoms; or (b) a group consisting of halides and pseudohalides. 2. The compound of item 1, wherein the alkyl residues are selected from methyl, ethyl, propyl, cyclopropyl, *iso*butyl, pentyl, hexyl, *iso*propyl, *iso*pentyl, and *tert*-butyl. 3.The compound of item 1 or 2, wherein the aryl substituted alkyl residues are selected from benzyl, p-methylbenzyl, phenylpropyl and naphthylmethyl. 4. The compound of any of items 1 to 3, wherein the aryl residues are selected from phenyl, 1-naphthyl, 2-naphthyl, biphenylyl, 1-anthracenyl, 2-anthracenyl, and 9-anthracenyl. 5. The compound of any of items 1 to 4, wherein the alkyloxy residues are selected from methoxy, ethoxy, propoxy, cyclopropoxy, *iso*butoxy, pentoxy, hexoxy, *iso*propoxy, *iso*pentoxy, and *tert*-butoxy. 6. The compound of any of items 1 to 5, wherein the aryl substituted alkyloxy residues are selected from benzyloxy, p-methylbenzyloxy, phenylpropyloxy and naphthylmethyloxy. 7. The compound of any of items 1 to 6, wherein the aryloxy residues are selected from phenoxy, 1-naphthoxy, 2-naphthoxy, biphenyloxy, 1-anthracenyloxy, 2- anthracenyloxy, and 9-anthracenyloxy. 8. The compound of any of items 1 to 7, wherein the heterocyclic residues are selected from saturated heterocyclic residues, unsaturated heterocyclic residues, and aromatic heterocyclic residues. 9. The compound of any of items 1 to 8, wherein the heterocyclic residues comprise at least one N atom as ring component atom. 10. The compound of any of items 1 to 9, wherein the heterocyclic residues comprise at least one O atom as ring component atom. 11. The compound of any of items 1 to 10, wherein the heterocyclic residues comprise at least one S atom as ring component atom. 12. The compound of any of items 1 to 11, wherein the heterocyclic residues are 5 membered heterocyclic residues. 13. The compound of any of items 1 to 12, wherein the heterocyclic residues are 6 membered heterocyclic residues. 14. The compound of any of items 1 to 13, wherein the heterocyclic residues are 7 membered heterocyclic residues. 15. The compound of any of items 1 to 8, wherein the heterocyclic residues are saturated heterocyclic residues, and are selected from 2-oxo-3-oxazolidinyl, tetrahydrofuranyl, tetrahydrothiophenyl, pyrrolidinyl, piperidinyl, tetrahydropyranyl, tetrahydrothiopyranyl, azapanyl, oxepanyl, and thiepanyl. 16. The compound of any of items 1 to 8, wherein the heterocyclic residues are unsaturated heterocyclic residues, and are selected from imidazolidinyl and thiazinyl. 17. The compound of items 1 and 8, wherein the heterocyclic residues are aromatic heterocyclic residues, and are selected from benzothiazo-2-yl, 5-methyl- 1,3,4-thiadiazo-2-yl, 1,3,4-thiadiazo-2-yl, pyrrolyl, furyl, thiophenyl, pyridinyl, azepinyl, oxepinyl, thiepinyl, pyrazolyl, triazolyl, imidazolyl, benzimidazolyl, indolyl, isoindolyl, quinolinyl, isoquinolinyl, pyrimidinyl, oxazolyl, and isoxazolyl. 18. The compound of any of the preceding items, wherein Z-O- is a residue of formula (II). 19.The compound of any of items 1 to 17, wherein Z-O- is a residue of formula (III). 20. The compound of any of items 1 to 17 and 19, wherein R³ and R⁴ are identical. 21. The compound of any of items 1 to 17 and 19, wherein R³ and R⁴ are different. 22. The compound of any of items 1 to 17, wherein Z-O-is a residue of formula (IV). 23. The compound of item 1, wherein Z-O- is a residue of formula (V). 24. The compound of item 1, wherein R¹ is a halide, particularly selected from fluoride, chloride, bromide and iodide, and is particularly chloride, or wherein R¹ is a pseudohalide, particularly selected from cyanide, azide, cyanate, isocyanate, thiocyanate, isothiocyanate, seleriocyanate, and tellurocyanate. 25. The compound of item 1, wherein the compound of formula (I) is

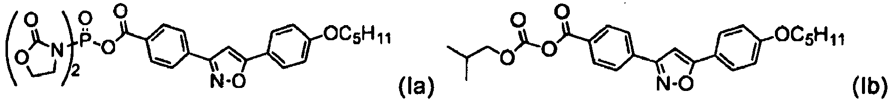

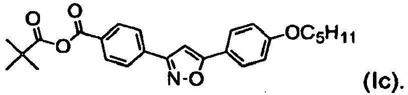

26. The compound of item 1, wherein the compound of formula (I) is

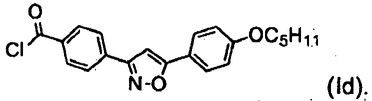

27. The compound of any of the preceding items in form of a salt. 28. Use of a compound of any of the preceding items in a process for the preparation of another compound of any the preceding items. 29. Use of a compound of any of items 1 to 27 in a process for the preparation of compounds suitable as intermediates for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid, or a salt thereof. 30. Use of a compound of any of items 1 to 27 in a process for the preparation of an antifungal agent, preferably {5-{(1*S*,2*S*)-2-[(3*S*,6*S*,9*S*,11*R*,15*S*,18*S*,20*R*,21*R*,24*S*,25*S*,26*S*)-3-[(1*R*)-2-carbarnoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid, or a salt thereof 31. The use of item 30, wherein the process comprises the step of reacting the compound of any of items 1 to 27 with a compound of formula (VI) or a salt thereof

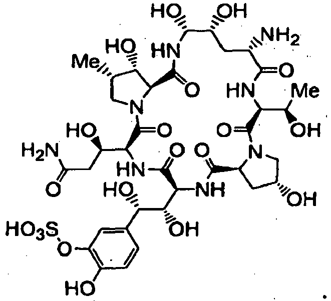

32. The use of item 31, wherein the step is carried out at a temperature of -30 °C to 50 °C, particularly from -20 °C to 30 °C, and more particularly -10 °C to room temperature. 33. The use of item 31 or 32, wherein the step is carried out in a solvent, particularly an aprotic solvent. 34. The use of item 33, wherein the solvent is selected from tetrahydrofuran (THF), dimethylformamide (DMF), toluene, 2-methyltetrahydrofuran, *N*-Methyl-2-pyrrolidone (NMP), and any combination thereof, and is particularly DMF. 35. The use of any of items 31 to 34, wherein in the step carried out in the presence of a base. 36, The use of item 35, wherein the compound is as defined in any of items 24, 26 and 27, and the base is present in an amount of 1-50 equivalent, particularly of 20-40 equivalent, and more particularly 30-35 equivalent, with respect to the compound of formula (VI) or a salt thereof. 37. The use of item 35 or 36, wherein the base is selected from pyridines, lutidines, picolines, dimethylaminopyridine, and collidines, and any combination thereof. 38. The use of item 35, wherein the compound is as defined in any of items 2 to 23, 25 and 27, and the base is present in an amount of 1.0-4.0 equivalent, particularly of 1.2-3.0 equivalent, and more particularly 1.5-2.1 equivalent, with respect to the compound of formula (VI) or a salt thereof. 39. The use of item 38, wherein the base is selected from tertiary amines, particularly triethylamine and diisopropylethylamine; cyclic tertiary amines, particularly N-methylpiperidine and N-methylmorpholine; aromatic bases, particularly pyridines, lutidines, picolines, dimethylaminopyridine, and collidines, and any combination thereof; guanidine bases, particularly guanidine and tetramethylguanidine, amidine bases, particularly 1,8-Diazabicyclo[5.4.0]undec-7-en; and any combination thereof. 40. The use of any of items 31 to 34, wherein the compound is as defined in any of items 2 to 23, 25 and 27 and the process is carried out in the absence of a base.41.A process for the preparation of a compound of formula (I) 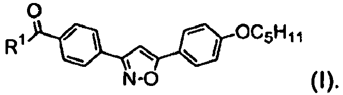 wherein R¹ is a Z-O- residue as defined in any of items 1 to 23, 25 and 27; wherein the process comprises the step of reacting a compound of formula (VII) or a salt thereof 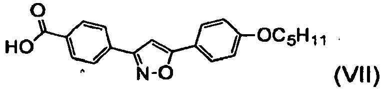 with a compound of formula (VIII)

Z-L (VIII)

wherein L is a leaving group. 42. The process of item 41, wherein L is selected from halides, particularly chloride; alkyl sulfonate; fluoroalkyl sulfonate; aryl sulfonate; fluorosulfonate; nitrate; alkyl phosphate; alkyl borate; trialkylammonium; dialkylsulfonium; 2,4,5-trichlorophenoxy; 2,4-dinitrophenoxy; succinimido-N-oxy; and imidazolyl. 43.The process of item 41, wherein compound of formula (VIII) is selected from methyl chloroformate, ethyl chloroformate, propyl chloroformate, cyclopropyl chloroformate, *iso*butyl chloroformate, pentyl chloroformate, hexyl chloroformate, *iso*propyl chloroformate, *iso*pentyl chloroformate, *tert*-butyl chloroformate, pivalic acid chloride, and bis(2-oxo-3-oxazolidinyl)phosphonic chloride. 44. The process of any of items 41 to 43, wherein the compound of formula (VIII) is present in an amount of 1.05-3 equivalent, particularly of 1.1-2 equivalent, and more particularly 1.1-1.4 equivalent, with respect to the compound of formula (VII) or a salt thereof. 45. The process of any of items 41 to 44, wherein the step is carried out in the presence of a base. 46.The process of item 45, wherein the base is selected from tertiary amines, particularly triethylamine and diisopropylethylamine; cyclic tertiary amines, particularly N-methylpiperidine; aromatic bases, particularly pyridines, lutidines, picolines, dimethylaminopyridine, and collidines, and any combination thereof; guanidine bases, particularly guanidine and tetramethylguanidine; amidine bases, particularly 1,8-Diazabicyclo[5.4.0]undec-7-en; and any combination thereof. 47. The process of item 45 or 46, wherein the base is present in an amount of 1.05-3 equivalent, particularly of 1.1-2 equivalent, and more particularly 1.1-1.4 equivalent, with respect to the compound of formula (VII) or a salt thereof. 48. The process of any of items 41 to 47, wherein the step is carried out at a temperature of -30 °C to 50 °C, particularly from -20 °C to 10 °C, and more particularly -10 °C to 0 °C. 49. The process of any of items 41 to 48, wherein the step is carried out in a solvent, particularly an aprotic solvent. 50. The process of item 49, wherein the solvent is selected from tetrahydrofuran (THF), dimethylformamide (DMF), toluene, 2-methyltetrahydrofuran, and any combination thereof, and is particularly DMF. 51.The process of item 41 for the preparation of a compound of formula (Ia) 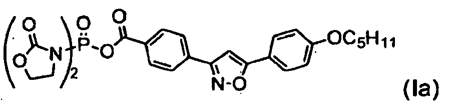 wherein the process comprises the step of reacting a compound of formula (VII) or a salt thereof with bis(2-oxo-3-oxazolidinyl)phosphonic chloride present in an amount of 1.1 equivalents, in the presence of 1.1 equivalents triethylamine, each with respect to the compound of formula (VII) or a salt thereof; at a temperature of 0 °C in DMF. 52. The process of item 41 for the preparation of a compound of formula (Ib) 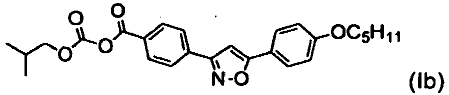 wherein the process comprises the step of reacting a compound of formula (VII) or a salt thereof with *iso*butyl chloroformate present in an amount of 1.1 equivalents, in the presence of 1.4 equivalents triethylamine, each with respect to the compound of formula (VII) or a salt thereof; at a temperature of - 10 °C in DMF. 53. The process of item 41 for the preparation of a compound of formula (Ic) 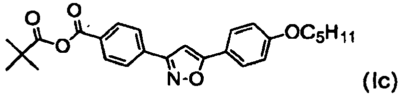 wherein the process comprises the step of reacting a compound of formula (VII) or a salt thereof with pivalic acid chloride present in an amount of 1.1 equivalents, in the presence of 1.4 equivalents triethylamine, each with respect to the compound of formula (VII) or a salt thereof; at a temperature of 0 °C in DMF. 54. A process for the preparation of a compound of formula (I) 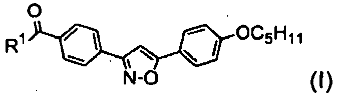 wherein R¹ is selected from a group consisting of halides and pseudohalides; wherein the process comprises the step of reacting a compound of formula (VII) or a salt thereof 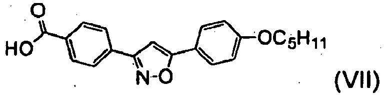 with a reagent capable of halogenating or pseudohalogenating a carboxylic acid. 55. The process of item 54, wherein the halide is selected from fluoride, chloride, bromide and iodide, and is particularly chloride. 56. The process of item 54, wherein the halide is chloride and the reagent capable of halogenating a carboxylic acid is selected from thionyl chloride, oxalyl chloride, carbonyl dichloride, cyanuric chloride, phosphorous(III) chloride, and phosphorous(V) chloride.57. The process of item 54, wherein the halide is iodide and the reagent capable of halogenating a carboxylic acid is selected from iodide and acetyl iodide. 58. The process of item 54, wherein the halide is bromide and the reagent capable of halogenating a carboxylic acid is phosphorus(V) bromide. 59. The process of item 54, wherein the halide is fluoride and the reagent capable of halogenating a carboxylic acid is cyanuric fluoride. 60. The process of any of items 54 to 59, wherein the reagent capable of halogenating or pseudohalogenating a carboxylic acid is present in an amount of 1.5-10 equivalent, particularly of 1.7-8 equivalent, and more particularly 2-4 equivalent, with respect to the compound of formula (VII) or a salt thereof. 61. The process of any of items 54 to 60, wherein the step is carried out at a temperature of -30 °C to 50 °C, particularly from -10 °C to 10 °C, and more particularly 0 °C. 62. The process of any of items 54 to 61, wherein the step is carried out in a solvent, particularly an aprotic solvent. 63. The process of item 62, wherein the solvent is selected from tetrahydrofuran (THF), dimethylformamide (DMF), toluene, 2-methyltetrahydrofuran, and any combination thereof, and is particularly DMF. 64. The process of item 54 for the preparation of a compound of formula (Id) 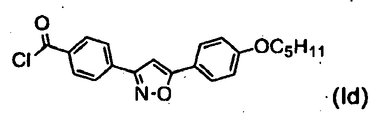 wherein the process comprises the step of reacting a compound of formula (VII) or a salt thereof with oxalyl chloride in an amount of 2 equivalents with respect to the compound of formula (VII) or a salt thereof, at a temperature of 0 °C in DMF. 65. A process for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21;25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid, or a salt thereof, comprising a process to obtain a compound of any of items 1 to 27, preferably by a process as defined in any of items 41 to 64. 66. The process of item 65, comprising the step of reacting the obtained compound with a compound of formula (VI) or a salt thereof. 67. The process of item 65, comprising the step of reacting the obtained compound without purification, with a compound of formula (VI) or a salt thereof. 68. The process of item 65, comprising the step of reacting a compound of formula (VII) or a salt thereof with bis(2-oxo-3-oxazolidinyl)phosphonic chloride present in an amount of 1.1 equivalents, in the presence of 1.1 equivalents triethylamine, each with respect to the compound of formula (VII) or a salt thereof; at a temperature of 0 °C in DMF; to obtain a compound of formula (Ia) 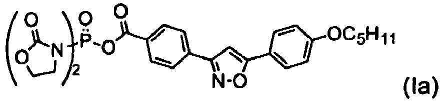 reacting the compound of formula (Ia) without purification, with a compound of formula (VI) or a salt thereof in the presence of 2.1 equivalents triethylamine, with respect to the compound of formula (VI) or a salt thereof; at a temperature of 0 °C in DMF. 69. The process of item 65, comprising the step of reacting a compound of formula (VII) or a salt thereof with *iso*butyl chloroformate present in an amount of 1.1 equivalents, in the presence of 1.4 equivalents triethylamine, each with respect to the compound of formula (VII) or a salt thereof; at a temperature of -10 °C in DMF to obtain a compound of formula (Ib) 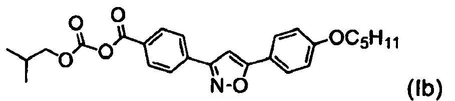 reacting the compound of formula (Ib) without purification, with a compound of formula (VI) or a salt thereof, in the presence of 1.5 equivalents triethylamine, with respect to the compound of formula (VI) or a salt thereof; at a temperature of -10 °C to -5 °C in DMF.

70. The process of item 65, comprising the step of reacting a compound of formula (VII) or a salt thereof with pivalic acid chloride present in an amount of 1.1 equivalents, in the presence of 1.4 equivalents triethylamine, each with respect to the compound of formula (VII) or a salt thereof; at a temperature of 0 °C in DMF; to obtain a compound of formula (Ic) 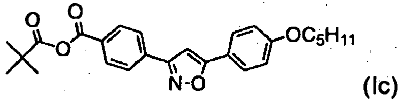 reacting the compound of formula (Ic) without purification, with a compound of formula (VI) or a salt thereof in the presence of 1.5 equivalents triethylamine, with respect to the compound of formula (VI) or a salt thereof; at room temperature in DMF. 71. The process of item 65, comprising the step of reacting a compound of formula (VII) or a salt thereof with oxalyl chloride in an amount of 2 equivalents with respect to the compound of formula (VII) or a salt thereof, at a temperature of 0 °C in DMF; to obtain a compound of formula (Id) 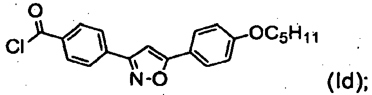 reacting the compound of formula (Id) without purification, with a compound of formula (VI) or a salt thereof in the presence of 29 equivalents pyridine, with respect to the compound of formula (VI) or a salt thereof; at a temperature of 0 °C in DMF.

### Items of part B:

1. A compound of formula **(I')** 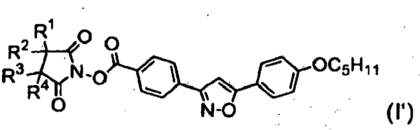 wherein R¹, R², R³ and R⁴ are same or different, and are independently selected from: hydrogen; alkyl residues, particularly having from 1 to 12 carbon atoms, wherein the alkyl residues are optionally aryl and/or aryloxy substituted; aryl residues, particularly having from 6 to 24 carbon atoms, wherein the aryl residues are optionally alkyl and/or alkyloxy substituted; alkyloxy residues, having from 1 to 12 carbon atoms, wherein the alkyloxy residues are optionally alkyl and/or aryl substituted; aryloxy residues, having from 6 to 24 carbon atoms, wherein the aryloxy residues are optionally alkyl and/or aryl substituted; heterocyclic residues; particularly having 2 to 14 carbon atoms; and having 1 to 4 heteroatoms as ring component atoms; wherein the heterocyclic residues are optionally alkyl, aryl, alkyloxy, aryloxy and/or oxo substituted; wherein the heteroatoms are same or different, and independently selected from N, O and S atoms; and wherein one of R¹ and R², and one of R³ and R⁴ are optionally fused to form a non-aromatic ring, wherein the other two are as defined above or form a bond; or an aromatic ring; or a bond, wherein the other two are as defined above. 2. The compound of item 1, wherein the alkyl residues are selected from methyl, ethyl, propyl, cyclopropyl, *iso*butyl, pentyl, hexyl, *iso*propyl, *iso*pentyl, and *tert-*butyl. 3. The compound of item 1 or 2, wherein the aryl substituted alkyl residues are selected from benzyl, p-methylbenzyl, phenylpropyl and naphthylmethyl. 4. The compound of any of items 1 to 3, wherein the aryl residues are selected from phenyl, 1-naphthyl; 2-naphthyl, biphenylyl, 1-anthracenyl, 2-anthracenyl, and 9-anthracenyl. 5. The compound of any of items 1 to 4, wherein the alkyloxy residues are selected from methoxy, ethoxy, propoxy, cyclopropoxy, *iso*butoxy, pentoxy, hexoxy, *iso*propoxy, *iso*pentoxy, and *tert*-butoxy.

6. The compound of any of items 1 to 5, wherein the aryl substituted alkyloxy residues are selected from benzyloxy, p-methylbenzyloxy, phenylpropyloxy and naphthylmethyloxy. 7. The compound of any of items 1 to 6, wherein the aryloxy residues are selected from phenoxy, 1-naphthoxy, 2-napihthoxy, biphenyloxy, 1-anthracenyloxy, 2-anthracenyloxy, and 9-anthracenyloxy. 8. The compound of any of items 1 to 7, wherein R¹, R², R³ and R⁴ are same, particularly hydrogen; or wherein three of R¹, R², R³ and R⁴ are same, particularly hydrogen, or wherein two of R¹, R², R³ and R⁴ are same, particularly hydrogen, particularly wherein one of R¹ and R² and one of R³ and R⁴ are same, and the others are different and are independently selected from each other. 9. The compound of any of items 1 to 7. wherein R¹, R², R³ and R⁴ are different and are independently selected from each other. 10. The compound of item 1, wherein one of R¹ and R² is fused with R³ to form a ring, wherein the ring is selected from a saturated ring, an unsaturated ring, and an aromatic ring. 11. The compound of item 1 or 10, wherein when one of R¹ and R² is fused with R⁴ to form a ring, wherein the ring is selected from a saturated ring, an unsaturated ring, and an aromatic ring.

12. The compound of any of items 1, 10 and 11, wherein the ring is a 5 membered ring.

13. The compound of any of items 1, 10 and 11, wherein the ring is a 6 membered ring.

14. The compound of any of items 1, 10 and 11, wherein the ring is a 7 membered ring.

15. The compound of any of items 1, and 10 to 14, wherein the ring is substituted, particularly with at least one residue selected from the group of alkyl residues, particularly having from 1 to 12 carbon atoms, wherein the alkyl residues are optionally aryl and/or aryloxy substituted; aryl residues, particularly having from 6 to 24 carbon atoms, wherein the aryl residues are optionally alkyl and/or alkyloxy substituted; alkyloxy residues, having from 1 to 12 carbon atoms, wherein the alkyloxy residues are optionally alkyl and/or aryl substituted; aryloxy residues, having from 6 to 24 carbon atoms, wherein the aryloxy residues are optionally alkyl and/or aryl substituted; and halides, particularly fluoride, chloride, bromide, and iodide. 16. The compound of any of items 1, 10 and 11, wherein the ring is an aromatic ring and selected from benzene, naphthylene, biphenylene, and anthracenylene. 17. The compound of any of items 1, 10 and 11, wherein the ring is a saturated ring and selected from cyclopentyl, cyclohexyl and cycloheptyl. 18. The compound of any of items 1, 10 and 11, wherein the ring is a non-saturated ring and selected from cyclopentenyl, cyclohexenyl and cycloheptenyl. 19. The compound of any of items 1, and 10 to 15, wherein the ring is a heterocyclic ring. 20. The compound of any of items 1, 15 and 19, wherein the ring is a heterocyclic ring selected from a saturated heterocyclic ring, an unsaturated heterocyclic ring, and an aromatic heterocyclic ring. 21. The compound of item 19 or 20, wherein the heterocyclic ring comprises at least one N atom as ring component atom. 22. The compound of any of items 19, 20 and 21, wherein the heterocyclic ring comprises at least one O atom as ring component atom. 23. The compound of any of items 19 and 20 to 22, wherein the heterocyclic ring comprises at least one S atom as ring component atom. 24. The compound of any of items 19 to 23, wherein the heterocyclic ring is 5 membered. 25. The compound of any of items 19 to 23, wherein the heterocyclic ring is 6 membered. 26. The compound of any of items 19 to 23, wherein the heterocyclic ring is 7 membered. 27.The compound of item 19, wherein the heterocyclic ring is a saturated heterocyclic ring, and is selected from 2-oxo-3-oxazolin, tetrahydrofuran, tetrahydrothiophene, pyrrolidine, piperidine, tetrahydropyran, tetrahydrothiopyran, azapane, oxepane, and thiepane. 28. The compound of item 19, wherein the heterocyclic ring is an unsaturated heterocyclic ring, and is selected from imidazoline and thiazine. 29.The compound of item 19, wherein the heterocyclic ring is an aromatic heterocyclic ring, and is selected from pyrrole, furan, thiophene, pyridine, azepine, oxepin, thiepin, pyrazole, triazole, imidazole, benzimidazole, indole, isoindole, quinoline, isoquinoline, pyrimidine, oxazole, and isoxazole. 30. The compound of item 1, wherein the heterocylic residues are as defined in any of items 20 to 29. 31. The compound of item 1, wherein the compound of formula (I') is

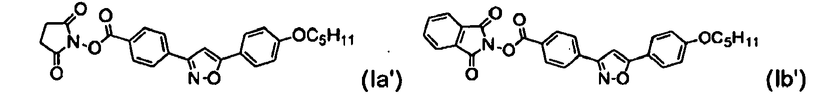

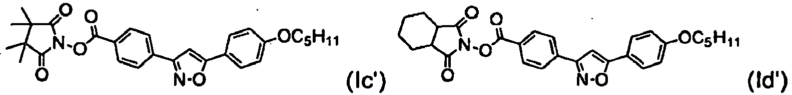

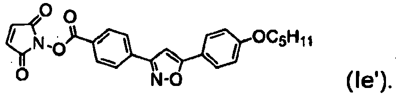

32. The compound of any of the preceding items in form of a salt. 33. Use of a compound of any of the preceding items in a process for the preparation of another compound of any the preceding items. 34. Use of a compound of any of items 1 to 32 in a process for the preparation of compounds suitable as intermediates for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[{1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof. 35. Use of a compound of any of items 1 to 32 in a process for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof. 36. The use of item 35, wherein the process comprises the step of reacting the compound of any of items 1 to 32 with a compound of formula (II') or a salt thereof.

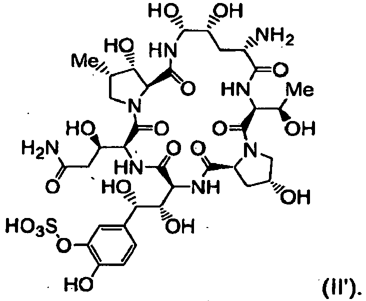

37. The use of item 36, wherein the step is carried out at a temperature of -30 °C to 50 °C, particularly from -20 °C to 30 °C, more particularly 0 °C to 30 °C, and most particularly at room temperature. 38. The use of item 36 or 37, wherein the step is carried out in a solvent, particularly an aprotic solvent. 39. The use of item 38, wherein the solvent is selected from tetrahydrofuran (THF), dimethylsulfoxid (DMSO), dimethylformamide (DMF), toluene, 2-methyltetrahydrofuran, *N-*Methyl-2-pyrrolidone (NMP), and any combination thereof, and is particularly DMF. 40. The use of any of items 36 to 39, wherein the step is carried out in the presence of a base. 41. The use of item 40, wherein the base is present in an amount of 1-50 equivalent, particularly of 1-10 equivalent, and more particularly 1-2 equivalent, with respect to the compound of formula (II') or a salt thereof. 42. The use of item 40 or 41, wherein the base is selected from pyridines, lutidines, picolines, dimethylaminopyridine, triethylamine, isopropylethylamine, N-methylmorpholine, tetramethylguanidine, and collidines, and any combination thereof. 43. The use of any of items 36 to 39, wherein the process is carried out in the absence of a base. 44. A process for the preparation of a compound of formula (I'), 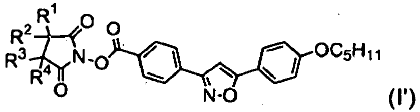 wherein R¹, R², R³ and R⁴ are as defined in any of items 1 to 31; wherein the process comprises the step of reacting a compound of formula (III') or a salt thereof 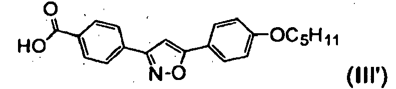 with a compound of formula (IV') 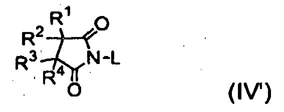 wherein L is a group to be formally substituted in the reaction of the compound of formula (III') or a salt thereof with the compound of formula (IV'). 45. The process of item 44, wherein L is hydroxyl. 46. The process of item 44, wherein the compound of formula (IV') is selected from N-hydroxysuccinimide, and N-hydroxyphthalimide. 47. The process of any of items 44 to 46, wherein the compound of formula (IV') is present in an amount of 1.05-3 equivalent, particularly of 1.1-2.5 equivalent, and more particularly 1.1-1.9 equivalent, with respect to the compound of formula (III') or a salt thereof. 48. The process of any of items 44 to 47, wherein the step is carried out in the presence of a coupling agent. 49. The process of item 48, wherein the coupling agent is a carbodiimide. 50. The process of item 48, wherein the coupling agent is selected from 1-ethyl-3-(3-dimethylaminopropyl) carbodiimide (EDCI), N,N'-dicyclohexylcarbodiimide (DCC), N,N'-diisopropylcarbodiimide (DIC), and is particularly EDCI. 51.The process of any of items 48 to 50, wherein the coupling agent is present in an amount of 1.05-3 equivalent, particularly of 1.1-2 equivalent, and more particularly 1.2-1.4 equivalent, with respect to the compound of formula (III') or a salt thereof. 52. The process of any of items 44 to 51, wherein the step is carried out at a temperature of -30 °C to 50 °C, particularly from -10 °C to 30 °C, and more particularly room temperature. 53. The process of any of items 44 to 52, wherein the step is carried out in a solvent, particularly an aprotic solvent. 54. The process of item 53, wherein the solvent is selected from tetrahydrofuran (THF), dimethylsulfoxide (DMSO), dimethylformamide (DMF), toluene, 2-methyltetrahydrofuran, and any combination thereof, and is particularly a combination of DMF and THF. 55. The process of item 44 for the preparation of a compound of formula (Ia') 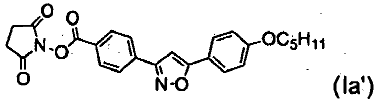 wherein the process comprises the step of reacting a compound of formula (III') or a salt thereof 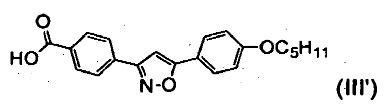 with *N*-hydroxysuccinimide, present in an amount of 1.1 equivalents, in the presence of 1.2 equivalents 1-ethyl-3-(3-dimethylaminopropyl) carbodiimide, each with respect to the compound of formula (III') or a salt thereof; at room temperature in a mixture of DMF and THF. 56. The process of item 44 for the preparation of a compound of formula (Ib') 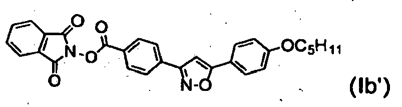 wherein the process comprises the step of reacting a compound of formula (III') or a salt thereof 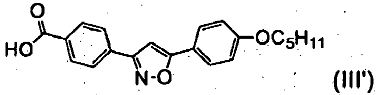 with N-hydroxyphthalimide, present in an amount of 1.2 equivalents, in the presence of 1.4 equivalents 1-ethyl-3-(3-dimethylaminopropyl) carbodiimide, each with respect to the compound of formula (III') or a salt thereof; at room temperature in a mixture of DMF and THF. 57. The process of any of items 44 to 47, wherein the process comprises or consists of the steps of (a) reacting the compound of formula (III') or a salt thereof with an activating agent to obtain an activated compound of formula (III') or a salt thereof, and then (b) reacting the activated compound of formula (III') or a salt thereof with the compound of formula (IV'). 58. The process of item 57, wherein the activating agent is selected from oxalyl chloride, thionyl chloride, carbonyl dichloride, phosphorous(III') chloride, phosphorous(V) chloride, phosphorus(V) bromide, and cyanuric chloride. 59. The process of item 57 or 58, wherein the activating agent is present in an amount of 1.05-3 equivalent, particularly of 1.5-2.5 equivalent, and more particularly 1.8-1.9 equivalent, with respect to the compound of formula (III') or a salt thereof. 60.The process of any of items 57 to 59, wherein step (a) is carried out at a temperature of -30 °C to 30 °C, particularly from -10 °C to 10 °C, and more particularly -5 °C to 0 °C. 61. The process of any of items 57 to 60, wherein step (a) is carried out in a solvent, particularly an aprotic solvent. 62. The process of item 61, wherein the solvent is selected from tetrahydrofuran (THF), dimethylformamide (DMF), toluene, 2-methyltetrahydrofuran, and any combination thereof, and is particularly DMF. 63. The process of any of items 57 to 62, wherein step (b) is carried out at a temperature of -30 °C to 50 °C, particularly from -10 °C to 30 °C, and more particularly room temperature. 64. The process of any of items 57 to 63, wherein step (b) is carried out in a solvent, particularly an aprotic solvent. 65. The process of item 64, wherein the solvent is selected from tetrahydrofuran (THF), dimethylformamide (DMF), toluene, 2-methyltetrahydrofuran, and any combination thereof, and is particularly DMF. 66. The process of any of items 57 to 65, wherein step (b) is carried out in the presence of a base. 67. The process of item 66, wherein the base is selected from tertiary amines, particularly triethylamine and diisopropylethylamine; cyclic tertiary amines, particularly N-methylpiperidine; pyridines, particularly pyridine, and any combination thereof. 68. The process of item 66 or 67, wherein the base is present in an amount of 4-7 equivalent, particularly of 5-6.5 equivalent, and more particularly 5.5-6.2 equivalent, with respect to the compound of formula (III') or a salt thereof. 69.The process of item 57 for the preparation of a compound of formula (Ia') 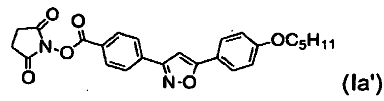 wherein the process comprises the steps of (a) reacting a compound of formula (III') or a salt thereof 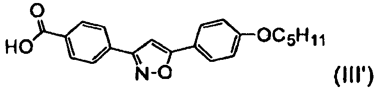 with oxalyl chloride, present in an amount of 1.9 equivalents with respect to the compound of formula (III') or a salt thereof; at a temperature of 0 °C in DMF, to obtain an activated compound of formula (III') or a salt thereof, and (b) reacting the activated compound of formula (III') or a salt thereof with *N-*Hydroxysuccinimide, present in an amount of 1.9 equivalents, in the presence of 5.9 equivalents triethylamine, each with respect to the compound of formula (III') or a salt thereof; at room temperature in DMF. 70.The process of item 57 for the preparation of a compound of formula (Ib') 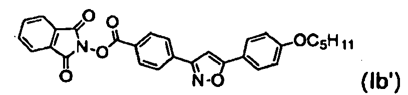 wherein the process comprises the steps of (a) reacting a compound of formula (III') or a salt thereof 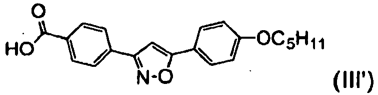 with oxalyl chloride, present in an amount of 1.9 equivalents with respect to the compound of formula (III') or a salt thereof; at a temperature of 0 °C in DMF, to obtain an activated compound of formula (III') or a salt thereof and (b) reacting the activated compound of formula (III') or a salt thereof with N-Hydroxyphthalimide, present in an amount of 1.9 equivalents, in the presence of 6.2 equivalents triethylamine, each with respect to the compound of formula (III') or a salt thereof; at room temperature in DMF. 71. A process for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof, comprising a process to obtain a compound of any of items 1 to 32, preferably by a process as defined in any of items 44 to 68. 72. The process of item 71, comprising the step of reacting the obtained compound with a compound of formula (II') or a salt thereof

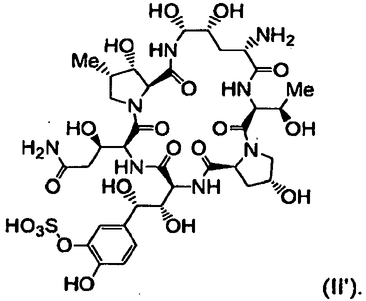

### Items of part C:

1. A compound of formula (I") 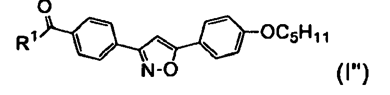 wherein R¹ is selected from Z-S- residues, wherein Z is selected from alkyl residues, particularly having from 1 to 12 carbon atoms, wherein the alkyl residues are optionally aryl and/or aryloxy substituted; aryl residues, particularly having from 6 to 24 carbon atoms, wherein the aryl residues are optionally alkyl and/or alkyloxy substituted; heterocyclic residues; particularly having 2 to 14 carbon atoms; particularly having 1 to 4 heteroatoms as ring component atoms, more particularly 2 to 3 heteroatoms; wherein the heteroatoms are same or different, and are independently selected from N, O and S atoms; wherein the heterocyclic residues are optionally alkyl, aryl, alkyloxy, aryloxy and/or oxo substituted; wherein the heterocyclic residues are optionally condensed with a hydrocarbon ring. 2. The compound of item 1, wherein the alkyl residues are selected from methyl, ethyl, propyl, cyclopropyl, isobutyl, pentyl, hexyl, isopropyl, *iso*pentyl, and *tert*-butyl. 3. The compound of item 1, wherein the aryl substituted alkyl residues are selected from benzyl, p-methylbenzyl, phenylpropyl and naphthylmethyl. 4.The compound of item 1, wherein the aryl residues are selected from phenyl, 1-naphthyl, 2-naphthyl, biphenylyl, 1-anthracenyl, 2-anthracenyl, and 9-anthracenyl. 5. The compound of item 1, wherein the heterocyclic residues are selected from saturated heterocyclic residues, unsaturated heterocyclic residues, and aromatic heterocyclic residues. 6. The compound of item 1 or 5, wherein the heterocyclic residues comprise at least one, preferably two, N atom as ring component atom. 7. The compound of item 1, 5 or 6, wherein the heterocyclic residues comprise at least one, preferably two, O atom as ring component atom. 8. The compound of any of items 1 and 5 to 7, wherein the heterocyclic residues comprise at least one, preferably two, S atom as ring component atom.

9. The compound of any of items 1 and 5 to 8, wherein the heterocyclic residues are 5 membered heterocyclic residues. 10. The compound of any of items 1 and 5 to 8, wherein the heterocyclic residues are 6 membered heterocyclic residues. 11. The compound of any of items 1 and 5 to 8, wherein the heterocyclic residues are 7 membered heterocyclic residues. 12. The compound of item 1 or 5, wherein the heterocyclic residues are saturated heterocyclic residues, and are selected from 2-oxo-3-oxazolidinyl, tetrahydrofuranyl, tetrahydrothiophenyl, pyrrolidinyl, piperidinyl, tetrahydropyranyl, tetrahydrothiopyranyl, azapanyl, oxepanyl, and thiepanyl. 13. The compound of item 1 or 5, wherein the heterocyclic residues are unsaturated heterocyclic residues, and are selected from imidazolidinyl and thiazinyl. 14. The compound of item 1 or 5, wherein the heterocyclic residues are aromatic heterocyclic residues, and are selected from benzothiazo-2-yl, 5-methyl-1,3,4-thiadiazo-2-yl, 1,3,4-thiadiazo-2-yl, pyrrolyl, furyl, thiophenyl, pyridinyl, azepinyl, oxepinyl, thiepinyl, pyrazolyl, triazolyl, imidazolyl, benzimidazolyl, indolyl, isoindolyl, quinolinyl, isoquinolinyl, pyrimidinyl, oxazolyl, and isoxazolyl. 15. The compound of any of items 1 to 11, wherein the heterocyclic residues are substituted, particularly with at least one residue selected from the group of alkyl residues, particularly having from 1 to 12 carbon atoms, wherein the alkyl residues are optionally aryl and/or aryloxy substituted; and aryl residues, particularly having from 6 to 24 carbon atoms, wherein the aryl residues are optionally alkyl and/or alkyloxy substituted. 16. The compound of any of items 1, 5 to 11 and 15, wherein the heterocyclic residues are condensed with a hydrocarbon ring and wherein the hydrocarbon ring is selected from non-aromatic or aromatic hydrocarbon rings. 17. The compound of item 16, wherein the hydrocarbon ring is an aromatic hydrocarbon ring and is selected from benzyl, naphthylyl, biphenylyl, and anthracenylyl. 18. The compound of item 16, wherein the hydrocarbon ring is a non-aromatic hydrocarbon ring and selected from cyclopentyl, cyclohexyl, cycloheptyl, cyclopentenyl, cyclohexenyl and cycloheptenyl. 19. The compound of item 1, wherein the phenyl residues or the naphthyl residues are substituted with at least two, three, four, or five electron withdrawing residues. 20.The compound of item 1 or 19, wherein the electron withdrawing residues are different and independently selected from each other; or wherein the electron withdrawing residues are same. 21. The compound of any of items 1, 19 and 20, wherein the electron withdrawing residues are selected from halides, particularly selected from fluoride, chloride, bromide and iodide, more particularly fluoride; cyano, nitro, trifluoromethyl, carbamoyl, carboxy, alkanoyl, alkoxycarbonyl, and alkylsulphonyl residues. 22. The compound of item 1, wherein the compound of formula (I") is

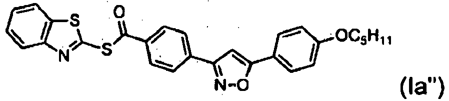

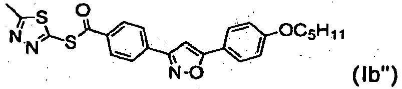

23. Also disclosed is the compound of item 1, wherein the compound of formula (I") is

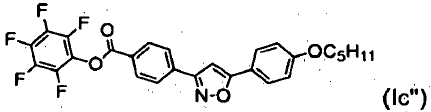

24. The compound of any of the preceding items in form of a salt. 25. Use of a compound of any of the preceding items in a process for the preparation of another compound of any the preceding items. 26. Use of a compound of any of items 1 to 24 in a process for the preparation of compounds suitable as intermediates for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof. 27. Use of a compound of any of items 1 to 24 in a process for the preparation of an antifungal agent, preferably {5-[(1*S*,2*S*)-2-[(3*S*,6*S*,9*S*,11*R*,15*S*,18*S*,20*R*,21*R*,24*S*,25*S*,26*S*)-3-[(1*R*)*-*2-carbamoyl-1-hydroxyethyl]-11,20,21,25-tetrahydroxy-15-[(1R)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof. 28. The use of item 27, wherein the process comprises the step of reacting a compound of any of items 1 to 24 with a compound of formula (II") or a salt thereof

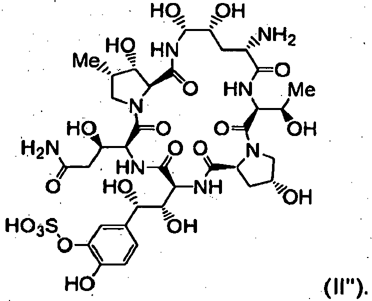

29. The use of item 28, wherein the step is carried out at a temperature of -30 °C to 50 °C, particularly from -20 °C. to 30 °C, and more particularly 0 °C to room temperature. 30.The use of item 28 or 29, wherein the step is carried out in a solvent, particularly an aprotic solvent. 31. The use of item 30, wherein the solvent is selected from tetrahydrofuran (THF), dimethylformamide (DMF), dimethylsulfoxide (DMSO), toluene, 2-methyltetrahydrofuran, *N-*Methyl-2-pyrrolidone (NMP), and any combination thereof, and is particularly DMF. 32. The use of any of items 28 to 31, wherein the step is carried out in the presence of a base. 33. The use of item 32, wherein the base is present in an amount of 1-50 equivalent, particularly of 1-10 equivalent, and more particularly 1-3 equivalent, with respect to the compound of formula (II") or a salt thereof. 34. The use of item 32 or 33, wherein the base is selected from pyridines, lutidines, picolines, dimethylaminopyridine, and collidines, tertiary amines, particularly triethylamine and diisopropylethylamine; cyclic tertiary amines, particularly N-methylpiperidine; aromatic bases, particularly pyridines, lutidines, picolines, dimethylaminopyridine and N-methylmorpholine, and collidines, and any combination thereof; guanidine bases, particularly guanidine and tetramethylguanidine; amidine bases, particularly 1,8-Diazabicyclo[5.4.0]undec-7-en; and any combination thereof. 35. The use of any of items 28 to 31, wherein the process is carried out in the absence of a base. 36. A process for the preparation of a compound of formula (I") 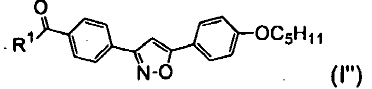 wherein R¹ is a Z-S- residue as defined in any of items 1 to 18; wherein the process comprises the step of reacting a compound of formula (III") or a salt thereof 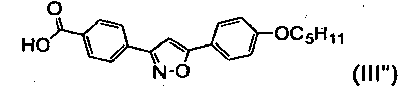 with a compound of formula (IV")

Z-S-L (IV")

wherein L is a group to be formally substituted in the reaction of the compound of formula (III") or a salt thereof with the compound of formula (IV"). 37. The process of item 36, wherein L is selected from hydrogen and Z-S- residues. 38.The process of item 36, wherein the compound of formula (IV") is selected from 2,2'-dithiobis(benzothiazole), 2-mercapto-5-methyl-1,3,4-thiadiazole, 2-mercapto-1,3,4-thiadiazole. 39. The process of any of items 36 to 39, wherein the compound of formula (IV") is present in an amount of 1.05-3 equivalent, particularly of 1.1-2 equivalent, and more particularly 1.4-1.5 equivalent, with respect to the compound of formula (III") or a salt thereof. 40.The process of any of items 36 to 39, wherein the step is carried out at a temperature of -30 °C to 50 °C, particularly from 10 °C to 30 °C, and more particularly at room temperature. 41. The process of any of items 36 to 40, wherein the step is carried out in a solvent, particularly an aprotic solvent. 42.The process of item 41, wherein the solvent is selected from tetrahydrofuran (THF), dimethylformamide (DMF), dimethylsulfoxide (DMSO), toluene, 2-methyltetrahydrofuran, and any combination thereof, and is particularly DMF. 43. The process of any of items 36 to 42, wherein the step is carried out in the presence of a coupling agent and/or an activating agent. 44.The process of item 43, wherein the coupling agent is selected from carbodiimides. 45.The process of item 43, wherein the coupling agent is a carbodiimide and is selected from 1-ethyl-3-(3-dimethylaminopropyl) carbodiimide (EDCI), N,N'-dicyclohexylcarbodiimide (DCC), N,N'-diisopropylcarbodiimide (DIC), and is particularly EDCI. 46. The process of item 43, wherein the activating agent is a reducing agent, particularly a tertiary phosphine, and more particularly triphenylphosphine. 47. The process of any of items 43 to 46, wherein the coupling agent or the activating agent is present in an amount of 1.05-3 equivalent, particularly of 1.2-2 equivalent, and more particularly 1.4-1.5 equivalent, with respect to the compound of formula (III") or a salt thereof. 48. The process of item 36 for the preparation of a compound of formula (Ia") 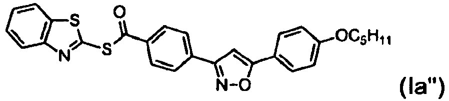 wherein the process comprises the step of reacting a compound of formula (III") or a salt thereof 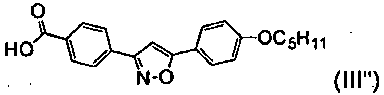 with.2,2'-dithiobis(benzothiazole), present in an amount of 1.5 equivalents, in the presence of 1.5 equivalents triphenylphosphine; each with respect to the compound of formula (III") or a salt thereof; at room temperature in DMF. 49. The process of item 36 for the preparation of a compound of formula (Ib") 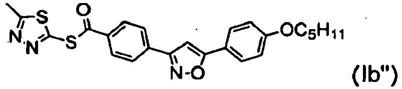 wherein the process comprises the step of reacting a compound of formula (III") or a salt thereof 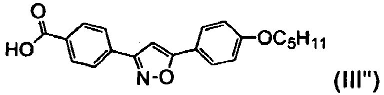 with 2-mercapto-5-methyl-1,3,4-thiadiazole present in an amount of 1.4 equivalents, in the presence of 1.4 equivalents EDCI, each with respect to the compound of formula (III") or a salt thereof; at room temperature in DMF. 50. Also disclosed is a process for the preparation of a compound of formula (I") 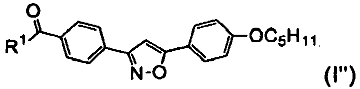 wherein R¹ is an Y-O-residues as defined in any of items 1, 19 to 21 and 23; wherein the process comprises the step of reacting a compound of formula (III") or a salt thereof 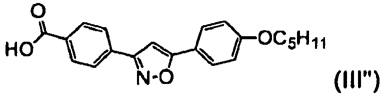 with a compound of formula (V")

Y-O-L (V")

wherein L is a group to be formally substituted in the reaction of the compound of formula (III") or a salt thereof with the compound of formula (V"). 51. The process of item 50, wherein L is hydrogen. 52. The process of item 50, wherein the compound of formula (V") is selected from pentafluorophenol, pentachlorophenol, trifluorophenol, and trichlorophenol.

53.The process of any of items 50 to 52, wherein the compound of formula (V") is present in an amount of 1.05-3 equivalent, particularly of 1.1-2 equivalent, and more particularly 1.1-1.5 equivalent, with respect to the compound of formula (III") or a salt thereof. 54. The process of any of items 50 to 53, wherein the step is carried out at a temperature of -30 °C to 50 °C, particularly from 10 °C to 30 °C, and more particularly at room temperature. 55. The process of any of items 50 to 54, wherein the step is carried out in a solvent, particularly an aprotic solvent. 56. The process of item 55, wherein the solvent is selected from tetrahydrofuran (THF), dimethylformamide (DMF), dimethylsulfoxide (DMSO), toluene, 2-methyltetrahydrofuran, and any combination thereof, and is particularly DMF. 57. The process of any of items 50 to 56, wherein the step is carried out in the presence of a coupling agent. 58. The process of item 57, wherein the coupling agent is selected from carbodiimides. 59. The process of item 57, wherein the coupling agent is a carbodiimide and is selected from 1-ethyl-3-(3-dimethylaminopropyl) carbodiimide (EDCI), N,N'-dicyclohexylcarbodiimide (DCC), N,N'-diisopropylcarbodiimide (DIC), and is particularly EDCI. 60. The process of any of items 57 to 59, wherein the coupling agent is present in an amount of 1.05-3 equivalent, particularly of 1.1-2 equivalent, and more particularly 1.1-1.4 equivalent, with respect to the compound of formula (III") or a salt thereof. 61. Also disclosed is the process of item 50 for the preparation of a compound of formula (Ic") 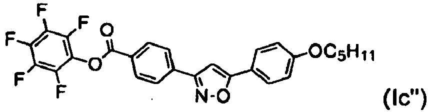 wherein the process comprises the step of reacting a compound of formula (III") or a salt thereof 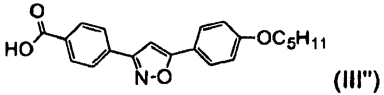 with pentafluorophenol present in an amount of 1.1 equivalents, in the presence of 1.1 equivalents EDCI, each with respect to the compound of formula (III") or a salt thereof; at room temperature in DMF. 62. A process for the preparation of an antifungal agent, preferably {5-[(1*S*,2*S*)-2-[(3*S*,6*S*,9*S,*11*R,*15*S*,18*S*,20*R*,21*R*,24*S*,25*S*,26*S*)-3-[(1*R*)-2-carbamoyl-1-hydroxyethyl]-11,20,21,25-tetrahydroxy-15-[(1R)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof, comprising a process to obtain a compound of any of items 1 to 24, preferably by a process as defined in any of items 36 to.

63. The process of item 62, comprising the step of reacting the obtained compound with a compound of formula (II") or a salt thereof

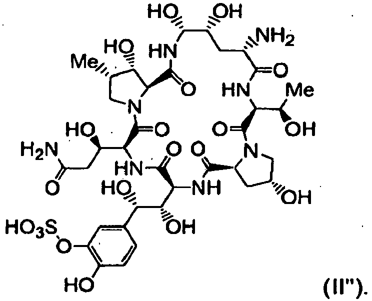

## Claims

1. A compound of formula (I) 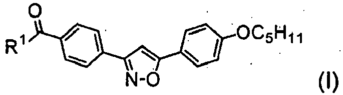 wherein R¹ is selected from: (a) Z-O- residues, wherein Z-O- is selected from
(i) residues of formula (II) 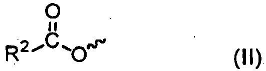
(ii) residues of formula (III) 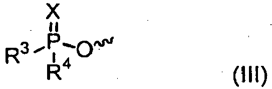 wherein R³ and R⁴ are identical or different; and
wherein X is selected from O or S;
(iii) residues of formula (IV) 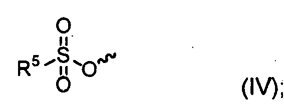 and
(iv) residues of formula (V): 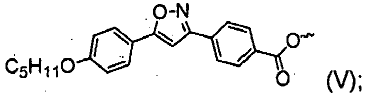
wherein R², R³, R⁴, and R⁵ are independently selected from alkyl residues having 1 to 12 carbon atoms, aryl residues having 6 to 24 carbon atoms, alkyloxy residues having 1 to 12 carbon atoms, aryloxy residues having 6 to 24 carbon atoms, or heterocyclic residues having 2 to 14 carbon atoms and 1 to 4 hetero atoms as ring component atoms; or
(b) a group consisting of halides and pseudohalides.

2. The compound of claim 1, wherein the compound of formula (I) is

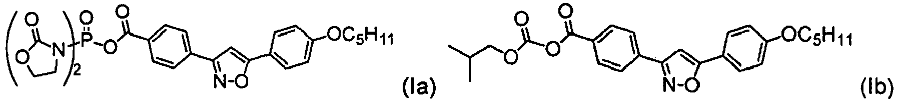

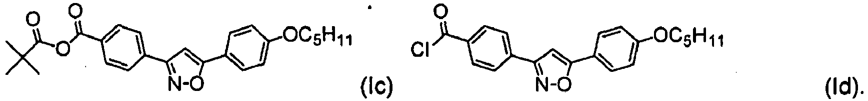

3. Use of a compound of claim 1 or 2 in a process for the preparation of
(i) another compound of claim 1 or 2,
(ii) compounds suitable as intermediates for the preparation of an echinocandin antifungal agent, or
(iii) an antifungal agent, preferably wherein the antifungal agent is {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof, wherein the process in (iii) preferably further comprises the step of reacting the compound of claim 1 or 2 with a compound of formula (VI) or a salt thereof 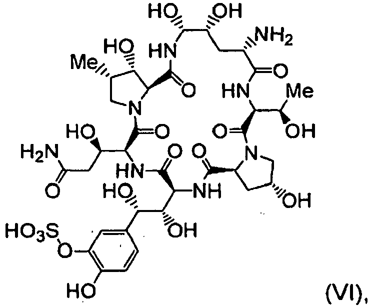
preferably wherein
(a) the step is carried out in the presence of a base, or
(b) in the compound of formula (I) R¹ is selected from Z-O- residues as defined in claim 1 and wherein the process is carried out in the absence of a base.

4. A process for the preparation of a compound of formula (I) 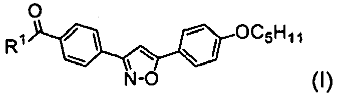 wherein
(i) R¹ is a Z-O- residue as defined in claim 1; and
wherein the process comprises the step of reacting a compound of formula (VII) or a salt thereof 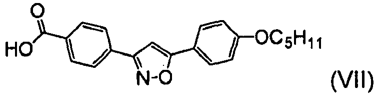 with a compound of formula (VIII)
Z-L (VIII)
wherein L is a leaving group; or
(ii) R¹ is selected from a group consisting of halides and pseudohalides; and
wherein the process comprises the step of reacting a compound of formula (VII) or a salt thereof 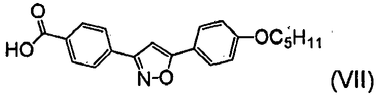 with a reagent capable of halogenating or pseudohalogenating a carboxylic acid.

5. A process for the preparation of an antifungal agent, comprising a process to obtain a compound of claim 1 or 2, preferably by a process as defined in claim 4, wherein the process preferably comprises the step of
(i) reacting the obtained compound with a compound of formula (VI) or a salt thereof 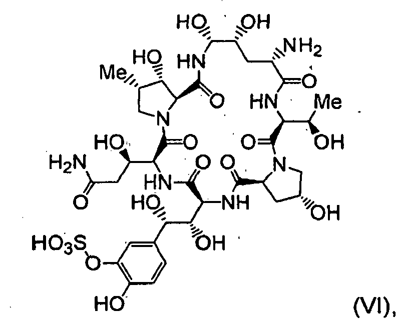 or
(ii) reacting the obtained compound without purification, with a compound of formula (VI) or a salt thereof 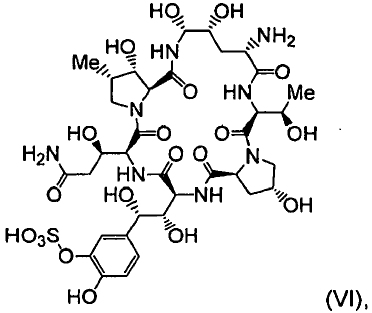
preferably, wherein the antifungal agent is {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof.

6. A compound of formula (I') 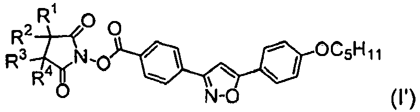 wherein R¹, R², R³ and R⁴ are same or different, and are independently selected from hydrogen; alkyl residues having 1 to 12 carbon atoms, aryl residues having 6 to 24 carbon atoms, alkyloxy residues having 1 to 12 carbon atoms, aryloxy residues having 6 to 24 carbon atoms, and heterocyclic residues having 2 to 14 carbon atoms and 1 to 4 hetero atoms as ring component atoms; and wherein one of R¹ and R², and one of R³ and R⁴ are optionally fused to form a non-aromatic ring, wherein the other two are as defined above or form a bond; or an aromatic ring; or a bond, wherein the other two are as defined above, preferably wherein the compound of formula (I') is

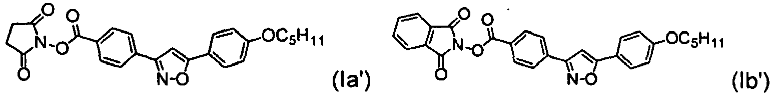

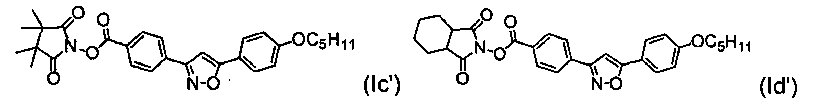

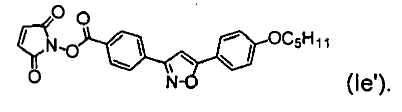

7. Use of a compound of claim 6 in a process for
(i) the preparation of another compound of claim 6, or
(ii) the preparation of an antifungal agent, preferably wherein the antifungal agent is {5-[(1*S*,2*S*)-2-[(3*S*,6*S*,9*S*,11*R*,15*S*,18*S*.20*R*,21R,24S,25S,26S)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof, preferably wherein the process of (ii) comprises the step of reacting the compound of claim 6 with a compound of formula (II') or a salt thereof 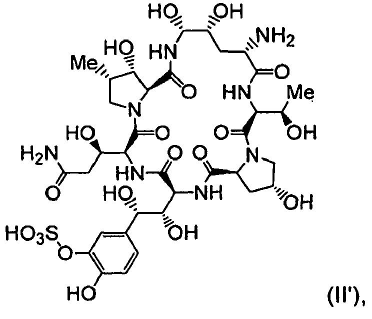
preferably said step of reacting is carried out in the presence of a base or wherein the process is carried out in the absence of a base.

8. A process for the preparation of a compound of formula (I') 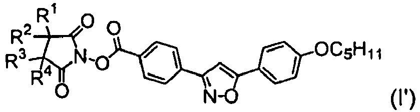 wherein R¹, R², R³ and R⁴ are as defined in claim 6; wherein the process comprises the step of reacting a compound of formula (III') or a salt thereof 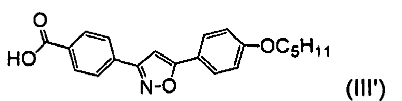 with a compound of formula (IV') 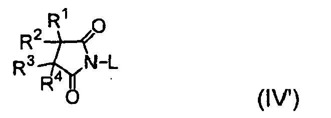 wherein L is a group to be formally substituted in the reaction of the compound of formula (III') or a salt thereof with the compound of formula (IV').

9. The process of claim 8, wherein (i) L is hydroxyl, and/or (ii) the step is carried out in the presence of a coupling agent, preferably wherein the coupling agent is selected from 1-ethyl-3-(3-dimethylaminopropyl) carbodiimide (EDCI), N,N'-dicyclohexylcarbodiimide (DCC), and N,N'-diisopropylcarbodiimide (DIC), preferably wherein the step comprises or consists of the steps of (a) reacting the compound of formula (III') or a salt thereof with an activating agent to obtain an activated compound of formula (III') or a salt thereof, and then
(b) reacting the activated compound of formula (III') or a salt thereof with the compound of formula (IV'), preferably, wherein the activating agent is selected from oxalyl chloride, thionyl chloride, carbonyl dichloride, phosphorous(III') chloride, phosphorous(V') chloride, phosphorus(V) bromide, and cyanuric chloride.

10. A process for the preparation of an antifungal agent comprising a process to obtain a compound of claim 6, preferably by a process as defined claim 8 or 9, preferably wherein the antifungal agent is {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof, wherein the process preferably comprises the step of reacting the obtained compound with a compound of formula (II') or a salt thereof

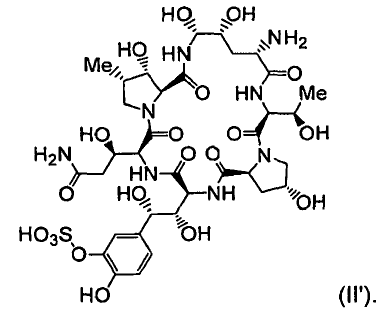

11. A compound of formula (I") 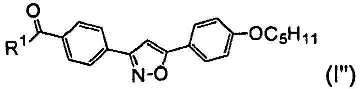 wherein R¹ is selected from Z-S- residues, wherein Z is selected from alkyl residues having 1 to 12 carbon atoms, aryl residues having 6 to 24 carbon atoms, and heterocyclic residues having 2 to 14 carbon atoms and 1 to 4 hetero atoms as ring component atoms; wherein the heterocyclic residues are optionally condensed with a hydrocarbon ring.

12. The compound of claim 11, wherein
(i) the heterocyclic residues are selected from saturated heterocyclic residues, unsaturated heterocyclic residues, and aromatic heterocyclic residues, or
(ii) the compound of formula (I") is

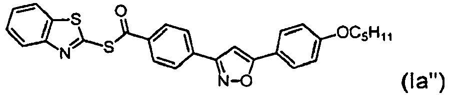

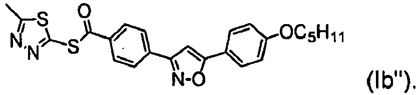

13. Use of a compound of claim 11 or 12 in a process for the preparation of an antifungal agent, preferably wherein the antifungal agent is {5-[(1S,2S)-2-[(3S,6S,9S,11R,15S,18S,20R,21R,24S,25S,26S)-3-[(1R)-2-carbamoyl-1-hydroxyethyl]-11,20,21,25-tetrahydroxy-15-[(1R)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof, further preferred wherein the process comprises the step of reacting the compound of claim 11 or 12 with a compound of formula (II") or a salt thereof

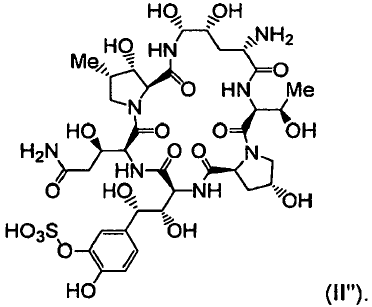

14. A process for the preparation of a compound of formula (I") 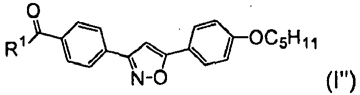 wherein R¹ is a Z-S- residue as defined in claim 11 or 12; wherein the process comprises the step of reacting a compound of formula (III") or a salt thereof 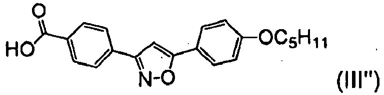 with a compound of formula (IV")
Z-S-L (IV")
wherein L is a group to be formally substituted in the reaction of the compound of formula (III") or a salt thereof with the compound of formula (IV"), preferably wherein (i) L is selected from hydrogen and Z-S- residues, or (ii) the compound of formula (IV") is selected from 2,2'-dithiobis(behzothiazole), 2-mercapto-5-methyl-1,3,4-thiadiazole, 2-mercapto-1,3,4-thiadiazole.

15. A process for the preparation of an antifungal agent, comprising a process to obtain a compound of claim 11 or 12, preferably by a process as defined in claim 14, preferably wherein the antifungal agent is {5-[(1*S*,2*S*)-2-[(3*S*,6*S*,9*S*,11*R*,15*S*,18*S*,20*R*,21*R*,24*S*,25*S*,26*S*)-3-[(1R)-2-carbamoyl-1-hydroxyethyl]-11,20,21,25-tetrahydroxy-15-[(1*R*)-1-hydroxyethyl]-26-methyl-2,5,8,14,17,23-hexaoxo-18-[(4-{5-[4-(pentyloxy)phenyl]-1,2-oxazol-3-yl}benzene)amido]-1,4,7,13,16,22-hexaazatricyclo[22.3.0.0^{9,13}]heptacosan-6-yl]-1,2-dihydroxyethyl]-2-hydroxyphenyl}oxidanesulfonic acid or a salt thereof, further preferred the process comprises the step of reacting the obtained compound with a compound of formula (II") or a salt thereof

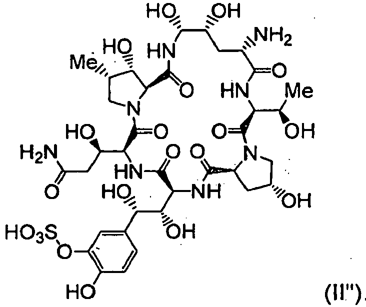

## Patentansprüche

1. Verbindung der Formel (I) 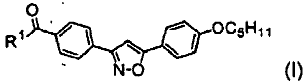 wobei R¹ ausgewählt ist aus: (a) Z-O- Resten, wobei Z-O- ausgewählt ist aus
(i) Resten der Formel (II) 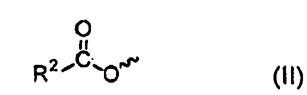
(ii) Resten der Formel (III) 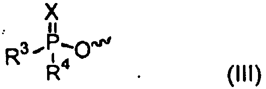 wobei R³ und R⁴ identisch oder verschieden sind; und
wobei X ausgewählt ist aus O oder S;
(iii) Resten der Formel (IV) 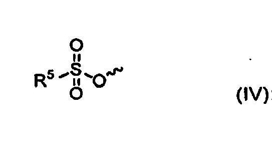 und
(iv) Resten der Formel (V): 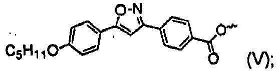
wobei R², R³, R⁴, und R⁵ jeweils unabhängig voneinander ausgewählt sind aus Alkyl-Resten mit 1 bis 12 Kohlenstoffatomen, Aryl-Resten mit 6 bis 24 Kohlenstoffatomen, Alkyloxy-Resten mit 1 bis 12 Kohlenstoffatomen, Aryloxy-Resten mit 6 bis 24 Kohlenstoffatomen, oder heterozyklischen Resten mit 2 bis 14 Kohlenstoffatomen und 1 bis 4 Heteroatomen als Ringkomponent-Atome; oder
(b) einer Gruppe bestehend aus Halogeniden und Pseudohalogeniden.

2. Verbindung nach Anspruch 1,
wobei die Verbindung der Formel (I) 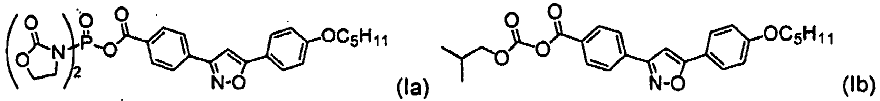 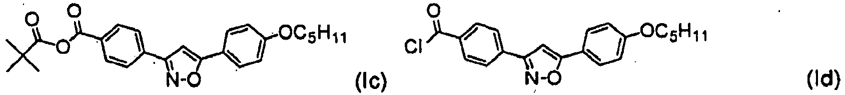 ist.

3. Verwendung einer Verbindung nach Anspruch 1 oder 2 in einem Verfahren für die Herstellung von
(i) einer anderen Verbindung nach Anspruch 1 oder 2,
(ii) Verbindungen, die als Zwischenprodukte für die Herstellung eines Echinocandin-antimykotischen Mittels geeignet sind, oder
(iii) einem antimykotischen Mittel, wobei das antimykotische Mittel vorzugsweise {5-[(1S,2S)-2-[(3S,6S,9S,11R,15S,18S,20R,21R,24S,25S,26S)-3-[(1R)-2-Carbamoyl-1-Hydroxyethyl]-11,20,21,25-Tetrahydroxy-15-[(1R)-1-Hydroxyethyl]-26-Methyl-2,5,8,14,17,23-Hexaoxo-18-[(4-{5-[4-(Pentyloxy)Phenyl]-1,2-Oxazol-3-yl}Benzol)Amido]-1,4,7,13,16,22-Hexaazatricyclo[22.30.0^{9,13}]Heptacosan-6-yl]-1,2-Dihydroxyethyl]-2-Hydroxyphenyl}Oxidan-Sulfonsäure oder ein Salz davon ist, wobei das Verfahren in (iii) vorzugsweise des Weiteren den Schritt des Reagierens der Verbindung von Anspruch 1 oder 2 mit einer Verbindung der Formel (VI) oder einem Salz davon umfasst 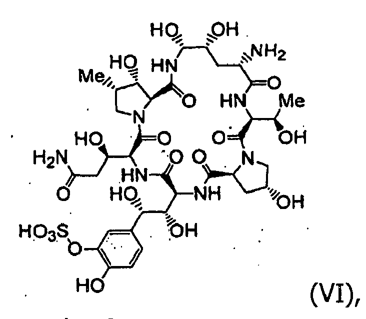
wobei vorzugsweise
(a) der Schritt in der Gegenwart einer Base durchgeführt wird, oder
(b) in der Verbindung der Formel (I) R¹ ausgewählt ist aus Z-O- Resten, wie definiert in Anspruch 1 und wobei das Verfahren in der Abwesenheit einer Base durchgeführt wird.

4. Verfahren für die Herstellung einer Verbindung der Formel (I) 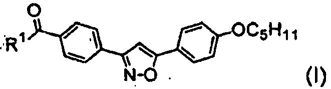 wobei
(i) R¹ ein Z-O- Rest, wie in Anspruch 1 definiert, ist; und
wobei das Verfahren den Schritt des Reagierens einer Verbindung der Formel (VII) oder eines Salzes davon 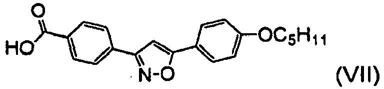 mit einer Verbindung der Formel (VIII)
Z-L (VIII)
umfasst, wobei L eine Abgangsgruppe ist; oder
(ii) R¹ ausgewählt ist aus einer Gruppe, bestehend aus Halogeniden und
Pseudohalogeniden; und wobei das Verfahren den Schritt des Reagierens einer Verbindung der Formel (VII) oder eines Salzes davon 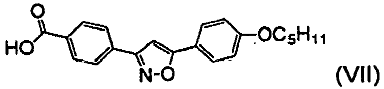
mit einem Reagenz, das fähig ist eine Carbonsäure zu halogenieren oder zu pseudohalogenieren, umfasst.

5. Verfahren für die Herstellung eines antimykotischen Mittels umfassend ein Verfahren, um eine Verbindung gemäß Anspruch 1 oder 2 zu erhalten, vorzugsweise durch ein Verfahren wie in Anspruch 4 definiert, wobei das Verfahren vorzugsweise den folgenden Schritt umfasst
(i) Reagieren der erhaltenen Verbindung mit einer Verbindung der Formel (VI) oder einem Salz davon 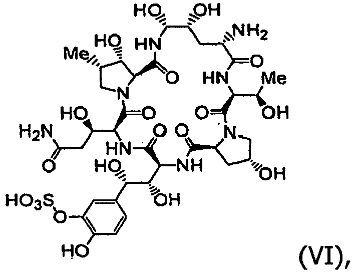 oder
(ii) Reagieren der erhaltenen Verbindung ohne Reinigung, mit einer Verbindung der Formel (VI) oder einem Salz davon 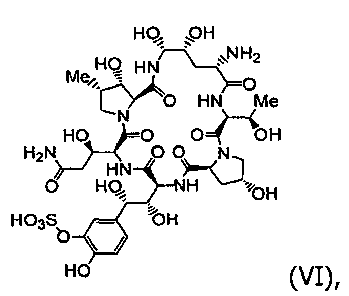
vorzugsweise, wobei das antimykotische Mittel {5-[(1S,2S)-2-[(3S,6S,9S,11R,15S,18S,20R,21R,24S,25S,26S)-3-[(1R)-2-Carbamoyl-1-Hydroxyethyl]-11,20,21,25-Tetrahydroxy-15-[(1R)-1-Hydroxyethyl]-26-Methyl-2,5,8,14,17,23-Hexaoxo-18-[(4-{5-[4-(Pentyloxy)Phenyl]-1,2-Oxazol-3-yl}Benzol)Amido]-1,4,7,13,16,22-Hexaazatricyclo[22.3.0.0^{9,13}]Heptacosan-6-yl]-1,2-Dihydroxyethyl]-2-Hydroxyphenyl}Oxidan-Sulfonsäure oder ein Salz davon ist.

6. Verbindung der Formel (I') 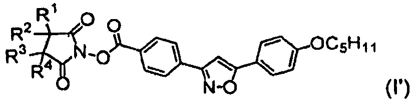 wobei R¹, R², R³ und R⁴ gleich oder verschieden sind, und jeweils unabhängig voneinander ausgewählt sind aus Wasserstoff; Alkyl-Resten mit 1 bis 12 Kohlenstoffatomen, Aryl-Resten mit 6 bis 24 Kohlenstoffatomen, Alkyloxy-Resten mit 1 bis 12 Kohlenstoffatomen, Aryloxy-Resten mit 6 bis 24 Kohlenstoffatomen, und heterozyklischen Resten mit 2 bis 14 Kohlenstoffatomen und 1 bis 4 Heteroatomen als Ringkomponent-Atome; und wobei einer von R¹ und R², und einer von R³ und R⁴ wahlweise verbunden sind zur Bildung eines nicht-aromatischen Rings, wobei die anderen zwei wie oben definiert sind oder eine Bindung bilden; oder eines aromatischen Rings; oder einer Bindung, wobei die anderen zwei wie oben definiert sind, vorzugsweise wobei die Verbindung der Formel (I') 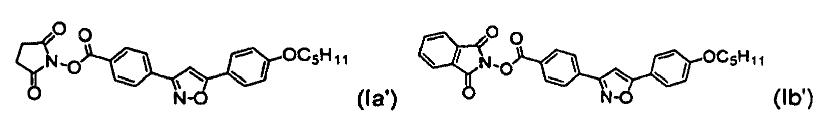 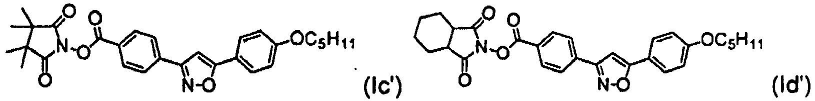 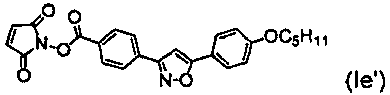 ist.

7. Verwendung einer Verbindung gemäß Anspruch 6 in einem Verfahren für
(i) die Herstellung einer anderen Verbindung nach Anspruch 6, oder
(ii) die Herstellung eines antimykotischen Mittels, vorzugsweise wobei das antimykotische Mittel {5-[(1S,2S)-2-[(3S,6S,9S,11R,15S,18S,20R,21R,24S,25S,26S)-3-[(1R)-2-Carbamoyl-1-Hydroxyethyl]-11,20,21,25-Tetrahydroxy-15-[(1R)-1-Hydroxyethyl]-26-Methyl-2,5,8,14,17,23-Hexaoxo-18-[(4-{5-[4-(Pentyloxy)Phenyl]-1,2-Oxazol-3-yl}Benzol)Amido]-1,4,7,13,16,22-Hexaazatricyclo[22.3.0.0⁹,¹³]Heptacosan-6-yl]-1,2-Dihydroxyethyl]-2-Hydroxyphenyl}Oxidan-Sulfonsäure oder ein Salz davon ist, vorzugsweise wobei das Verfahren von (ii) den Schritt des Reagierens der Verbindung gemäß Anspruch 6 mit einer Verbindung der Formel (II') oder einem Salz davon umfasst 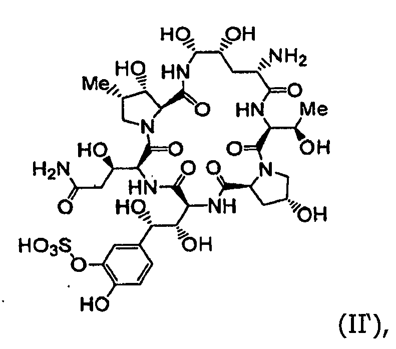
wobei der Schritt des Reagierens vorzugsweise in der Anwesenheit einer Base durchgeführt wird, oder wobei das Verfahren in der Abwesenheit einer Base durchgeführt wird.

8. Verfahren für die Herstellung einer Verbindung der Formel (I') 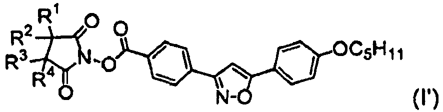 wobei R¹, R², R³ und R⁴ wie in Anspruch 6 definiert sind; wobei das Verfahren den Schritt des Reagierens einer Verbindung der Formel (III') oder eines Salzes davon 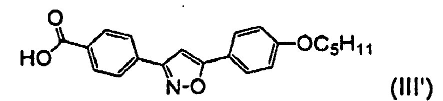 mit einer Verbindung der Formel (IV') 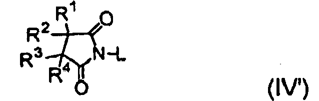 wobei L eine Gruppe ist, die formal in der Reaktion der Verbindung der Formel (III') oder eines Salzes davon mit der Verbindung der Formel (IV') substituiert werden soll, umfasst.

9. Verfahren nach Anspruch 8,
wobei (i) L Hydroxyl ist, und/oder (ii) wobei der Schritt in der Gegenwart eines Kupplungsreagenz durchgeführt wird, wobei das Kupplungsreagenz vorzugsweise ausgewählt ist aus 1-Ethyl-3-(3-Dimethylaminopropyl) Carbodiimid (EDCI), N,N'-Dicyclohexylcarbodiimid (DCC), and N,N'-Diisopropylcarbodiimid (DIC), wobei der Schritt vorzugsweise umfasst oder besteht aus den Schritten (a) Reagieren der Verbindung der Formel (III') oder eines Salzes davon mit einem Aktivierungsmittel, um eine aktivierte Verbindung der Formel (III') oder eines Salzes davon zu erhalten, und dann
(b) Reagieren der aktivierten Verbindung der Formel (III') oder eines Salzes davon mit der Verbindung der Formel (IV'), vorzugsweise, wobei das Aktivierungsmittel ausgewählt ist aus Oxalylchlorid, Thionylchlorid, Carbonyldichlorid, Phosphor(III')chlorid, Phosphor(V')chlorid, Phosphor(V')bromid, und Cyanurchlorid.

10. Verfahren für die Herstellung eines antimykotischen Mittels umfassend ein Verfahren, um eine Verbindung gemäß Anspruch 6 zu erhalten, vorzugsweise durch das Verfahren wie in Anspruch 8 oder 9 definiert, wobei das antimykotische Mittel {5-[(1S,2S)-2-[(3S,6S,9S,11R,15S,18S,20R,21R,24S,25S,26S)-3-[(1R)-2-Carbamoyl-1-Hydroxyethyl]-11,20,21,25-Tetrahydroxy-15-[(1R)-1-Hydroxyethyl]-26-Methyl-2,5,8,14,17,23-Hexaoxo-18-[(4-{5-[4-(Pentyloxy)Phenyl]-1,2-Oxazol-3-yl}Benzol)Amido]-1,4,7,13,16,22-Hexaazatricyclo[22.3.0.0^{9,13}]Heptacosan-6-yl]-1,2-Dihydroxyethyl]-2-Hydroxyphenyl}Oxidan-Sulfonsäure oder ein Salz davon ist, wobei das Verfahren vorzugsweise den Schritt des Reagierens der erhaltenen Verbindung mit einer Verbindung der Formel (II') oder eines Salzes davon umfasst

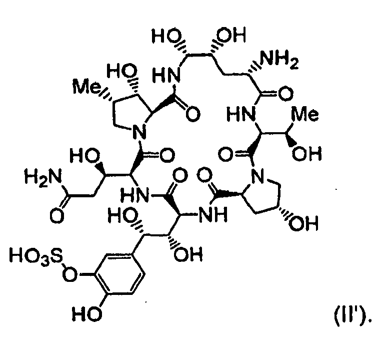

11. Verbindung der Formel (I") 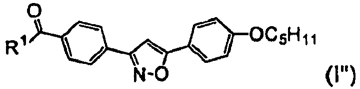 wobei R¹ ausgewählt ist aus Z-S- Resten, wobei Z ausgewählt ist aus Alkyl-Resten mit 1 bis 12 Kohlenstoffatomen, Aryl-Resten mit 6 bis 24 Kohlenstoffatomen, und heterozyklischen Resten mit 2 bis 14 Kohlenstoffatomen und 1 bis 4 Heteroatomen als Ringkomponent-Atome; wobei die heterozyklischen Reste wahlweise mit einem Kohlenwasserstoff-Ring kondensiert sind.

12. Verbindung nach Anspruch 11,
wobei
(i) die heterozyklischen Reste ausgewählt sind aus gesättigten heterozyklischen Resten, ungesättigten heterozyklischen Resten, und aromatischen heterozyklischen Resten, oder
(ii) die Verbindung der Formel (I") 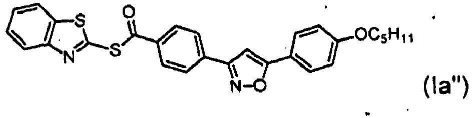 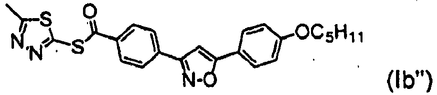
ist.

13. Verwendung einer Verbindung gemäß Anspruch 11 oder 12 in einen Verfahren für die Herstellung eines antimykotischen Mittels, vorzugsweise wobei das antimykotische Mittel {5-[(1S,2S)-2-[(3S,6S,9S,11R,15S,18S,20R,21R,24S,25S,26S)-3-[(1R)-2-Carbamoyl-1-Hydroxyethyl]-11,20,21,25-Tetrahydroxy-15-[(1R)-1-Hydroxyethyl]-26-Methyl-2,5,8,14,17,23-Hexaoxo-18-[(4-{5-[4-(Pentyloxy)Phenyl]-1,2-Oxazol-3-yl}Benzol)Amido]-1,4,7,13,16,22-Hexaazatricyclo[22.3.0.0^{9,13}]Heptacosan-6-yl]-1,2-Dihydroxyethyl]-2-Hydroxyphenyl}Oxidan-Sulfonsäure oder ein Salz davon ist, wobei das Verfahren des Weiteren vorzugsweise den Schritt des Reagierens der Verbindung gemäß Anspruch 11 oder 12 mit einer Verbindung der Formel (II") oder eines Salzes davon umfasst

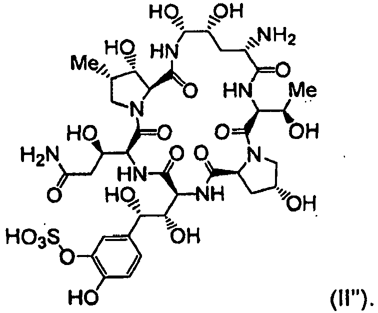

14. Verfahren für die Herstellung einer Verbindung der Formel (I") 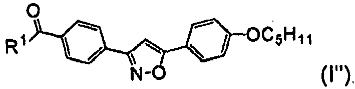 wobei R¹ ein Z-S- Rest wie in Anspruch 11 oder 12 definiert ist; wobei das Verfahren den Schritt des Reagierens einer Verbindung der Formel (III") oder eines Salzes davon 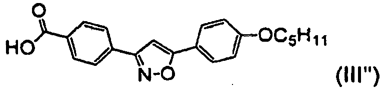 mit einer Verbindung der Formel (IV"),
Z-S-L (IV")
wobei L eine Gruppe ist, die formal in der Reaktion der Verbindung der Formel (III") oder eines Salzes davon mit der Verbindung der Formel (IV") substituiert werden soll, umfasst,
wobei vorzugsweise (i) L ausgewählt ist aus Wasserstoff und Z-S- Resten, oder (ii) die Verbindung der Formel (IV") ausgewählt ist aus 2,2'-Dithiobis(Benzothiazol), 2-Mercapto-5-Methyl-1,3,4-Thiadiazol, 2-Mercapto-1,3,4-Thiadiazol.

15. Verfahren für die Herstellung eines antimykotischen Mittels, umfassend ein Verfahren, um eine Verbindung gemäß Anspruch 11 oder 12 zu erhalten, vorzugsweise durch ein Verfahren wie in Anspruch 14 definiert, vorzugsweise wobei das antimykotische Mittel {5-[(1S,2S)-2-[(3S,6S,9S,11R,15S,18S,20R,21R,24S,25S,26S)-3-[(1R)-2-Carbamoyl-1-Hydroxyethyl]-11,20,21,25-Tetrahydroxy-15-[(1R)-1-Hydroxyethyl]-26-Methyl-2,5,8,14,17,23-Hexaoxo-18-[(4-{5-[4-(Pentyloxy)Phenyl]-1,2-Oxazol-3-yl}Benzol)Amido]-1,4,7,13,16,22-Hexaazatricyclo[22.3.0.0^{9,13}]Heptacosan-6-yl]-1,2-Dihydroxyethyl]-2-Hydroxyphenyl}Oxidan-Sulfonsäure oder ein Salz davon ist, wobei das Verfahren des Weiteren bevorzugt den Schritt des Reagierens der erhaltenen Verbindung mit einer Verbindung der Formel (II") oder eines Salzes davon umfasst

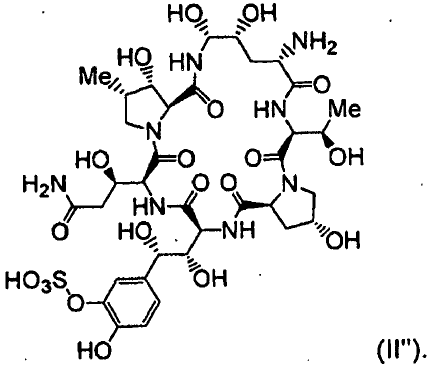

## Revendications

1. Composé de formule (I) 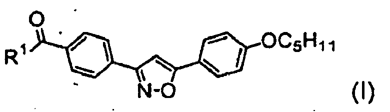 dans laquelle R¹ est choisi parmi :
(a) des résidus Z-O-, dans laquelle Z-O- est choisi parmi
(i) les résidus de formule (II) 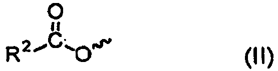
(ii) les résidus de formule (III) 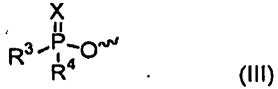 dans laquelle R³ et R⁴ sont identiques ou différents ; et
dans laquelle X est choisi parmi O ou S ;
(iii) les résidus de formule (IV) 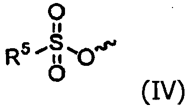 et
(iv) les résidus de formule (V) 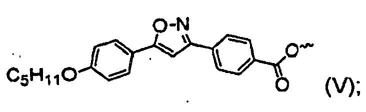
dans laquelle R², R³, R⁴ et R⁵ sont indépendamment choisis parmi des résidus alkyle ayant 1 à 12 atomes de carbone, des résidus aryle ayant 6 à 24 atomes de carbone, des résidus alkyloxy ayant 1 à 12 atomes de carbone, des résidus aryloxy ayant 6 à 24 atomes de carbone, ou des résidus hétérocycliques ayant 2 à 14 atomes de carbone et 1 à 4 hétéroatomes comme atomes constitutifs du cycle ; ou
(b) un groupe constitué par les halogénures et les pseudohalogénures.

2. Composé selon la revendication 1, dans lequel le composé de formule (I) est

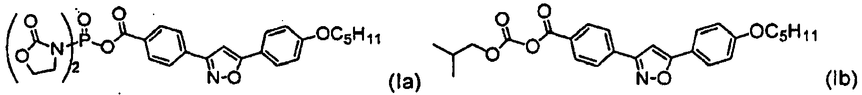

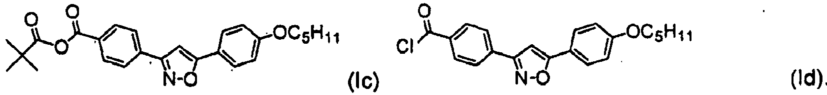

3. Utilisation d'un composé selon la revendication 1 ou 2, dans un procédé pour la préparation
(i) d'un autre composé selon la revendication 1 ou 2,
(ii) de composés appropriés en tant qu'intermédiaires pour la préparation d'un agent antifongique de type échinocandine, ou
(iii) d'un agent antifongique, dans lequel de préférence l'agent antifongique est l'acide {5-[(1*S*,2*S*)-2-[(3*S*,6*S*,9*S*,11*R*,15*S*,18*S*,20*R*,21*R*,24*S*,25*S*,26*5*)-3-[(1*R*)-2-carbamoyl-1-hydroxyéthyl]-11,20,21,25-tétrahydroxy-15-[(1*R*)-1-hydroxyéthyl]-26-méthyl-2,5,8,14,17,23-hexaoxo-18-[(4-{5-[4-(pentyloxy)phényl]-1,2-oxazol-3-yl}benzène)amido]-1,4,7,13,16,22-hexaazatricyclo[22.3.0.0^{9,13}]heptacosan-6-yl]-1,2-dihydroxyéthyl]-2-hydroxyphényl}oxidanesulfonique ou un sel de celui-ci, dans lequel le procédé en (iii) comprend de préférence en outre l'étape de réaction du composé selon la revendication 1 ou 2 avec un composé de formule (VI) ou un sel de celui-ci 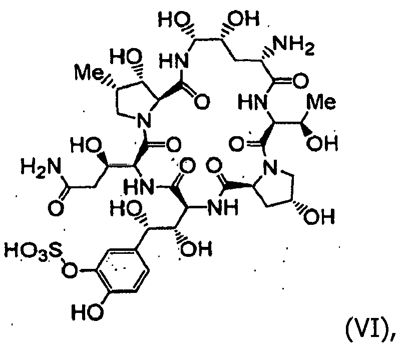
dans lequel de préférence
(a) l'étape est réalisée en présence d'une base, ou
(b) dans le composé de formule (I), R¹ est choisi parmi les résidus Z-O- tels que définis dans la revendication 1 et dans lequel le procédé est réalisé en l'absence de base.

4. Procédé pour la préparation d'un composé de formule (I) 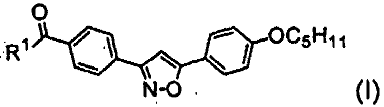 dans laquelle
(i) R¹ est un résidu Z-O- tel que défini dans la revendication 1 ; et
dans lequel le procédé comprend l'étape de réaction d'un composé de formule (VII) ou d'un sel de celui-ci 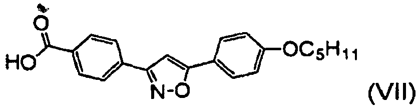 avec un composé de formule (VIII)
Z-L (VIII)
dans laquelle L est un groupe partant ; ou
(ii) R¹ est choisi dans un groupe constitué par les halogénures et les pseudohalogénures ; et
dans lequel le procédé comprend l'étape de réaction d'un composé de formule (VII) ou d'un sel de celui-ci 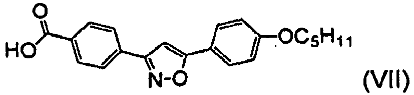 avec un réactif capable d'halogénation ou de pseudohalogénation d'un acide carboxylique.

5. Procédé pour la préparation d'un agent antifongique, comprenant un procédé pour obtenir un composé selon la revendication 1 ou 2, de préférence par un procédé tel que défini dans la revendication 4, dans lequel le procédé comprend de préférence l'étape de
(i) réaction du composé obtenu avec un composé de formule (VI) ou un sel de celui-ci 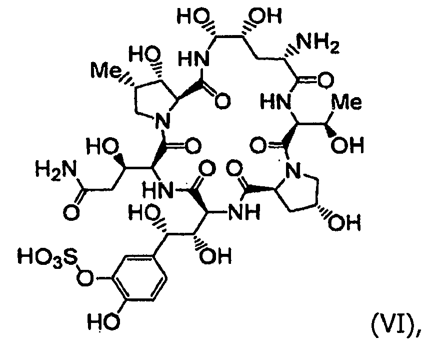 ou
(ii) réaction du composé obtenu sans purification, avec un composé de formule (VI) ou un sel de celui-ci 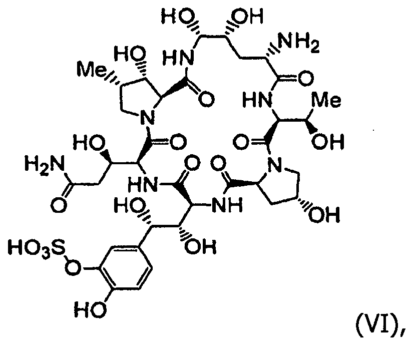
de préférence, dans lequel l'agent anti-fongique est l'acide {5-[(1*S*,2*S*)-2-[(3*S*,6*S*,9*S*,11*R*,15*S*,18*S*,20*R*,21*R*,24*S*,25*S*,26*S*-3-[(1*R*)-2-carbamoyl-1-hydroxyéthyl]-11,20,21,25-tétrahydroxy-15-[(1*R*)-1-hydroxy-éthyl]-26-méthyl-2,5,8,14,17,23-hexaoxo-18-[(4-{5-[4-(pentyloxy)phényl]-1,2-oxazol-3-yl}benzène)amido]-1,4,7,13,16,22-hexaazatricyclo[22.3.0.0^{9,13}]heptacosan-6-yl]-1,2-dihydroxyéthyl]-2-hydroxyphényl}oxidanesulfonique ou un sel de celui-ci.

6. Composé de formule (I') 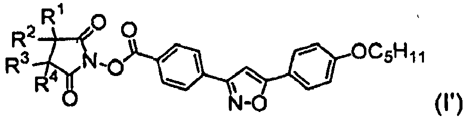 dans laquelle R¹, R², R³ et R⁴ sont identiques ou différents, et sont indépendamment choisis parmi un hydrogène ; des résidus alkyle ayant 1 à 12 atomes de carbone, des résidus aryle ayant 6 à 24 atomes de carbone, des résidus alkyloxy ayant 1 à 12 atomes de carbone, des résidus aryloxy ayant 6 à 24 atomes de carbone, et des résidus hétérocycliques ayant 2 à 14 atomes de carbone et 1 à 4 hétéroatomes comme atomes constitutifs du cycle ; et dans laquelle l'un de R¹ et R², et l'un de R³ et R⁴ sont éventuellement condensés pour former un cycle non aromatique, dans laquelle les deux autres sont tels que définis ci-dessus ou forment une liaison ; ou un cycle aromatique ; ou une liaison, dans laquelle les deux autres sont tels que définis ci-dessus, de préférence dans laquelle le composé de formule (I') est

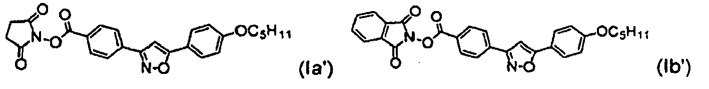

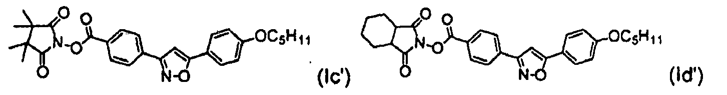

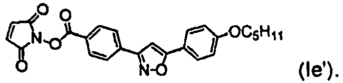

7. Utilisation d'un composé selon la revendication 6 dans un procédé pour
(i) la préparation d'un autre composé selon la revendication 6, ou
(ii) la préparation d'un agent antifongique, dans lequel de préférence l'agent antifongique est l'acide {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxy-éthyl]-11,20,21,25-tétrahydroxy-15-[(1*R*)-1-hydroxyéthyl]-26-méthyl-2,5,8,14,17,23-hexaoxo-18-[(4-{5-[4-(pentyloxy)phényl]-1,2-oxazol-3-yl}benzène)amido]-1,4,7,13,16,22-hexaazatricyclo[22.3.0.0^{9,13}]heptacosan-6-yl]-1,2-dihydroxyéthyl]-2-hydroxyphényl}oxidanesulfonique ou un sel de celui-ci, dans lequel de préférence le procédé en (ii) comprend l'étape de réaction du composé selon la revendication 6 avec un composé de formule (II') ou un sel de celui-ci 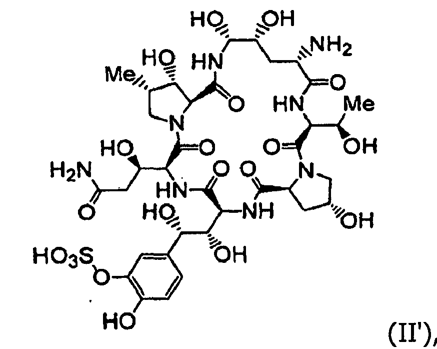
de préférence ladite étape de réaction est effectuée en présence d'une base ou dans lequel le procédé est réalisé en l'absence de base.

8. Procédé pour la préparation d'un composé de formule (I') 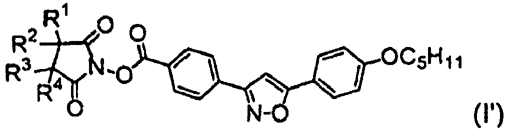 dans laquelle R¹, R², R³ et R⁴ sont tels que définis dans la revendication 6 ; dans lequel le procédé comprend l'étape de réaction d'un composé de formule (III') ou d'un sel de celui-ci 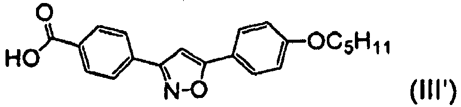 avec un composé de formule (IV') 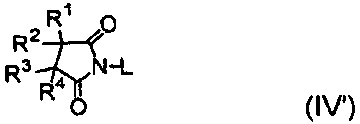 dans laquelle L est un groupe à substituer systématiquement dans la réaction du composé de formule (III') ou d'un sel de celui-ci avec le composé de formule (IV').

9. Procédé selon la revendication 8, dans lequel (i) L est un hydroxyle, et/ou (ii) l'étape est réalisée en présence d'un agent de couplage, dans lequel de préférence l'agent de couplage est choisi parmi le 1-éthyl-3-(3-diméthylaminopropyl)carbodiimide (EDCI), le N,N'-dicyclohexylcarbodiimide (DCC) et le N,N'-diisopropylcarbodiimide (DIC), dans lequel de préférence l'étape comprend ou consiste en les étapes de (a) réaction du composé de formule (III') ou d'un sel de celui-ci avec un agent d'activation pour obtenir un composé activé de formule (III') ou un sel de celui-ci, puis
(b) réaction du composé activé de formule (III') ou d'un sel de celui-ci avec le composé de formule (IV'), de préférence, dans lequel l'agent d'activation est choisi parmi le chlorure d'oxalyle, le chlorure de thionyle, le dichlorure de carbonyle, le chlorure de phosphore (III'), le chlorure de phosphore (V'), le bromure de phosphore (V') et le chlorure cyanurique.

10. Procédé pour la préparation d'un agent antifongique comprenant un procédé pour obtenir un composé selon la revendication 6, de préférence par un procédé tel que défini selon la revendication 8 ou 9, dans lequel de préférence l'agent antifongique est l'acide {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyéthyl]-11,20,21,25-tétrahydroxy-15-[(1*R*)-1-hydroxyéthyl]-26-méthyl-2,5,8,14,17,23-hexaoxo-18-[(4-{5-[4-(pentyloxy)phényl]-1,2-oxazol-3-yl}benzène)amido]-1,4,7,13,16,22-hexaazatricyclo[22.3.0.0^{9,13}]heptacosan-6-yl]-1,2-dihydroxyéthyl]-2-hydroxyphényl}oxidanesulfonique ou un sel de celui-ci, dans lequel le procédé comprend de préférence l'étape de réaction du composé obtenu avec un composé de formule (II') ou un sel de celui-ci

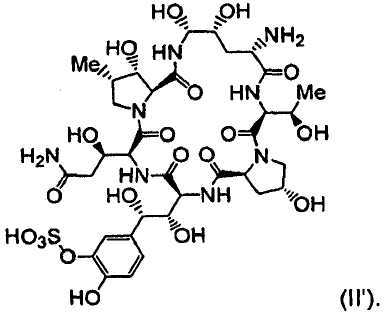

11. Composé de formule (I") 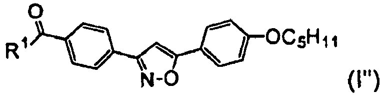 dans laquelle R¹ est choisi parmi les résidus Z-S-, dans lesquels Z est choisi parmi des résidus alkyle ayant 1 à 12 atomes de carbone, des résidus aryle ayant 6 à 24 atomes de carbone, et des résidus hétérocycliques ayant 2 à 14 atomes de carbone et 1 à 4 hétéroatomes comme atomes constitutifs du cycle ; dans laquelle les résidus hétérocycliques sont éventuellement condensés à un cycle hydrocarboné.

12. Composé selon la revendication 11, dans lequel
(i) les résidus hétérocycliques sont choisis parmi des résidus hétérocycliques saturés, des résidus hétérocycliques insaturés, et des résidus hétérocycliques aromatiques, ou
(ii) le composé de formule (I") est

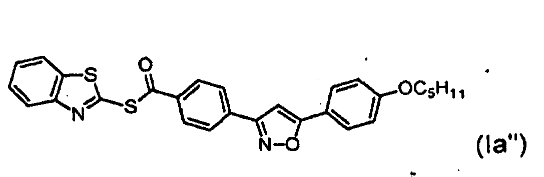

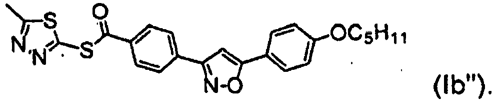

13. Utilisation d'un composé selon la revendication 11 ou 12, dans un procédé pour la préparation d'un agent antifongique, dans lequel de préférence l'agent antifongique est l'acide {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyéthyl]-11,20,21,25-tétrahydroxy-15-[(1*R*)-1-hydroxyéthyl]-26-méthyl-2,5,8,14,17,23-hexaoxo-18-[(4-{5-[4-(pentyloxy)phényl]-1,2-oxazol-3-yl}benzène)amido]-1,4,7,13,16,22-hexaazatricyclo[22.3.0.0^{9,13}]heptacosan-6-yl]-1,2-dihydroxyéthyl]-2-hydroxyphényl}oxidanesulfonique ou un sel de celui-ci, de préférence encore dans lequel le procédé comprend l'étape de réaction du composé selon la revendication 11 ou 12, avec un composé de formule (II") ou un sel de celui-ci

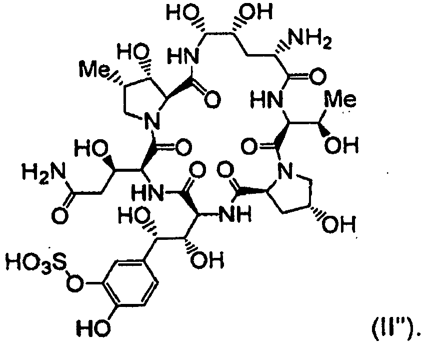

14. Procédé pour la préparation d'un composé de formule (I") 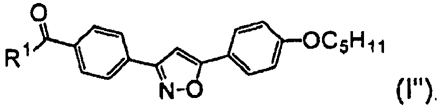 dans lequel R¹ est un résidu Z-S- tel que défini selon la revendication 11 ou 12 ; dans lequel le procédé comprend l'étape de réaction d'un composé de formule (III") ou d'un sel de celui-ci 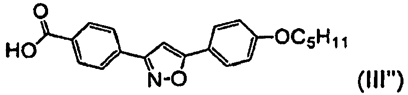 avec un composé de formule (IV")
Z-S-L (IV")
dans laquelle L est un groupe à substituer systématiquement dans la réaction du composé de formule (III") ou d'un sel de celui-ci avec le composé de formule (IV"), dans lequel de préférence (i) L est choisi parmi l'hydrogène et les résidus Z-S-, ou (ii) le composé de formule (IV") est choisi parmi le 2,2'-dithiobis(benzothiazole), le 2-mercapto-5-méthyl-1,3,4-thiadiazole, le 2-mercapto-1,3,4-thiadiazole.

15. Procédé pour la préparation d'un agent antifongique, comprenant un procédé pour obtenir un composé selon la revendication 11 ou 12, de préférence par un procédé tel que défini selon la revendication 14, dans lequel de préférence l'agent antifongique est l'acide {5-[(1*S*,2*S*)-2-[(3*S*,6*S*,9*S*,11*R*,15*S*,18*S*,20*R*,21*R*,24*S*,25*S*,26*S*)-3-[(1*R*)-2-carbamoyl-1-hydroxyéthyl]-11,20,21,25-tétrahydroxy-15-[(1*R*)-1-hydroxyéthyl]-26-méthyl-2,5,8,14,17,23-hexaoxo-18-[(4-{5-[4-(pentytoxy)phény)]-1,2-oxazol-3-yl}benzène)amido]-1,4,7,13,16,22-hexaazatricyclo[22.3.0.0^{9,13}]heptacosan-6-yl]-1,2-dihydroxyéthyl]-2-hydroxyphényl}oxidanesulfonique ou un sel de celui-ci, de préférence encore, le procédé comprend l'étape de réaction du composé obtenu avec un composé de formule (II") ou un sel de celui-ci
